# EUROPEAN PATENT SPECIFICATION

(11) **EP 1 329 457 B1**
(45) Date of publication and mention of the grant of the patent: **02.11.2005**
(21) Application number: 03009409.8
(22) Date of filing: 05.01.1999
(51) Int. Cl.: C07K 5/027, C07D 413/12, C07D 261/18, C07D 207/27, A61K 38/05, A61K 31/40, A61K 31/42, C07K 5/02, A61P 31/14

(54) **Antipicornaviral compounds, their preparation and use**
Antipicornavirale Verbindungen, deren Herstellung und Verwendung
Composés antipicornavirals, leur préparation et utilisation

(30) Priority: 30.04.1998 US 83828 P; 28.08.1998 US 98358 P
(43) Date of publication of application: 23.07.2003
(62) Divisional of application: 99900780.0
(73) Proprietor: AGOURON PHARMACEUTICALS, INC., San Diego, California 92121 (US)
(72) Inventor: Dragovich, Peter S., Encinitas, California 92024 (US); Marakovits, Joseph T., Encinitas, California 92024 (US); Prins, Thomas J., Cardiff, California 92007 (US); Tikhe, Jayashree Girish, San Diego, California 92121 (US); Webber, Stephen E., San Diego, California 92122 (US); Zhou, Ru, Carlsbad, California 92008 (US); Johnson, Theodore O.,Jr., San Diego, California 92130 (US)
(74) Representative: Grünecker, Kinkeldey, Stockmair & Schwanhäusser Anwaltssozietät

(56) References cited:
- WO-A-97/43305
- WO-A-98/43950
- WO-A-99/31122
- KALDOR S W ET AL: "Glutamine-derived aldehydes for the inhibition of human rhinovirus 3C protease" BIOORGANIC & MEDICINAL CHEMISTRY LETTERS, vol. 5, no. 17, 7 September 1995 (1995-09-07), page 2021-2026 XP004135357
- MURRAY P J ET AL: "The enantiospecific synthesis of novel lysine analogues incorporating a pyrrolidine containing side chain" TETRAHEDRON LETTERS, vol. 39, no. 37, 10 September 1998 (1998-09-10), page 6721-6724 XP004132587

## Description

### FIELD AND INDUSTRIAL APPLICABILITY OF THE INVENTION

The invention pertains to peptide-like and peptidomimetic compounds that advantageously inhibit the enzymatic activity of picornaviral 3C proteases, especially rhinovirus 3C proteases (RVPs), and that retard viral growth in cell culture. The invention also relates to the use of such compounds in pharmaceutical compositions and therapeutic treatments for rhinoviral infections. The invention further relates to processes for synthesizing such compounds and compounds useful in such syntheses.

### BACKGROUND OF THE INVENTION

The picornaviruses are a family of tiny non-enveloped positive-stranded RNA-containing viruses that infect humans and other animals. These viruses include the human rhinoviruses, human polioviruses, human coxsackieviruses, human echoviruses, human and bovine enteroviruses, encephalomyocarditis viruses, meningitis virus, foot and mouth viruses, hepatitis A virus, and others. The human rhinoviruses are a major cause of the common cold. To date, there are no effective therapies on the market that cure the common cold, only treatments that relieve the symptoms.

Picornaviral infections may be treated by inhibiting the proteolytic 3C enzymes. These enzymes are required for the natural maturation of the picornaviruses. They are responsible for the autocatalytic cleavage of the genomic, large polyprotein into the essential viral proteins. Members of the 3C protease family are cysteine proteases, where the sulfhydryl group most often cleaves the glutamine-glycine amide bond. Inhibition of 3C proteases is believed to block proteolytic cleavage of the polyprotein, which in turn can retard the maturation and replication of the viruses by interfering with viral particle production. Therefore, inhibiting the processing of this cysteine protease with selective small molecules that are specifically recognized should represent an important and useful approach to treat and cure viral infections of this nature and, in particular, the common cold.

Some small-molecule inhibitors of the enzymatic activity of picornaviral 3C proteases (i.e., antipicornaviral compounds) have been recently discovered. WO 97/43305 discloses inhibitors of picornavirus 3C proteases. WO 98/43950 discloses antipicornaviral compounds. WO 99/31122 discloses picornaviral 3C protease inhibitors. Peter John Murray et. al. disclose in Tetrahedron Letters 39 (1958), 6721 - 6724 the enantiospecific synthesis of novel lysine analogues. S. W. Kaldor et al. disclose in Bioorganic and Medicinal Chemistry Letters, Vol. 5, No. 17, 2021-2026 (1995) glutamine derived aldehydes for the inhibition of human rhinovirus 3C protease.

There is still a desire to discover small-molecule compounds that are especially potent antipicornaviral agents.

### SUMMARY OF THE INVENTION

Thus, an object of this invention is to discover small-molecule compounds that are especially potent antipicornaviral agents. A further object of the invention is to provide intermediates useful for the synthesis of said protease-inhibiting compounds and synthetic methods useful for such syntheses. A yet further object of the invention is to achieve pharmaceutical compositions that are highly effective for treating maladies mediated by inhibition of picornaviral 3C proteases, such as the common cold.

Such objects have been attained through the discovery of compounds of the invention, which are picornaviral 3C protease inhibitors displaying particularly strong antiviral activity. It has surprisingly been discovered that peptido and peptidomimetic compounds containing a five-membered heterocyclic group have high rhinoviral-protease-inhibiting activity. It has further been surprisingly found that the rhinoviral-protease-inhibiting activity of peptido and peptidomimetic compounds may be significantly enhanced by replacing a glutamine-like moiety found in some known rhinoviral-protease-inhibiting compounds with a side-chain comprising a gamma- or delta-lactam.

The inhibitors of the present invention are of the following general formula (I): 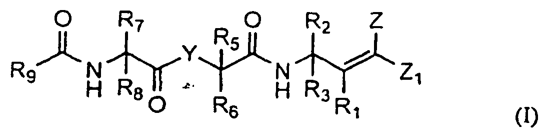 wherein the residues are as defined in claim 1. Preferred embodiments are defined in claims 2 to 64.
The invention also pertains to prodrugs, pharmaceutically acceptable salts, pharmaceutically active metabolites, and pharmaceutically acceptable solvates of compounds of the formula I.

In preferred embodiments of the compounds of the formula I, R₂ and R₃ are each independently H; 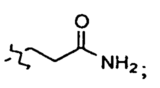 or 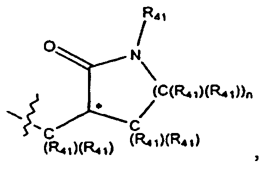 where n is an integer from 0 to 5, each R₄₁ is independently H or lower alkyl, and the stereochemistry at the carbon denoted with an asterisk may be R or S; provided that at least one of R₂ and R₃ is

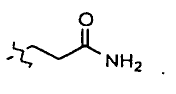

Preferably, R₉ is a five-membered heterocycle having one to three heteroatoms selected from O, N, and S.

In other preferred embodiments, the variables of formula I are as follows. Z and Z₁ are each independently selected from H, F, lower alkyl, -CO₂R₂₁, and -C(O)NR₂₁R₂₂, provided that Z and Z₁ are not both H, where R₂₁ and R₂₂ are each independently H, an alkyl group, a cycloalkyl group, a heterocycloalkyl group, an aryl group, a heteroaryl group, an acyl group, or a thioacyl group, or R₂₁ and R₂₂, together with the atom(s) to which they are bonded, form a heterocycloalkyl group. At least one of R₂ or R₃ is 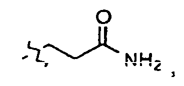 and the other is H. R₅ and R₆ are each independently selected from H, F, an alkyl group, a cycloalkyl group, a heterocycloalkyl group, an aryl group, and a heteroaryl group, more preferably one of R₅ and R₆ is H and the other is alkyl or aryl (e.g., unsubstituted or substituted phenylmethyl). R₇ and R₈ are each independently H, an alkyl group, a cycloalkyl group, a heterocycloalkyl group, an aryl group, or a heteroaryl group; and more preferably one of R₇ and R₈ is H and the other is alkyl (e.g., 2-propyl, 2-methyl-2-propyl, or 2-methyl-1-propyl) or arylmethyl (e.g., unsubstituted or substituted phenylmethyl or naphthylmethyl). R₉ is a five-membered heterocycle having from one to three heteroatoms selected from O, N, and S, more preferably a five-membered heterocycle having at least one nitrogen heteroatom and at least one oxygen heteroatom (e.g., unsubstituted or substituted 1,2-oxazolyl (i.e., isoxazolyl), 1,3-oxazolyl (i.e., oxazolyl), or oxadiazolyl (1,2,3-oxadiazolyl, 1,2,4-oxadiazolyl, or 1,2,5-oxadiazolyl). When R₉ is oxadiazolyl, unsubstituted and monomethyl-substituted 1,2,4-oxadiazolyl are preferred. In especially preferred embodiments, R₉ is 3-isoxazolyl or 5-isoxazolyl, either unsubstituted or substituted with one or two methyl groups and/or halogens, with chlorine and fluorine being preferred halogen substituents.

In a preferred embodiment, the compounds, prodrugs, pharmaceutically acceptable salts, pharmaceutically active metabolites, and solvates have an antipicornaviral activity with an EC₅₀ less than or equal to 100 µM in the H1-HeLa cell culture assay, and more preferably an antirhinoviral activity with an EC₅₀ less than or equal to 10 µM in the H1-HeLa cell culture.

The invention also relates to pharmaceutical compositions containing a therapeutically effective amount of at least one compound of the formula I, or a pharmaceutically acceptable salt, or solvate thereof. Additionally, the invention relates to methods of inhibiting picornaviral 3C protease by administering a therapeutically effective amount of at least one compound of the formula I, or a pharmaceutically acceptable salt, or solvate thereof.

### DETAILED DESCRIPTION AND PREFERRED EMBODIMENTS

The present invention relates to compounds of the formula I: 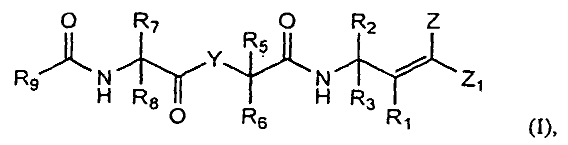 wherein Y, R₁, R₂, R₃, R₅, R₆, R₇, R₈, R₉, Z, and Z₁ are as defined above, and to pharmaceutically acceptable salts, and solvates thereof. Preferably, such compounds, pharmaceutically acceptable salts, and solvates have antipicornaviral activity, more preferably antirhinoviral activity, corresponding to an EC₅₀ less than or equal to 100 µM in the H1-HeLa cell culture assay, more preferably corresponding to an EC₅₀ less than or equal to 10 µM in the H1-HeLa cell culture assay.

The present invention additionally relates to preferred compounds of the formulas I-A, I-B, and I-C: 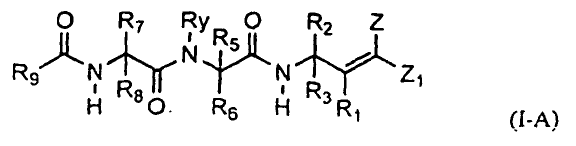 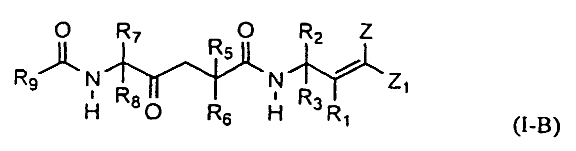 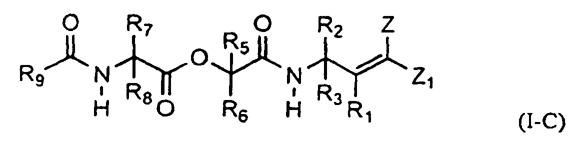 wherein R_{y} (in formula I-A) is H or lower alkyl, and R₁, R₂, R₃, R₅, R₆, R₇, R₈, R₉, Z, and Z₁ are as defined above, and to pharmaceutically acceptable salts, and solvates thereof.

The inventive compounds of formulas I-A, which are referred to herein as "peptide-like" compounds, I-B, which are referred to herein as "ketomethylene-type" compounds, and I-C, which are referred to herein as "depsipeptide" compounds, differ in their backbones, which may affect the specific biodistribution or other physical properties; nonetheless each possesses a strong rhinoviral-protease-inhibiting activity.

In preferred embodiments of compounds of formulas I-A, I-B, and I-C above:
R₁ is H, F, or an alkyl group;
R_{y} (in formula I-A) is H or methyl;
R₃, R₅, and R₈ are each H;
R₂ is 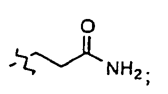
R₆ is an alkyl group, which has as a preferred optional substituent an aryl group;
R₇ is an alkyl group, a cycloalkyl group, a heterocycloalkyl group, an aryl group, or a heteroaryl group;
R₉ is a five-membered heterocycle having from one to three heteroatoms selected from O, N, and S, preferably where at least one of the heteroatoms is nitrogen, that is unsubstituted or substituted, where the optional substituents are preferably halogen or lower alkyl, and more preferably mono-chloro or -fluoro or a methyl group; and
Z and Z₁ are each independently H, F, an alkyl group, a cycloalkyl group, a heterocycioaikyi group, an aryl group, a heteroaryl group, -C(O)R₂₁, -CO₂R₂₁, -CN, -C(O)NR₂₁R₂₂, -C(O)NR₂₁OR₂₂, -C(S)R₂₁, -C(S)NR₂₁R₂₂, -NO₂, -SOR₂₁, -SO₂R₂₁, -SO₂NR₂₁R₂₂, -SO(NR₂₁)(OR₂₂), -SONR₂₁, -SO₃R₂₁, -PO(OR₂₁)₂, -PO(R₂₁)(R₂₂), -PO(NR₂₁R₂₂)(OR₂₃), -PO(NR₂₁R₂₂)(NR₂₃R₂₄), -C(O)NR₂₁NR₂₂R₂₃, or -C(S)NR₂₁NR₂₂R₂₃, where Z and Z₁ are not both H, and where R₂₁, R₂₂, R₂₃, and R₂₄ are each independently H, an alkyl group, a cycloalkyl group, a heterocycloalkyl group, an aryl group, a heteroaryl group, an acyl group, or a thioacyl group, or where any two of R₂₁, R₂₂, R₂₃, and R₂₄, together with the atom(s) to which they are bonded, form a heterocycloalkyl group,
or Z and Z₁ (both as defined above), together with the atoms to which they are attached, form a heterocycloalkyl group.

In preferred embodiments, the RVP-inhibiting agents of the invention are compounds of any of the stereospecific formulas I-A", I-B", and I-C": 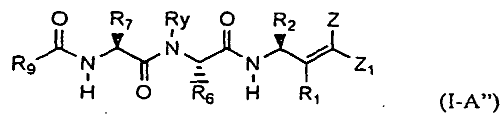 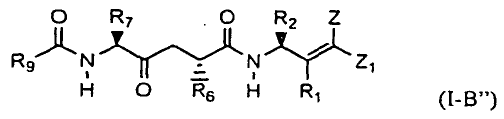 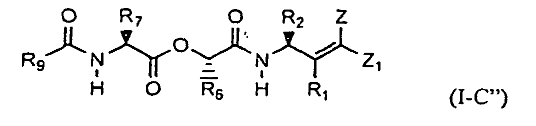 wherein R_{y}, R₁, R₂, R₆, R₇, R₉, Z, and Z₁ are as defined above, and pharmaceutically acceptable salts, prodrugs, active metabolites, and solvates thereof.

In preferred embodiments of compounds of the formula I-A", I-B", or I-C":
R₁ is H, F, or methyl;
R_{y} (in formula I-A') is H or methyl;
R₂ is 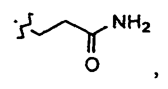
R₆ is arylmethyl or arylthiomethyl, where aryl is preferably an optionally substituted phenyl group;
R₇ is an alkyl group, more preferably selected from 2-propyl, 2-methyl-2-pronyl, 2-methyl-1-propyl, and arylmethyl, where the aryl group is preferably phenyl or naphthyl;
R₉ is isoxazolyl, oxazolyl, or oxadiazolyl, optionally substituted with one or two lower alkyl groups and/or halogens; and
Z is H, and Z₁ is -CO₂R₂₁, -CN, or -C(O)NR₂₁R₂₂, where R₂₁ and R₂₂ are as defined above, or Z and Z₁ together form a cyclic ester or amide.

In accordance with a convention used in the art, 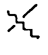 is used in structural formulas herein to depict the bond that is the point of attachment of the moiety or substituent to the core or backbone structure.

Where chiral carbons are included in chemical structures, unless a particular orientation is depicted, both stereoisomeric forms are intended to be encompassed.

As used herein, the term "alkyl group" is intended to mean a straight- or branched-chain monovalent radical of saturated and/or unsaturated carbon atoms and hydrogen atoms, such as methyl (Me), ethyl (Et), propyl, isopropyl, butyl, isobutyl, t-butyl, ethenyl, pentenyl, butenyl, propenyl, ethynyl, butynyl, propynyl, pentynyl, hexynyl, and the like, which may be unsubstituted (i.e., containing only carbon and hydrogen) or substituted by one or more suitable substituents as defined below (e.g., one or more halogens, such as F, Cl, Br, or I, with F and Cl being preferred). A "lower alkyl group" is intended to mean an alkyl group having from 1 to 4 carbon atoms in its chain.

A "cycloalkyl group" is intended to mean a non-aromatic monovalent monocyclic, bicyclic, or tricyclic radical containing 3, 4, 5, 6, 7, 8, 9, 10, 11, 12, 13, or 14 carbon ring atoms, each of which may be saturated or unsaturated, and which may be unsubstituted or substituted by one or more suitable substituents as defined below, and to which may be fused one or more heterocycloalkyl groups, aryl groups, or heteroaryl groups, which themselves may be unsubstituted or substituted by one or more substituents. Illustrative examples of cycloalkyl groups include the following moieties:

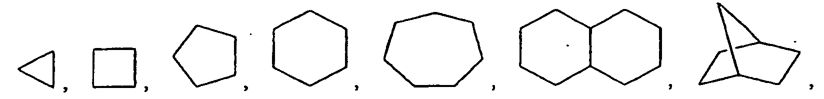

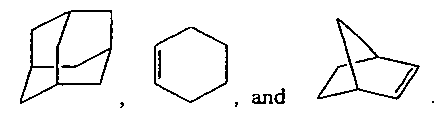

A "heterocycloalkyl group" is intended to mean a non-aromatic monovalent monocyclic, bicyclic, or tricyclic radical, which is saturated or unsaturated, containing 3, 4, 5, 6, 7, 8, 9, 10, 1 1, 12, 13, 14, 15, 16, 17, or 18 ring atoms, which includes 1, 2, 3, 4, or 5 heteroatoms selected from nitrogen, oxygen, and sulfur, where the radical is unsubstituted or substituted by one or more suitable substituents as defined below, and to which may be fused one or more cycloalkyl groups, aryl groups, or heteroaryl groups, which themselves may be unsubstituted or substituted by one or more suitable substituents. Illustrative examples of heterocycloalkyl groups include the following moieties:

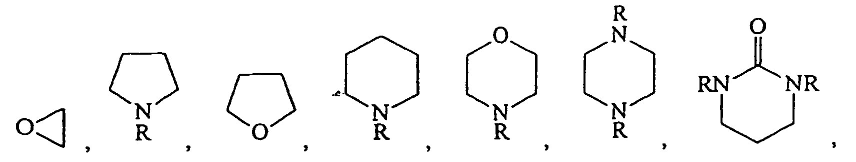

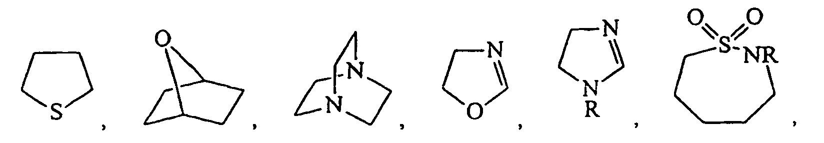

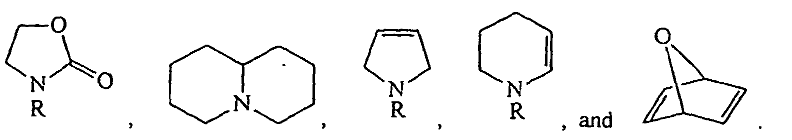

An "aryl group" is intended to mean an aromatic monovalent monocyclic, bicyclic, or tricyclic radical containing 6, 10, 14, or 18 carbon ring atoms, which may be unsubstituted or substituted by one or more suitable substituents as defined below, and to which may be fused one or more cycloalkyl groups, heterocycloalkyl groups, or heteroaryl groups, which themselves may be unsubstituted or substituted by one or more suitable substituents. Illustrative examples of aryl groups include the following moieties:

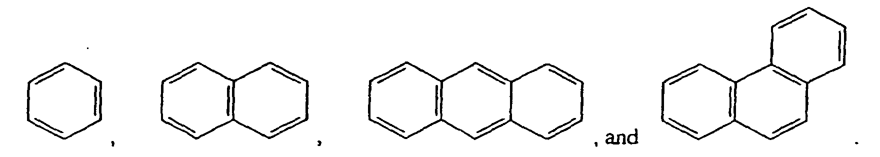

A "heteroaryl group" is intended to mean an aromatic monovalent monocyclic, bicyclic, or tricyclic radical containing 4, 5, 6, 7, 8, 9, 10, 11, 12, 13, 14, 15, 16, 17, or 18 ring atoms, including 1, 2, 3, 4, or 5 heteroatoms selected from nitrogen, oxygen, and sulfur, which may be unsubstituted or substituted by one or more suitable substituents as defined below, and to which may be fused one or more cycloalkyl groups, heterocycloalkyl groups, or aryl groups, which themselves may be unsubstituted or substituted by one or more suitable substituents. Illustrative examples of heteroaryl groups include the following moieties:

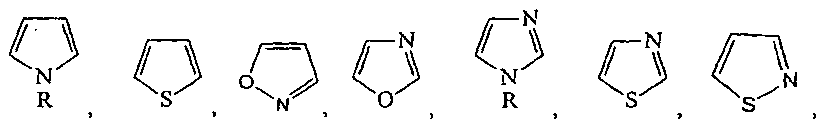

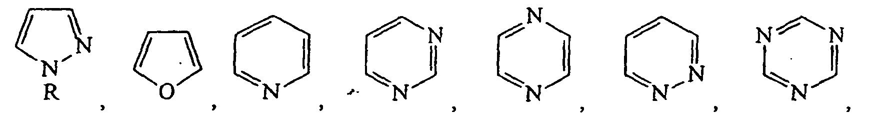

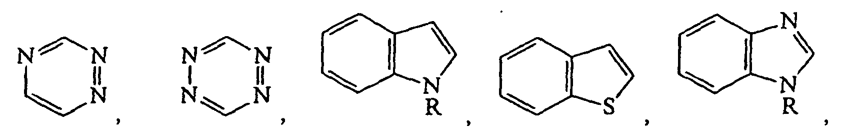

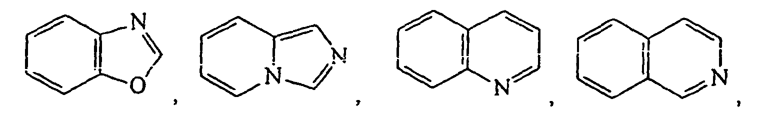

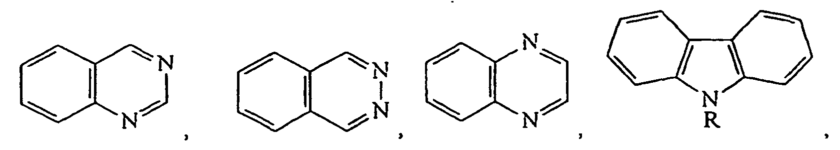

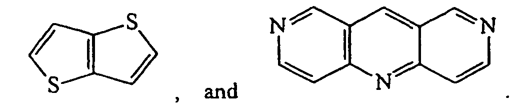

A "heterocycle" is intended to mean a heteroaryl or heterocycloalkyl group (each of which, as defined above, are optionally substituted).

An "acyl group" is intended to mean a -C(O)-R radical, where R is a substituent as defined below.

A "thioacyl group" is intended to mean a -C(S)-R radical, where R is a substituent as defined below.

A "sulfonyl group" is intended to mean a -SO₂R radical, where R is a substituent as defined below.

A "hydroxy group" is intended to mean the radical -OH.

An "amino group" is intended to mean the radical -NH₂.

An "alkylamino group" is intended to mean the radical -NHRₐ, where Rₐ is an alkyl group.

A "dialkylamino group" is intended to mean the radical -NRₐR_{b}, where Rₐ and R_{b} are each independently an alkyl group.

An "alkoxy group" is intended to mean the radical -ORₐ, where Rₐ is an alkyl group. Exemplary alkoxy groups include methoxy, ethoxy, propoxy, and the like.

An "alkoxycarbonyl group" is intended to mean the radical -C(O)ORₐ, where Rₐ is an alkyl group.

An "alkylsulfonyl group" is intended to mean the radical -SO₂Rₐ, where Rₐ is an alkyl group.

An "alkylaminocarbonyl group" is intended to mean the radical -C(O)NHRₐ, where Rₐ is an alkyl group.

A "dialkylaminocarbonyl group" is intended to mean the radical -C(O)NRₐR_{b}, where Rₐ and R_{b} are each independently an alkyl group.

A "mercapto group" is intended to mean the radical -SH.

An "alkylthio group" is intended to mean the radical -SRₐ, where Rₐ is an alkyl group.

A "carboxy group" is intended to mean the radical -C(O)OH.

A "carbamoyl group" is intended to mean the radical -C(O)NH₂.

An "aryloxy group" is intended to mean the radical -OR_{c}, where R_{c} is an aryl group.

A "heteroaryloxy group" is intended to mean the radical -OR_{d}, where R_{d} is a heteroaryl group.

An "arylthio group" is intended to mean the radical -SR_{c}, where R_{c} is an aryl group.

A "heteroarylthio group" is intended to mean the radical -SR_{d}, where R_{d} is a heteroaryl group.

The term "suitable organic moiety" is intended to mean any organic moiety recognizable, such as by routine testing, to those skilled in the art as not adversely affecting the inhibitory activity of the inventive compounds. Illustrative examples of suitable organic moieties include, but are not limited to, hydroxy groups, alkyl groups, oxo groups, cycloalkyl groups, heterocycloalkyl groups, aryl groups, heteroaryl groups, acyl groups, sulfonyl groups, mercapto groups, alkylthio groups, alkoxy groups, carboxy groups, amino groups, alkylamino groups, dialkylamino groups, carbamoyl groups, arylthio groups, heteroarylthio groups, and the like.

The term "substituent" or "suitable substituent" is intended to mean any suitable substituent that may be recognized or selected, such as through routine testing, by those skilled in the art. Illustrative examples of suitable substituents include hydroxy groups, halogens, oxo groups, alkyl groups, acyl groups, sulfonyl groups, mercapto groups, alkylthio groups, alkoxy groups, cycloalkyl groups, heterocycloalkyl groups, aryl groups, heteroaryl groups, carboxy groups, amino groups, alkylamino groups, dialkylamino groups, carbamoyl groups, aryloxy groups, heteroaryloxy groups, arylthio groups, heteroarylthio groups, and the like.

The term "optionally substituted" is intended to expressly indicate that the specified group is unsubstituted or substituted by one or more suitable substituents, unless the optional substituents are expressly specified, in which case the term indicates that the group is unsubstituted or substituted with the specified substituents. As defined above, various groups may be unsubstituted or substituted (i.e., they are optionally substituted) unless indicated otherwise herein (e.g., by indicating that the specified group is unsubstituted).

A "solvate" is intended to mean a pharmaceutically acceptable solvate form of a specified compound that retains the biological effectiveness of such compound. Examples of solvates include compounds of the invention in combination with water, isopropanol, ethanol, methanol, DMSO, ethyl acetate, acetic acid, or ethanolamine.

A "pharmaceutically acceptable salt" is intended to mean a salt that retains the biological effectiveness of the free acids and bases of the specified compound and that is not biologically or otherwise undesirable. Examples of pharmaceutically acceptable salts include sulfates, pyrosulfates, bisulfates, sulfites, bisulfites, phosphates, monohydrogenphosphates, dihydrogenphosphates, metaphosphates, pyrophosphates, chlorides, bromides, iodides, acetates, propionates, decanoates, caprylates, acrylates, formates, isobutyrates, caproates, heptanoates, propiolates, oxalates, malonates, succinates, suberates, sebacates, fumarates, maleates, butyne-1,4-dioates, hexyne-1,6-dioates, benzoates, chlorobenzoates, methylbenzoates, dinitrobenzoates, hydroxybenzoates, methoxybenzoates, phthalates, sulfonates, xylenesulfonates, phenylacetates, phenylpropionates, phenylbutyrates, citrates, lactates, γ-hydroxybutyrates, glycollates, tartrates, methane-sulfonates, propanesulfonates, naphthalene-I-sulfonates, naphthalene-2-sulfonates, and mandelates.

If an inventive compound is a base, a desired salt may be prepared by any suitable method known to the art, including treatment of the free base with an inorganic acid, such as hydrochloric acid; hydrobromic acid; sulfuric acid; nitric acid; phosphoric acid; and the like, or with an organic acid, such as acetic acid; maleic acid; succinic acid; mandelic acid; fumaric acid; malonic acid; pyruvic acid; oxalic acid; glycolic acid; salicylic acid; pyranosidyl acid, such as glucuronic acid or galacturonic acid; alpha-hydroxy acid, such as citric acid or tartaric acid; amino acid, such as aspartic acid or glutamic acid; aromatic acid, such as benzoic acid or cinnamic acid; sulfonic acid, such as p-toluenesulfonic acid or ethanesulfonic acid; or the like.

If an inventive compound is an acid, a desired salt may be prepared by any suitable method known to the art, including treatment of the free acid with an inorganic or organic base, such as an amine (primary, secondary, or tertiary); an alkali metal or alkaline earth metal hydroxide; or the like. Illustrative examples of suitable salts include organic salts derived from amino acids such as glycine and arginine; ammonia; primary, secondary, and tertiary amines; and cyclic amines, such as piperidine, morpholine, and piperazine; as well as inorganic salts derived from sodium, calcium, potassium, magnesium, manganese, iron, copper, zinc, aluminum, and lithium.

In the case of compounds, salts, or solvates that are solids, it is understood by those skilled in the art that the inventive compounds, salts, and solvates may exist in different crystal forms, all of which are intended to be within the scope of the present invention and specified formulas.

The inventive compounds may exist as single stereoisomers, racemates, and/or mixtures of enantiomers and/or diastereomers. All such single stereoisomers, racemates, and mixtures thereof are intended to be within the broad scope of the present invention. Preferably, however, the inventive compounds are used in optically pure form.

As generally understood by those skilled in the art, an optically pure compound is one that is enantiomerically pure. As used herein, the term "optically pure" is intended to mean a compound comprising at least a sufficient activity. Preferably, an optically amount of a single enantiomer to yield a compound having the desired pharmacological pure compound of the invention comprises at least 90% of a single isomer (80% enantiomeric excess), more preferably at least 95% (90% e.e.), even more preferably at least 97.5% (95% e.e.), and most preferably at least 99% (98% e.e.).

Preferably in the compounds of the formula I (or of any of the subgeneric formula), R₁ is H or F.

In the compounds of formula I, preferably R₉ is an unsubstituted or substituted isoxazolyl group, where the optional substituents are preferably one or two methyl groups and/or halogens.

Especially preferred embodiments of the invention are described below in reference to the following formula I-A":

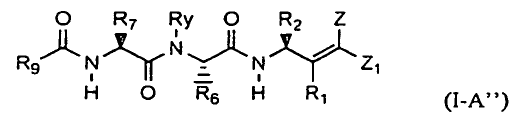

Preferred compounds of the present invention include peptido (peptide-like) Compounds **(A-1)** - **(A-8)** of the formula I-A'' above, wherein R₁ is H, Z is H, R_{y} is H, and R₂, R₆, R₇, Z₁, and R₉ are as respectively defined below:
**(A-1)** R₂ is CH₂CH₂C(O)NH₂, R₆ is CH₂Ph, R₇ is CH₂CH(CH₃)₂, Z₁ is CO₂CH₂CH₃, and R₉ is 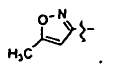
**(A-2)** R₂ is CH₂CH₂C(O)NH₂, R₆ is CH₂Ph, R₇ is CH₂CH(CH₃)₂, Z₁ is CO₂CH₂CH₃, and R₉ is 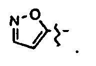
**(A-3)** R₂ is CH₂CH₂C(O)NH₂, R₆ is 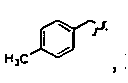 R₇ is C(CH₃)₃, Z₁ is CO₂CH₂CH₃, and R₉ is 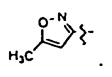
**(A-4)** R₂ is CH₂CH₂C(O)NH₂, R₆ is 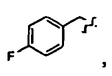 R₇ is C(CH₃)₃, Z₁ is CO₂CH₂CH₃, and R₉ is 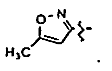
**(A-6)** R₂ is CH₂CH₂C(O)NH₂, R₆ is 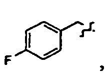 R₇ is CH(CH₃)₂, Z₁ is CO₂CH₂CH₃, and R₉ is

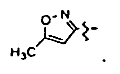

Preferred peptide-like compounds of the formula I-A'''' further include Compounds **(A-9)** - **(A-13)** below, wherein R₁ is H, Z is H, Z₁ is CO₂CH₂CH₃, R_{y} is CH₃, and R₂, R₆, R₇, and R₉ are as respectively defined below:
**(A-9)** R₂ is CH₂CH₂C(O)NH₂, R₆ is 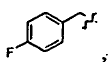 R₇ is 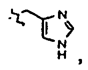 and R₉ is 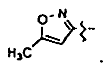
**(A-10)** R₂ is CH₂CH₂C(O)NH₂, R₆ is CH₂Ph, R₇ is CH₂CH(CH₃)₂, and R₉ is 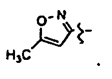
**(A-11)** R₂ is CH₂CH₂C(O)NH₂, R₆ is 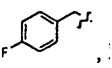 R₇ is 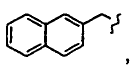 and R₉ is 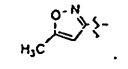
**(A-12)** R₂ is CH₂CH₂C(O)NH₂, R₆ is 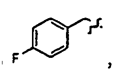 R₇ is CH₂CH(CH₃)₂, and R₉ is 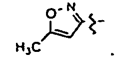
**(A-13)** R₂ is CH₂CH₂C(O)NH₂, R₆ is 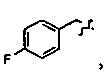 R₇ is 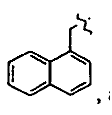 and R₉ is

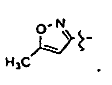

Other preferred peptide-like compounds include the following:

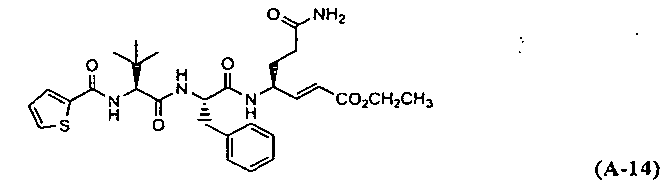

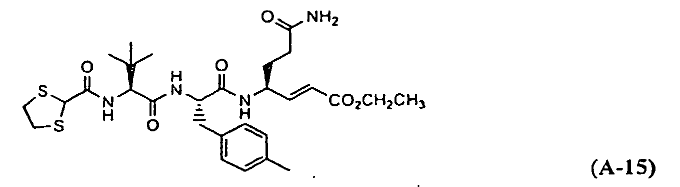

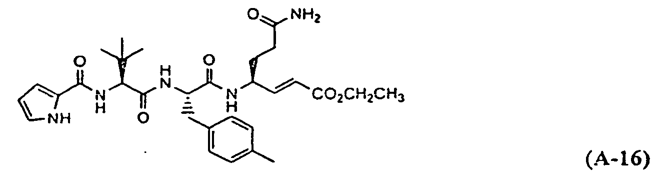

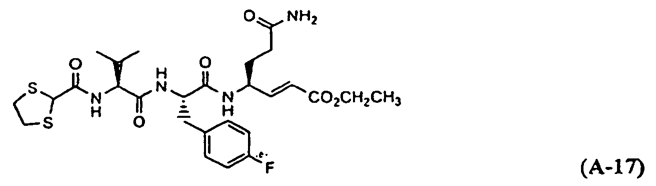

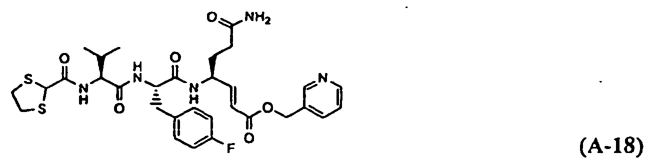

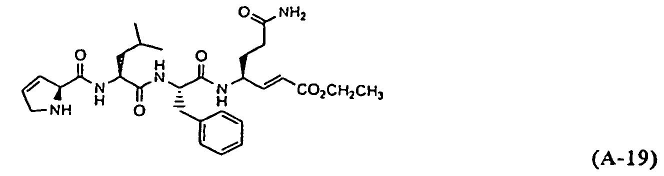

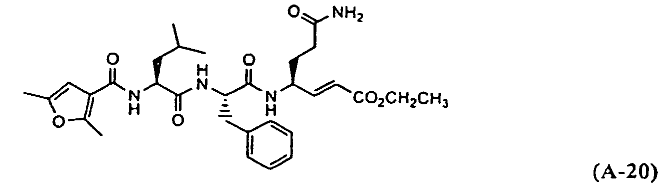

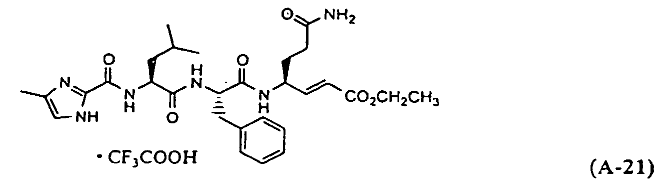

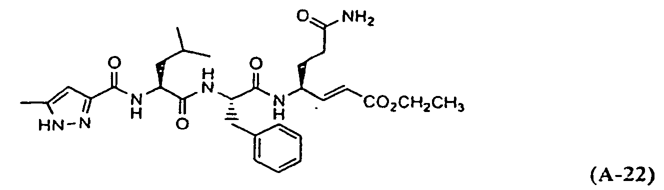

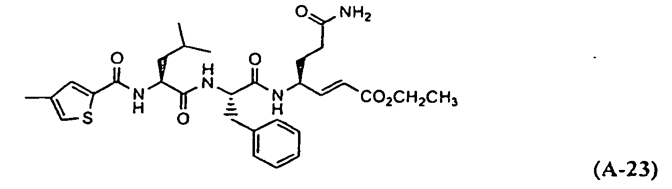

Preferred ketomethylene-type Compounds **(B-1)** - **(B-4)** of the invention are described below in reference to the following formula I-B": 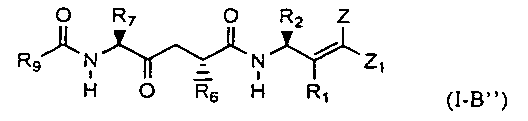
**(B-1)** R₂ is CH₂CH₂C(O)NH₂, R₆ is 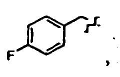 R₇ is CH(CH₃)₂, Z is H, Z₁ is CO₂CH₂CH₃, and R₉ is

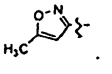

Preferred depsipeptide-type Compounds **(C-1)** and **(C-2)** of the invention are described below in reference to the following formula I-C", where R₁ is H: 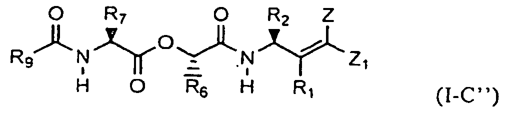
**(C-1)** Z is H, R₂ is CH₂CH₂C(O)NH₂, R₆ is 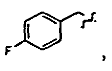 R₇ is CH(CH₃)₂, Z₁ is CO₂CH₂CH₃, and R₉ is

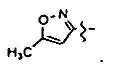

Additional compounds may be prepared in reference to formula I by selecting the variables from the following substituents:
R_{y} = H or CH₃;
R₁ = H or CH₃;
R₂ = 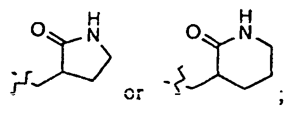
R₆ = 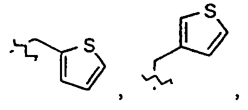 or phenylmethyl (i.e., benzyl), where the aryl group is optionally substituted with one, two, or three substituents each independently selected from halogens, methoxy and methyl;
R₇ = 2-methyl-1-propyl, 2-propyl, 2-methyl-2-propyl, benzyl, or 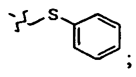 and
R₉ =

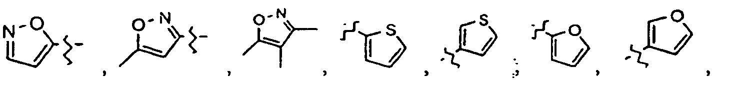

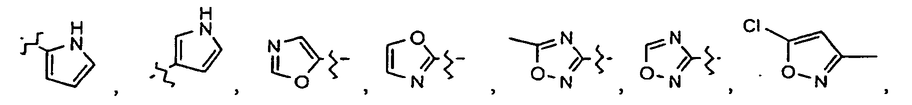

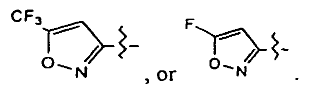

The present invention is also directed to a method of inhibiting picornaviral 3C protease activity, comprising contacting the protease with an effective amount of a compound of formula I, or a pharmaceutically acceptable salt, prodrug, pharmaceutically active metabolite, or solvate thereof. For example, picornaviral 3C protease activity may be inhibited in mammalian tissue by administering a compound of formula I or a pharmaceutically acceptable salt, prodrug, pharmaceutically active metabolite, or solvate thereof. More preferably, the present method is directed at inhibiting rhinoviral protease activity.

"Treating" or "treatment" is intended to mean at least the mitigation of a disease condition in a mammal, such as a human, that is alleviated by the inhibition of the activity of one or more picornaviral 3C proteases, such as human rhinoviruses, human poliovirus, human coxsackieviruses, encephalomyocarditis viruses, meningitis virus, and hepatitis A virus, and includes: (a) prophylactic treatment in a mammal, particularly when the mammal is found to be predisposed to having the disease condition but not yet diagnosed as having it; (b) inhibiting the disease condition; and/or (c) alleviating, in whole or in part, the disease condition.

The activity of the inventive compounds as inhibitors of picornaviral 3C protease activity may be measured by any of the suitable methods known to those skilled in the art, including *in vivo* and *in vitro* assays. An example of a suitable assay for activity measurements is the antiviral H1-HeLa cell culture assay described herein.

Administration of the compounds of the formula I and their pharmaceutically acceptable prodrugs, salts, active metabolites, and solvates may be performed according to any of the accepted modes of administration available to those skilled in the art. Illustrative examples of suitable modes of administration include oral, nasal, parenteral, topical, transdermal, and rectal. Intranasal delivery is especially preferred.

An inventive compound of formula I or a pharmaceutically acceptable salt, prodrug, active metabolite, or solvate thereof may be administered as a pharmaceutical composition in any pharmaceutical form recognizable to the skilled artisan as being suitable. Suitable pharmaceutical forms include solid, semisolid, liquid, or lyophilized formulations, such as tablets, powders, capsules, suppositories, suspensions, liposomes, and aerosols.
Pharmaceutical compositions of the invention may also include suitable excipients, diluents, vehicles, and carriers, as well as other pharmaceutically active agents, depending upon the intended use. In preferred embodiments, the inventive pharmaceutical compositions are delivered intranasally in the form of suspensions.

Acceptable methods of preparing suitable pharmaceutical forms of the pharmaceutical compositions are known or may be routinely determined by those skilled in the art. For example, pharmaceutical preparations may be prepared following conventional techniques of the pharmaceutical chemist involving steps such as mixing, granulating, and compressing when necessary for tablet forms, or mixing, filling, and dissolving the ingredients as appropriate, to give the desired products for oral, parenteral, topical, intravaginal, intranasal, intrabronchial, intraocular, intraaural, and/or rectal administration.

Solid or liquid pharmaceutically acceptable carriers, diluents, vehicles, or excipients may be employed in the pharmaceutical compositions. Illustrative solid carriers include starch, lactose, calcium sulfate dihydrate, terra alba, sucrose, talc, gelatin, pectin, acacia, magnesium stearate, and stearic acid. Illustrative liquid carriers include syrup, peanut oil, olive oil, saline solution, and water. The carrier or diluent may include a suitable prolonged-release material, such as glyceryl monostearate or glyceryl distearate, alone or with a wax. When a liquid carrier is used, the preparation may be in the form of a syrup, elixir, emulsion, soft gelatin capsule, sterile injectable liquid (e.g., solution), or a nonaqueous or aqueous liquid suspension.

A dose of the pharmaceutical composition contains at least a therapeutically effective amount of the active compound (i.e., a compound of formula I or a pharmaceutically acceptable salt, prodrug, active metabolite, or solvate thereof), and preferably is made up of one or more pharmaceutical dosage units. The selected dose may be administered to a mammal, for example, a human patient, in need of treatment mediated by inhibition of picornaviral 3C protease activity, by any known or suitable method of administering the dose, including topically, for example, as an ointment or cream; orally; rectally, for example, as a suppository; parenterally by injection; or continuously by intravaginal, intranasal, intrabronchial, intraaural, or intraocular infusion.

A "therapeutically effective amount" is intended to mean the amount of an inventive compound that, when administered to a mammal in need thereof, is sufficient to effect treatment for disease conditions alleviated by the inhibition of the activity of one or more picornaviral 3C proteases, such as human rhinoviruses, human poliovirus, human coxsackieviruses, encephalomyocarditis viruses, menigovirus, and hepatitis A virus. The amount of a given compound of the invention that will be therapeutically effective will vary depending upon factors such as the particular compound, the disease condition and the severity thereof, the identity of the mammal in need thereof, which amount may be routinely determined by artisans.

By way of illustration, a formulation for nasal delivery of the inventive compounds for treatment of rhinoviral infections can be prepared as follows, where all percentages are weight/weight and the suspension is prepared in purified water. A formula-I compound is micronized to a reduced particle size such that D₉₀ < 10 µm. A suspension is prepared to contain a final concentration of from about 0.01 % to about 2% of the active compound, preferably about from 0.2% to 2%. An appropriate preservative selected from those known in the art may be included, for example, benzalkonium chloride/EDTA, in appropriate final-concentration ranges, e.g., about 0.02%/0.01%. A suspending agent, such as mixture of microcrystalline cellulose (final concentration of about 1% - 1.5%, preferably about 1.2%) and sodium carboxymethylcellulose cellulose (final concentration of about 0.1% - 0.2%, preferably about 0.13%) may be included. A surfactant such as polysorbate 80 may be included in a final concentration of about from 0.05% to 0.2%, preferably about 0.1 %. A tonicity modifier such as dextrose may be included to give a final concentration of about from 4% to 6%, preferably about 5%. The pH of the final solution is adjusted as appropriate to a physiological range, e.g., 4-6, using non-toxic acid and/or base, such as HCl and/or NaOH.

An exemplary formulation for nasal delivery of the inventive compound of Example 17 has the following composition, where all percentages are weight/weight and the suspension is prepared in purified water:

| | | |
|---|---|---|
| Active Compound | **(B-2)** | 0.2 - 2 % |
| Preservative | Benzalkortium chloridelEDTA | 0.02 % / 0.01 % |
| Suspending Agent | Microcrystalline cellulose/Nacarboxymethylcellulose | 1.2%/0.13% |
| Surfactant | Polysorbate 80 | 0.1 % |
| Tonicity Modifier | Dextrose | 5% |
| pH Adjustment | NaOH/HCl | pH 4 - 6 |

### GENERAL SYNTHESES

The inventive compounds of formula (I) may be advantageously prepared by the methods of the present invention, including the general methods described below. In each of these general methods, R₁, R₂, R₃, R₅, R₆, R₇, R₈, R₉, R_{y}, Z, and Z ₁ are as defined above, and R₄ is used (as a shorthand representation) to mean: 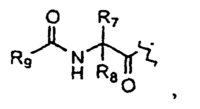 where R₇, R₈, and R₉ are as defined above.

In General Method I, useful for synthesis of peptide-like compounds of formula I-A, an amino acid **A**, where P₁ is an appropriate protecting group for nitrogen, is subjected to an amide-forming reaction with amino alcohol (or salt thereof) **B** to produce amide **C**. Compound **C** is then deprotected to give free amine (or salt thereof) **D**. Amine **D** and amino acid **E**, which may incorporate either an R₄ group or a protecting group for nitrogen (P₂), are subjected to a bond-forming reaction generating compound **F**. Compound **F** is oxidized to intermediate **G**, which is then transformed into unsaturated product **H**. If protecting groups have been used on amino acid **E**, or on any R groups (R₁-R₉) and/or on R_{y} and/or on Z and/or Z₁, product **H** is deprotected and/or further modified to yield deprotected or modified **H**.

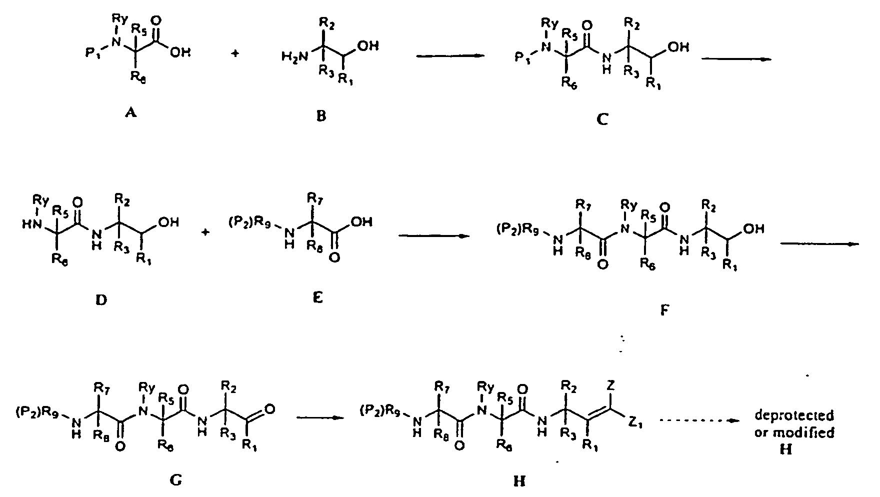

An alternative method to prepare intermediate **F** is described as follows. Amino acid **E** and amino acid (or salt thereof) **I,** where P₃ is an appropriate protecting group for oxygen, are subjected to a bond-forming reaction to produce intermediate **J.** Molecule **J** is deprotected to yield free carboxylic acid **K,** which is subsequently subjected to an amide-forming reaction with amino alcohol (or salt thereof) **B** to genérate intermediate **F.**

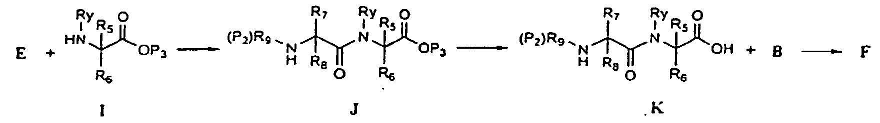

In General Method II, which is also useful for synthesizing peptide-like compounds of formula I-A, an amino acid **L,** where P₁ is an appropriate protecting group for nitrogen, is converted to a carbonyl derivative **M,** where "Lv" is a leaving group. Compound **M** is subjected to a reaction where Lv is replaced by R₁ to give derivative **N.** Derivative **N** is then transformed into unsaturated product **O**. Unsaturated compound **O** is deprotected to give free amine (or salt thereof) **P,** or modified at Z or Z₁ first to give **O'** and then deprotected to **P.** Intermediate **P** is subsequently subjected to an amide-forming reaction with carboxylic acid **K** to give final product **H.** If protecting groups have been used on any R group (R₁-R₉) and/or on R_{y} and/or on Z and/or Z₁, product **H** is deprotected and/or further modified to yield deprotected or modified **H.**

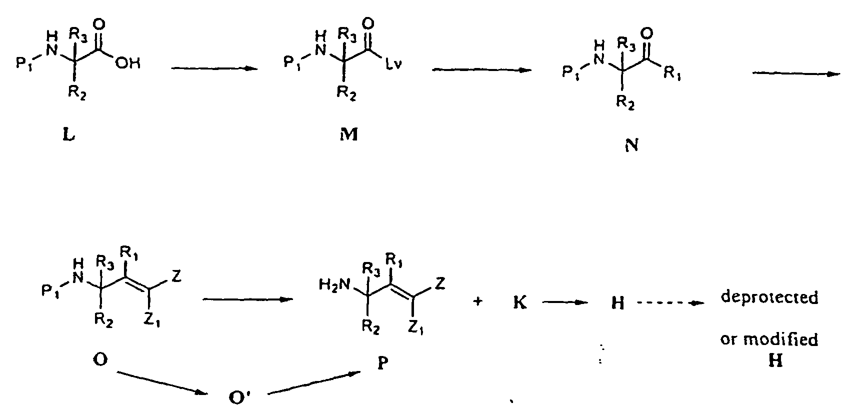

An alternative method to prepare intermediate **N** is described as follows. Compound **M** is subjected to a reaction where "Lv" is reduced to protected amino alcohol **Q**. Intermediate **Q** is subsequently oxidized to derivative **N**.

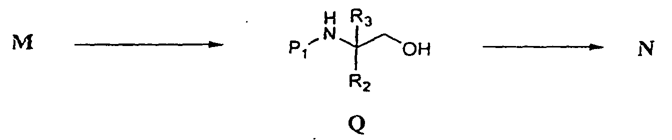

In General Method III, useful for synthesis of peptide-like compounds of formula I-A, an amino acid L, where P₁ is an appropriate protecting group for nitrogen, is converted to a carbonyl derivative **M**, where "Lv" is a leaving group. Compound **M** is deprotected to give free amine (or salt thereof) **R**, which subsequently is subjected to an amide-forming reaction with carboxylic acid **K** to give intermediate **S**. Compound **S** is then either directly converted to carbonyl intermediate **G**, or successively reduced to alcohol F first, which is oxidized to **G**. Compound **G** is subjected to a reaction to yield the unsaturated final product **H**. If protecting groups have been used on any R groups (R₁-R₉) and/or on R_{y} and/or on Z and/or Z₁, product **H** is deprotected and/or further modified to yield deprotected or modified **H**.

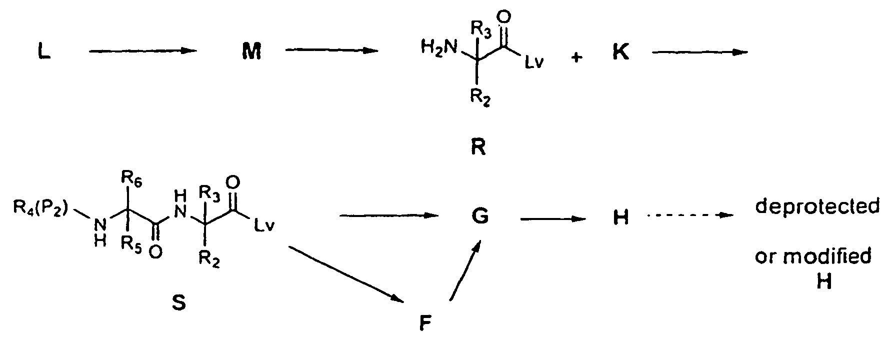

In General Method IV, useful for synthesis of peptide-like compounds of formula I-A, free amine (or salt thereof) **P**, prepared from intermediate **O** as described in General Method II, is converted to amide T by reaction with amino acid **A**, where P₁ is an appropriate protecting group for nitrogen. Compound **T** is further deprotected to free amine (or salt thereof) **U**, which is subsequently converted to **H** with amino acid **E**. If protecting groups have been used on any R groups (R₁-R₉) and/or on R_{y} and/or on Z and/or Z₁, product **H** is deprotected and/or further modified to yield deprotected or modified **H.**

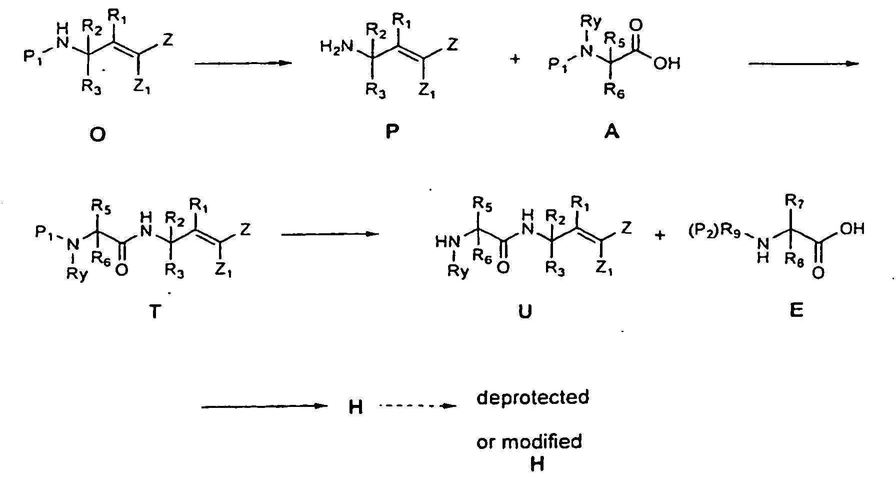

In General Method V, useful for synthesis of ketomethylene compounds of formula I-B, optically active lactone **AA**, where P₄ is an appropriate protecting group for nitrogen, and R₅ and R₈ are H (which may be prepared by the method described below and by various literature methods, including: (a) Herold et al., *J Org. Chem.* **1989**, *54*, 1178; (b) Bradbury et al., *Tetrahedron Lett*. **1989,** *30,* 3845; (c) Bradbury et al., *J. Med Chem.* **1990,** *33,* 2335; (d) Wuts et al., *J. Org. Chem.* **1992**, *57*, 6696; (e) Jones et al., *J. Org. Chem.* **1993**, *58*, 2286; (f) Pégorier et al., *Tetrahedron Lett*. **1995**, *36*, 2753; and (g) Dondoni et al., *J. Org. Chem.* **1995,** *60*, 7927) is transformed by a two-step procedure (basic hydrolysis and subsequent oxidation) into carboxylic acid **BB**. This material is not isolated, but is subjected to an amide-forming reaction with amine (or salt thereof) **P** to provide final product **CC**. The P₄ protecting group, along with any additional protecting groups that have been used on any R groups (R₁, R₂, R₃, R₆, and/or R₇) and/or on Z and/or on Z₁, is subsequently deprotected and/or further modified to yield deprotected or modified **CC**.

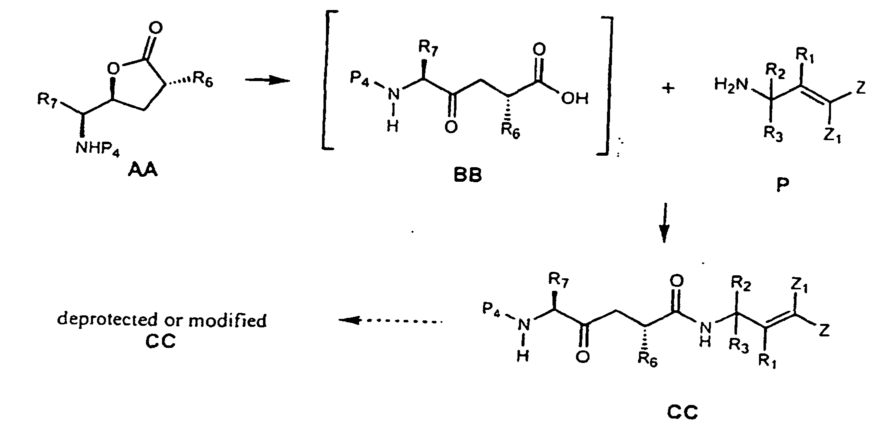

Lactone **AA** may be prepared in optically active form by the following General Method VI (see: Herold et al., *J. Org. Chem*. **1989**, *54,* 1178; Bradbury et al., *Tetrahedron Lett*. **1989,** *30*, 3845; and Bradbury et al., *J. Med. Chem*. **1990,** *33,* 2335). A γ,δ-unsaturated carboxylic acid **DD,** which incorporates R₇, is transformed into the corresponding acid chloride (not shown). This acid chloride is subjected to an amide-forming reaction with a chiral amine or a chiral oxazolidone to provide derivative **EE** (in which X₁ is a chiral amine or a chiral oxazolidone). Compound **EE** is subsequently deprotonated, and the resulting enolate is diastereoselectively alkylated with an electrophile corresponding to R₆ to provide compound **FF,** where R₅ is **H**. This material is then subjected to a halolactonization reaction to provide halo-lactone **GG**, in which R₅ and R₈ are H and "hal" is Br or I. Halo-lactone **GG** is subsequently transformed into azide **HH,** and this material is then converted into lactone **AA,** where P₄ is an appropriate protecting group for nitrogen.

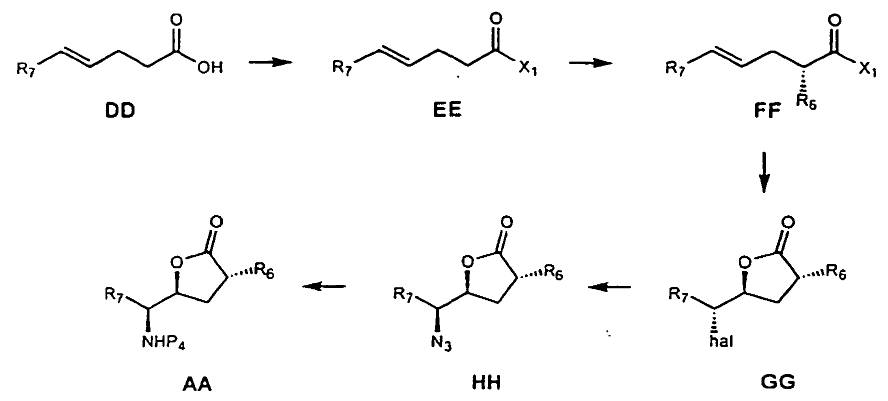

γ,δ-Unsaturated carboxylic acid **DD** may be prepared by the following General Method VII (see: Herold et al., *J. Org. Chem*. **1989**, *54*, 1178). An aldehyde **II**, which incorporates R₇, is coupled with vinylmagnesium bromide to give alcohol **JJ.** Alcohol **JJ** is then transformed into γ,δ-unsaturated carboxylic acid **DD** by a three-step procedure as follows: (i) treatment with diethyl malonate and catalytic Ti(OEt)₄ at 160°C for 1 hour, (ii) heating at 190°C for 4 hours, and (iii) hydrolysis with ethanolic KOH at reflux.

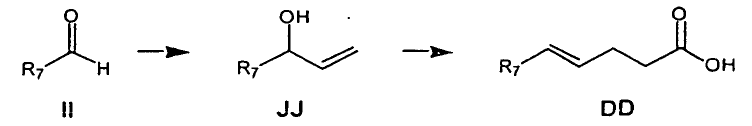

Carboxylic acid **BB** may also be prepared by General Method VIII (see Hoffman et al., *Tetrahedron*, **1997**, *53,* 7119). An amino acid **KK,** which incorporates R₇ and where P₄ is an appropriate protecting group for nitrogen, is transformed into β-ketoester **LL.** Compound **LL** is deprotonated and the resulting anion is condensed with triflate **MM,** which incorporates R₆. The coupling product thus obtained is treated with trifluoroacetic acid to provide ketoester **NN,** and this material is subsequently hydrolyzed to afford carboxylic acid **BB**. If basic hydrolysis results in epimerization, ketoester **NN** can be transesterified (allyl alcohol, Ti(O*i*-Pr)₄) and subsequently deprotected under neutral conditions (Pd(PPh₃)₄, morpholine) to give carboxylic acid **BB.** Triflate **MM,** in turn, may be prepared from the corresponding alcohol by treatment with trifluoromethanesulfonic anhydride and 2,6-lutidine.

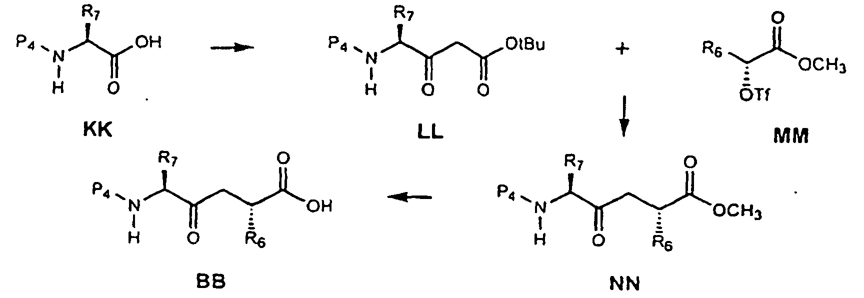

Lactone **AA** may also be prepared by General Method IX (see: Askin et al., *J. Org. Chem.* **1992,** *57*, 2771; and McWilliams et al., *J. Am. Chem. Soc.* **1996,** *118*, 11970). An amino acid KK, which incorporates R₇ and where P₄ is an appropriate protecting group for nitrogen, is transformed into epoxide **OO** (single diastereomer) by the method described in Luly et al., J *Org. Chem.* **1987,** *52*, 1487. Epoxide **OO** is condensed with the anion derived from compound **PP,** which incorporates R₆ and in which X₂ is a chiral auxiliary (including (1*S*,2*R*)-1-aminoindan-2-ol acetonide) to afford coupling product **QQ.** This material is subsequently cyclized under acidic conditions to provide lactone **AA.** Compound **PP** may be prepared from the corresponding carboxylic acid (not shown) by the method outlined in Askin et al., *J. Org. Chem.* **1992,** *57*, 2771.

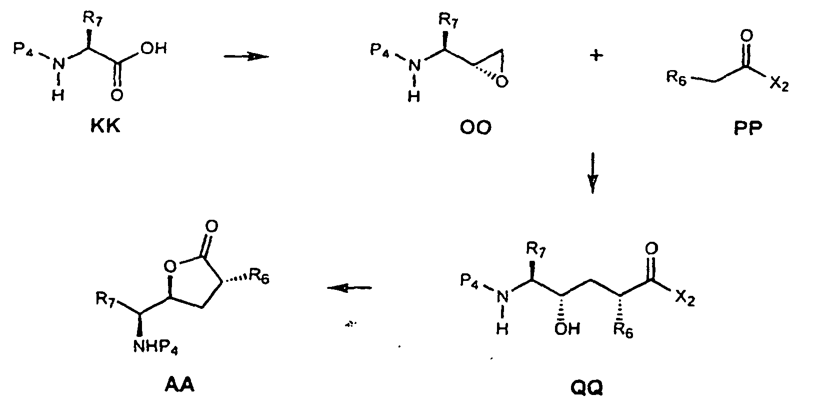

General Method X, useful in preparation of depsipeptide compounds of the formula I-C, illustrates a method to prepare intermediate **TT.** Amino acid **E** and alcohol **RR,** where P₅ is an appropriate protecting group for oxygen, are subjected to an ester bond-forming reaction to produce intermediate **SS**. Molecule **SS** is deprotected to yield free carboxylic acid **TT,** which may be utilized in lieu of carboxylic acid **K** in any of the general methods described above.

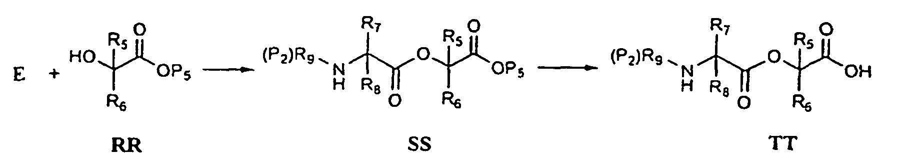

### SPECIFIC SYNTHESES

The following specific methods may also be used to prepare various compounds according to the invention.

Specific Method I describes the preparation of specific intermediate **O1**, which may be utilized as intermediate **O** in the general methods described above. Thus, ester **A1** (prepared as described in Chida et al., *J. Chem. Soc., Chem. Commun*. **1992,** 1064) is hydrolyzed to give acid **B1**, which, in tum, is transformed into oxazolidinone **C1**. Compound **C1** is subsequently deprotonated, and the resulting enolate is diastereoselectively alkylated to give allyl intermediate **D1**. This entity is oxidized via ozonolysis, and the resulting aldehyde (not shown) is subjected to a reductive amination reaction producing lactam **E1**. Acid-catalyzed methanolysis of **E1** then affords alcohol **F1**. This intermediate is oxidized via the method of Swern (or other suitable oxidation conditions) to the resulting aldehyde (not shown), which is subsequently subjected to an olefin-forming reaction to provide specific intermediate **O1**.

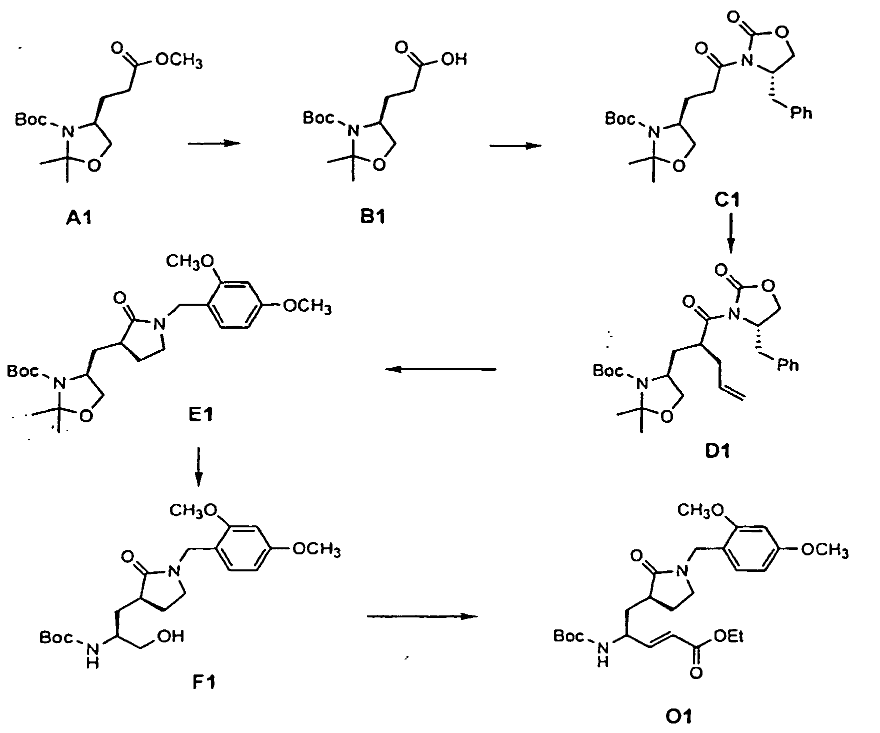

Specific Method II describes the preparation of specific intermediate **O2**, which may be utilized as intermediate **O** in the general methods described above. Allyl intermediate **D1** is subjected to a hydroboration/oxidation sequence to afford a primary alcohol (not shown). This entity is oxidized via the method of Swern (or other suitable oxidation conditions), and the resulting aldehyde (not shown) is subjected to a reductive-amination reaction, producing lactam **G1**. Acid-catalyzed methanolysis of **G1** then affords alcohol **H1**. This intermediate is oxidized via the method of Swern (or other suitable oxidation conditions) to the resulting aldehyde (not shown), which is subsequently subjected to an olefin-forming reaction to provide specific intermediate **O2.**

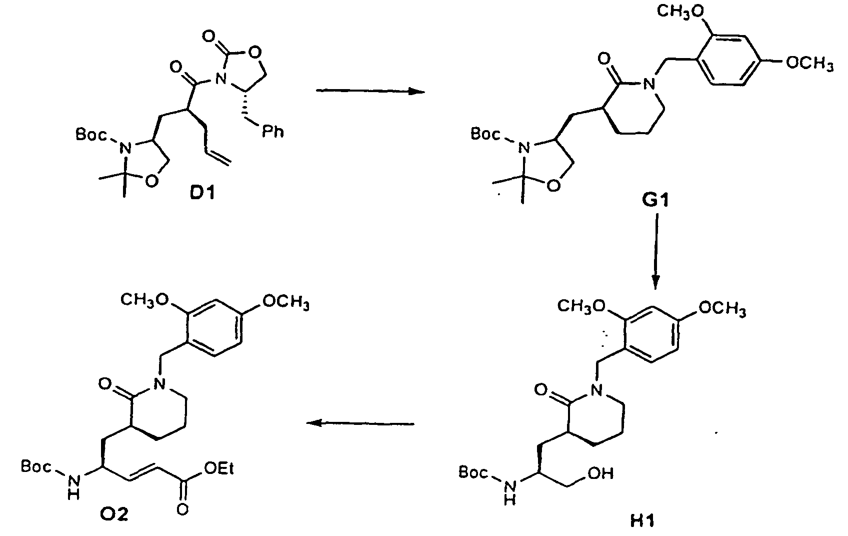

The following intermediates **P1, P2,** and **P3** may be used in the above general methods in place of intermediate **O,** to vary the substituent group in the R₂ position.

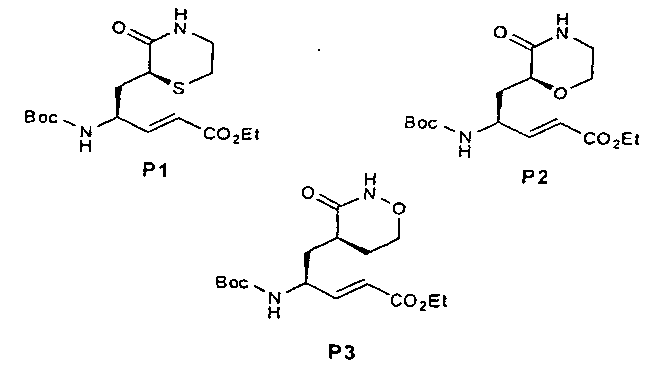

A synthesis of intermediate **P1** is described below. Intermediate **C1** (described above) is deprotonated, and the resulting enolate is trapped with an appropriate disulfide (symmetrical or mixed) to give sulfide **p1** (P is a suitable protecting group for oxygen). The oxygen-protecting group is then removed to give alcohol **p2**. This intermediate is oxidized via the method of Swern (or using other suitable oxidation.conditions), and the resulting aldehyde (not shown) is subjected to a reductive amination reaction to give lactam **p3**. Acid-catalyzed methanolysis of **p3** then affords alcohol **p4**. This intermediate is oxidized via the method of Swern (or using other suitable oxidation conditions) to the resulting aldehyde (also not shown), which is subsequently subjected to an olefin-forming reaction to provide intermediate **p5.** This compound may be utilized in place of intermediate **O** in the above general synthetic methods; alternatively, the lactam-protecting group may be removed to give intermediate **P1**.

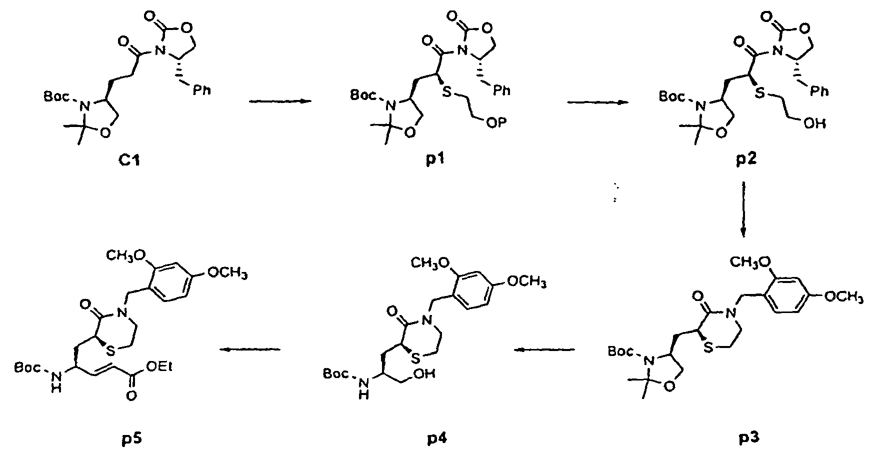

To synthesize intermediate **P2,** intermediate **C1** is deprotonated, and the resulting enolate is trapped with an appropriate source of electrophilic oxygen (e.g., an oxaziridine) to give alcohol **p6.** This intermediate is alkylated with a suitably functionalized alkyl halide or triflate to give ether **p7** (P is an appropriate protecting group for nitrogen). The nitrogen-protecting group is then removed, and the resulting amine (not shown) is subjected to cyclization conditions to give lactam **p8**. Acid-catalyzed methanolysis of **p8** then affords alcohol **p9**. This intermediate is oxidized via the method of Swern (or using other suitable oxidation conditions) to the resulting aldehyde (also not shown), which is subsequently subjected to an olefin-forming reaction to provide intermediate **P2**.

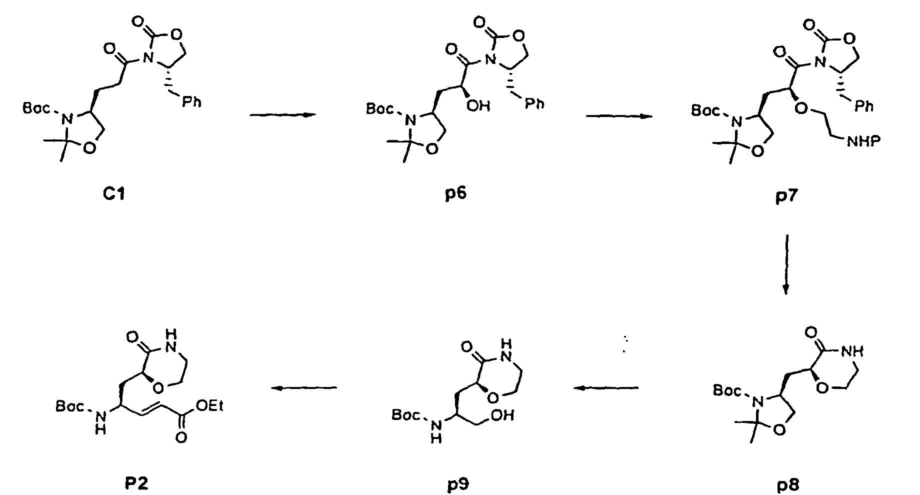

A synthesis of specific intermediate **P3** is now described. Intermediate **D1** (described above) is ozonized, and the resulting aldehyde (not shown) is reduced to the corresponding alcohol (also not shown). This intermediate is then protected to afford compound **p10** (P₁ is an appropriate protecting group for oxygen). The imide functionality present on **p10** is hydrolyzed to carboxylic acid **p11**, and this intermediate is coupled with a suitably protected hydroxylamine derivative to give amide **p12** (P₂ is an appropriate protecting group for oxygen that is stable to conditions which will remove P₁). The P₁ protecting group is then removed, and the resulting alcohol **(p13)** is transformed into an appropriate leaving group (halide or triflate, not shown). The P₂ protecting group is then removed, and the resulting hydroxamic acid is cyclized to give intermediate **p14.** Acid-catalyzed methanolysis of **p14** then affords alcohol **p15**. This intermediate is oxidized via the method of Swern (or using other suitable oxidation conditions) to the resulting aldehyde (not shown), which is subsequently subjected to an olefin-forming reaction to provide intermediate **P3.**

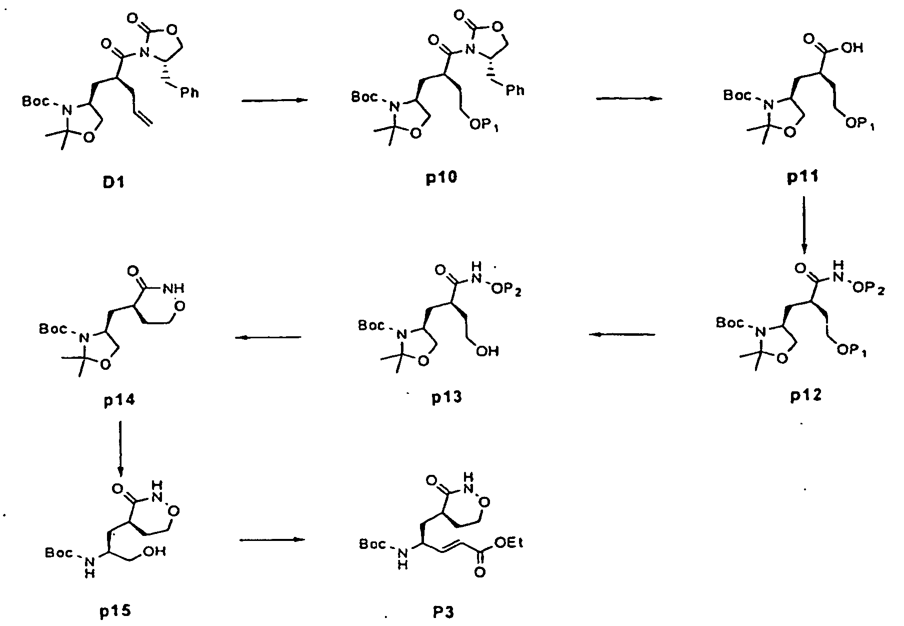

Specific Method III describes the preparation of intermediates **Q1, Q2,** and **Q3,** which may be utilized in the general methods described above. The known compound **I1** is transformed into the literature molecule **J1** by a modification of a published procedure (Ikuta et al., *J. Med. Chem.* **1987,** vol. 30, p. 1995). Independently, the amino acid ester **K1** is protected to afford silyl ether **L1**. The ether is reduced with DIBAL (or using other suitable reduction conditions), and the resulting aldehyde (not shown) is subjected to an olefin-forming reaction with intermediate **J1**, producing **M1**. Silyl deprotection of **M1** then affords alcohol **N1**. This intermediate is subjected to a variety of hydrogenation conditions to provide intermediates **Q1, Q2,** and **Q3.** These intermediates may be transformed into intermediates analogous to intermediate **O1** (see Specific Method I above) by oxidation and subsequent olefination.

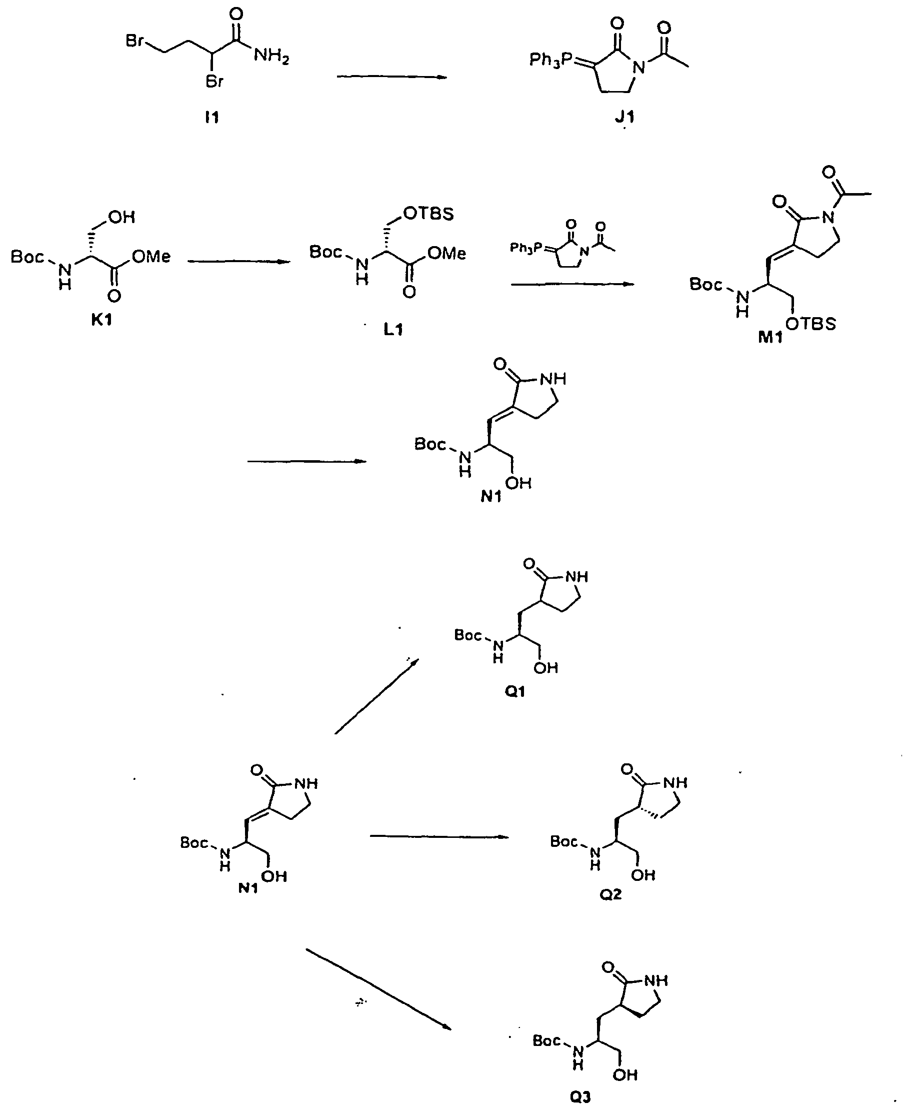

The artisan will recognize that various compounds of the invention may be made by following the above-described general and specific methods as well as teachings in the art, including the references cited herein, the disclosures of which are hereby incorporated by reference. Additionally, the artisan may prepare various compounds of the invention according to the example described below or through routine modifications to the syntheses described herein.

### EXAMPLES

Examples of various preferred compounds of formula I are set forth below. The structures of the compounds of the following examples were confirmed by one or more of the following: proton magnetic resonance spectroscopy, infrared spectroscopy, elemental microanalysis, mass spectrometry, thin layer chromatography, melting-point determination, and boiling-point determination. Where there is any discrepancy between the given structural formula shown for a compound and its chemical name provided, the structural formula applies.

Proton magnetic resonance (¹H NMR) spectra were determined using a Varian UNITY*plus* 300 spectrometer operating at a field strength of 300 megahertz (MHz). Chemical shifts are reported in parts per million (ppm, δ) downfield from an internal tetramethylsilane standard. Alternatively, ¹H NMR spectra were referenced to residual protic solvent signals as follows: CHCl₃ = 7.26 ppm; DMSO = 2.49 ppm; C₆HD₅ = 7.15 ppm. Peak multiplicities are designated as follows: s = singlet; d = doublet; dd = doublet of doublets; t = triplet; q = quartet; br = broad resonance; and m = multiplet. Coupling constants are given in Hertz. Infrared absorption (IR) spectra were obtained using a Perkin-Elmer 1600 series FTIR spectrometer. Elemental microanalyses were performed by Atlantic Microlab Inc. (Norcross, GA) and gave results for the elements stated within ±0.4% of the theoretical values. Flash column chromatography was performed using Silica gel 60 (Merck Art 9385). Analytical thin layer chromatography (TLC) was performed using precoated sheets of Silica 60 F₂₅₄ (Merck Art 5719). Melting points (abbreviated as mp) were determined on a Mel-Temp apparatus and are uncorrected. All reactions were performed in septum-sealed flasks under a slight positive pressure of argon, unless otherwise noted. All commercial reagents were used as received from their respective suppliers with the following exceptions: tetrahydrofuran (THF) was distilled from sodium-benzophenone ketyl prior to use; dichloromethane (CH₂Cl₂) was distilled from calcium hydride prior to use; anhydrous lithium chloride was prepared by heating at 110°C under vacuum overnight.

The following abbreviations are used herein: Et₂O refers to diethyl ether; DMF refers to *N,N*-dimethylformamide; DMSO refers to dimethylsulfoxide; and MTBE refers to *tert*-butyl methyl ether. Other abbreviations include: CH₃OH (methanol), EtOH (ethanol), EtOAc (ethyl acetate), DME (ethylene glycol dimethyl ether), Ac (acetyl), Me (methyl), Ph (phenyl), Tr (triphenylmethyl), Cbz (benzyloxycarbonyl), Boc (*tert*-butoxycarbonyl), TFA (trifluoroacetic acid), DIEA (*N*,*N*-diisopropylethylamine), DBU (1,8-diazabicyclo[5.4.0]undec-7-ene), HOBt (1-hydroxybenzotriazole hydrate), PyBOP (benzotriazolel-yl-oxy-tris-pyrrolidino-phosphonium hexafluorophosphate), HATU (*O*-(7-azabenzotriazol-1-yl)-*N*,*N*,*N'*,*N'*-tetramethyluronium hexafluorophosphate), EDC (1-(3-dimethylaminopropyl)-3-ethylcarbarbodiimide hydrochloride), DCC (dicyclohexylcarbodiimide), DDQ (2,3-dichloro-5,6-dicyano-1,4-benzoquinone), DMAP (4-dimethylaminopyridine), Gln (glutamine), Leu (leucine), Phe (phenylalanine), Val (valine), His (histidine), 1-Naphth (1-naphthlyalanine), 2-Naphth (2-naphthylalanine), α-t-Butyl-Gly (tert-butyl glycine), (*S*)-Pyrrol-Ala ((2*S*,3'*S*)-2-amino-3-(2'-oxopyrrolidin-3'-yl)-propionic acid), and (*S*)-Piper-Ala ((2*S*,3'*S*)-2-amino-3-(2'-oxo-piperidin-3'-yl)-propionic acid). Additionally, "L" represents naturally occurring amino acids.

A simplified naming system employing amino acid abbreviations is used to identify some intermediates and final products. When naming compounds, italicized amino acid abbreviations represent modifications at the C-terminus of that residue where the following apply: (1) acrylic acid esters are reported as "E" (trans) propenoates; (2) substituted 3-methylene-dihydrofuran-2-ones are reported as "E" (trans) 2-(α-vinyl-γ-butyrolactones); and (3) 5-vinylisoxazoles are reported as "E" (trans) propenisoxazoles. In addition, the terminology "AA₁Ψ[COCH₂]-AA₂" indicates that, for any peptide sequence, two amino acids (AA₁ and AA₂) usually linked by an amide bond are replaced by a ketomethlyene dipeptide isostere moiety. The terminology "AA₁-NCH₃-AA₂" indicates that, for any peptide sequence, the amide bond that usually connects the two amino acids (AA₁ and AA₂) is replaced by an *N*-methyl amide linkage. The terminology "AA₁-O-AA₂" indicates that, for any peptide sequence, the amide bond that usually connects the two amino acids (AA₁ and AA₂) is replaced by an ester linkage.

Examples of embodiments in accordance with the invention are described below.

### Example 1 - Preparation of Comparison Compound #1: 5-(3'-(Cbz-L-Leu-L-Phe-L-Gln)-E-Propene)-isoxazole

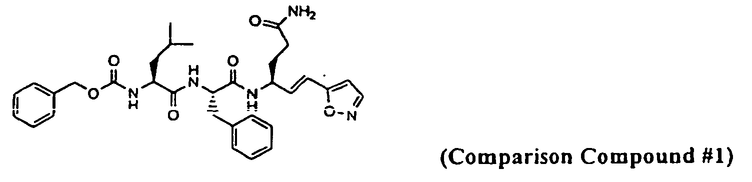

### Preparation of Intermediate Cbz-L-(Tr-Gln)-OMe

Cbz-L-(Tr-Gln) (0.26 g, 0.50 mmol, 1 equiv.) was added to a solution of acetyl chloride (0.25 mL, 3.52 mmol, 7.0 equiv.) in CH₃OH (5 mL), and stirring was continued at 23°C for 1 h (hour). The solvent was removed under reduced pressure, and the residue was dissolved in CH₂Cl₂ (100 mL) and washed with water (100 mL), saturated NaHCO₃ (100 mL), and brine (100 mL). The organic layer was dried over Na₂SO₄ and was concentrated. The residue was purified by flash column chromatography (20% EtOAc in hexanes) to afford Cbz-L-(Tr-Gln)-OMe (0.23 g, 84% yield) as a white solid: mp = 139-140°C; IR (cm⁻¹) 1742, 1207; ¹H NMR (DMSO-*d*₆) δ 1.16 (t, 1H, *J* = 7.0), 1.77 (m, 1H), 1.97 (m, 1H), 3.61 (s, 3H), 4.99 (m, 1H), 5.03 (s, 2H), 7.02-7.55 (m, 20H), 7.69 (d, 1H, *J* = 7.7), 8.59 (s, 1H); Anal. (C₃₃H₃₂N₂O₅) C, H, N.

### Preparation of Intermediate Cbz-L-(Tr-Glutaminol)

Lithium chloride (0.24 g, 5.66 mmol, 2.0 equiv.) was added to a solution of Cbz-L-(Tr-Gln)-OMe (1.50 g, 2.79 mmol, 1 equiv.) in 2:1 THF:EtOH (30 mL), and the mixture was stirred at 23°C until all solids had dissolved (10 minutes). Sodium borohydride (0.21 g, 5.55 mmol, 2.0 equiv.) was added, and the reaction mixture was stirred overnight at 23°C. The solvents were removed under reduced pressure, the residue was taken up in water (50 mL), and the pH was adjusted to 2-3 with 10% HCl. The product was extracted with EtOAc (50 mL), and the organic layer was washed with water (50 mL) and brine (50 mL) before drying over MgSO₄. The organic layer was concentrated, and the residue was purified by flash column chromatography (gradient elution, 20→50% EtOAc in benzene) to give Cbz-L-(Tr-glutaminol) (1.02 g, 72% yield) as a white glassy solid: mp = 66-70°C; IR (cm⁻¹) 3318, 1699, 1510, 1240; ¹H NMR (DMSO-*d*₆) δ 1.40 (m, 1H), 1.72 (m, 1H), 2.26 (m, 2H), 3.17-3.50 (m, 3H), 4.64 (t, 1H, *J* = 5.0), 5.00 (s, 2H), 7.00-7.40 (m, 20H), 6.96 (d, 1H, *J* = 8.5), 8.54 (s, 1H); Anal. (C₃₂H₃₂N₂O₄) C, H, N.

### Preparation of Intermediate L-(Tr-Glutaminol)

A suspension of Cbz-L-(Tr-glutaminol) (1.93 g, 3.79 mmol) in CH₃OH (25 mL) and Pd/C (10%, 0.19 g) was stirred under a hydrogen atmosphere (balloon) for 4 hours, then was filtered through a layer of Celite. The filtrate was concentrated under reduced pressure to give L-(Tr-glutaminol) as a white amorphous solid (1.38 g, 98% yield): mp = 191-193°C; IR (cm⁻¹) 3255 (br), 1642, 1527; ¹H NMR (DMSO-*d*₆) δ 1.29 (m, 1H), 1.53 (m, 1H), 2.29 (m, 2H), 3.08 (m, 1H), 3.18 (m, 2H), 3.38 (s, br, 2H), 4.43 (s, br, 1H), 7.14-7.28 (m, 15H), 8.62 (s, 1H).

### Preparation of Intermediate Cbz-L-Leu-L-Phe-L-(Tr-Glutaminol)

Carbonyldiimidazole (0.17 g, 1.05 mmol, 1.0 equiv.) was added to a solution of Cbz-L-Leu-L-Phe-OH (0.41 g, 1.0 mmol, 0.95 equiv.) in THF (10 mL), and the reaction mixture was stirred at 23°C for 1 hour. L-(Tr-Glutaminol) (0.39 g, 1.05 mmol, 1 equiv.) was then added, and the resulting solution was stirred overnight. The volatiles were removed under reduced pressure, and the residue was purified by flash column chromatography (gradient elution, 2→4% CH₃OH in CHCl₃) to give Cbz-L-Leu-L-Phe-L-(Tr-glutaminol) (0.47 g, 62% yield) as a white amorphous solid: mp = 92-95°C; IR (cm⁻¹) 3302, 1657, 1520, 1238; ¹H NMR (DMSO-*d*₆) δ 0.79 (t, 6H, *J* = 7.0), 1.30 (m, 2H), 1.44 (m, 2H), 1.75 (m, 1H), 2.22 (m, 2H), 2.82 (m, 1H), 2.97 (m, 1H), 3.14 (m, 1H), 3.25 (m, 1H), 3.63 (m, 1H), 3.95 (m, 1H), 4.48 (m, 1H), 4.65 (t, 1H, *J* = 5.0), 4.96 (d, 1H, *J* = 13.0), 5.02 (d, 1H, *J* = 13.0), 7.07-7.33 (m, 25H), 7.42 (d, 1H, *J* = 8.0), 7.66 (d, 1H, *J* = 8.5), 7.86 (d, 1H, *J* = 8.0), 8.52 (s, 1H); Anal. (C₄₇H₅₂N₄O₆•0.5H₂O) C, H, N.

### Preparation of Intermediate Cbz-L-Leu-L-Phe-L-(Tr-Gln)-H

*o*-Iodoxybenzoic acid (0.63 g, 2.25 mmol, 3.0 equiv.) was added to a solution of Cbz-L-Leu-L-Phe-L-(Tr-glutaminol) (0.58 g, 0.75 mmol, 1 equiv.) in DMSO (7.5 mL) at 23°C. After stirring for 2 hours, the DMSO was removed under reduced pressure. The residue was twice diluted with CH₂Cl₂, and the solvent evaporated to remove any remaining DMSO. The residue was diluted with EtOAc (30 mL) and filtered, and the filtrate was washed with 5% Na₂S₂O₃/5% NaHCO₃ solution (30 mL), water (30 mL), and brine (30 mL), and then dried over Na₂SO₄. The solvent was removed under reduced pressure to give Cbz-L-Leu-L-Phe-L-(Tr-Gln)-H (0.53 g, 92% yield) as a white glassy solid, which was used immediately without further purification: ¹H NMR (DMSO-*d*₆) δ 0.79 (m, 6H), 1.00-1.98 (m, 5H), 2.27 (m, 2H), 2.84 (m, 1H), 3.02 (m, 1H), 3.98 (m, 2H), 4.58 (m, 1H), 4.99 (s, 2H), 7.14-7.32 (m, 25H), 7.39 (d, 1H, *J* = 8.1), 7.97 (d, 1H, *J* = 8.5), 8.38 (d, 1H, *J* = 8.0), 8.60 (s, 1H), 9.20 (s, 1H).

### Preparation of Intermediate 5-{3'-(Cbz-L-Leu-L-Phe-L-(Tr-Gln))-E-Propene}-Isoxazole

A solution of KN((CH₃)₃Si)₂ (0.95 mL of a 0.5 M solution in THF, 0.477 mmol, 1.0 equiv.) was added to a suspension of isoxazol-5-ylmethyl-triphenylphosphonium bromide (0.222 g, 0.525 mmol, 1.1 equiv) in THF (20 mL) at 0°C, and the reaction was stirred at 0°C for 30 minutes. A solution of Cbz-L-Leu-L-Phe-L-(Tr-Gln)-H (0.366 g, 0.477 mmol, 1 equiv.) in THF (10 mL) was added, and the reaction mixture was then stirred overnight at 23°C. The solvent was removed *in vacuo*, and the residue was diluted with EtOAc (30 mL), washed with water (30 mL), and then dried over MgSO₄. The solvent was removed under reduced pressure and the residue purified by flash silica gel chromatography (gradient elution, 0→1% CH₃OH in CHCl₃) to give 5-{3'-(Cbz-L-Leu-L-Phe-L-(Tr-*Gln*))-E-propene}-isoxazole (0.307 g, 70% yield) as an amorphous solid: IR (cm⁻¹) 3423, 1678, 1568, 1265, 1043, 711; ¹H NMR (DMSO-*d*₆) δ 0.77-0.81 (m, 6H), 1.21-1.36 (m, 2H), 1.40-1.55 (m, 1H), 1.60-1.80 (m, 2H), 2.34-2.45 (m, 2H), 2.82-2.87 (m, 1H), 2.91-3.04 (m, 1H), 3.95-4.00 (m, 1H), 4.41-4.50 (m, 1H), 4.53-4.60 (m, 1H), 4.99 (q, 2H, *J* = 6.0), 6.19 (d, 1H, *J* = 15.0), 6.36 (dd, 1H, *J* = 15.0, 6.0), 6.46 (s, 1H), 7.15-7.33 (m, 20H), 7.42 (d, 1H, *J* = 9.0), 7.56-7.63 (m, 5H), 7.96 (d, 1H, *J* = 9.0), 8.08 (d, 1H, *J* = 9.0), 8.51 (s, 1H), 8.58 (s, 1H); HRMS calcd. for C₅₁H₅₃N₅O₆+Cs 964.3050 (M+Cs), found 964.3018.

### Preparation of Product: 5-(3'-(Cbz-L-Leu-L-Phe-L-Gln)-E-Propene)-Isoxazole

Trifluoroacetic acid (1 mL) was added to a solution of 5-{3'-(Cbz-L-Leu-L-Phe-L-(Tr-*Gln*))-E-propene}-isoxazole (0.214 g, 0.257 mmol) in CH₂Cl₂ (10 mL), and the reaction mixture was stirred at 23°C overnight. The solvent was removed *in vacuo* and the residue purified by flash silica gel chromatography (gradient elution, 0→1% CH₃OH in CHCl₃) to give 5-(3'-(Cbz-L-Leu-L-Phe-L-*Gln*)-E-propene)-isoxazole as a white solid (0.054 g, 36% yield): ¹H NMR (DMSO-*d*₆) δ 0.77-0.83 (m, 6H), 1.26-1.46 (m, 2H), 1.47-1.62 (m, 1H), 1.69-1.79 (m, 2H), 2.04-2.29 (m, 2H), 2.83-2.88 (m, 1H), 2.97-3.10 (m, 1H), 3.99-4.12 (m, 1H), 4.37-4.43 (m, 1H), 4.48-4.57 (m, 1H), 5.01 (q, 2H, *J* = 6.0), 6.20 (d, 1H, *J* = 15.0), 6.36 (dd, 1H, *J* = 15.0, 6.0), 6.45 (d, 1H, *J* = 3.0), 6.75 (s, 1H), 7.14-7.29 (m, 6H), 7.31-7.40 (m, 5 H), 7.45 (d, 1H, *J* = 9.0), 8.04 (d, 1H, *J* = 9.0), 8.07 (d, 1H, *J* = 9.0), 8.51 (d, 1H, *J* = 3.0); Anal. (C₃₂H₃₉N₅O₆) C, H, N.

### Example 2 - Preparation of Compound A-1: Ethyl-3-((5'-Methylisoxazole-3'-carbonyl)-L-Leu-L-Phe-L-Gln)-E-Propenoate

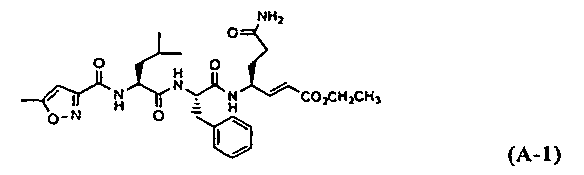

### Preparation of Intermediate Boc-L-(Tr-Gln)-N(OMe)Me

Isobutyl chloroformate (4.77 mL, 36.8 mmol, 1.0 equiv.) was added to a solution of Boc-L-(Tr-Gln)-OH (18.7 g, 36.7 mmol, 1 equiv.) and 4-methylmorpholine (8.08 mL, 73.5 mmol, 2.0 equiv.) in CH₂Cl₂ (250 mL) at 0°C. The reaction mixture was stirred at 0°C for 20 min. (minutes), then *N,O*-dimethylhydroxylamine hydrochloride (3.60 g, 36.7 mmol, 1.0 equiv.) was added. The resulting solution was stirred at 0°C for 20 min. and at 23°C for 2 hours (h), and then was partitioned between water (150 mL) and CH₂Cl₂ (2 x 150 mL). The combined organic layers were dried over Na₂SO₄, and were concentrated. Purification of the residue by flash column chromatography (gradient elution, 40→20% hexanes in EtOAc) provided Boc-L-(Tr-Gln)-N(OMe)Me (16.1 g, 82% yield) as a white foam: IR (cm⁻¹) 3411, 3329, 3062, 1701, 1659; ¹H NMR (CDCl₃) δ 1.42 (s, 9H), 1.63-1.77 (m, 1H), 2.06-2.17 (m, 1H), 2.29-2.43 (m, 2H), 3.17 (s, 3H), 3.64 (s, 3H), 4.73 (s, br, 1H), 5.38-5.4 (m, 1H), 7.20-7.31 (m, 15H); Anal. (C₃₁H₃₇N₃O₅) C, H, N.

### Preparation of Intermediate Boc-L-(Tr-Gln)-H

Diisobutylaluminum hydride (50.5 mL of a 1.5 M solution in toluene, 75.8 mmol, 2.5 equiv.) was added to a solution of Boc-L-(Tr-Gln)-N(OMe)Me (16.1 g, 30.3 mmol, 1 equiv.) in THF at -78°C, and the reaction mixture was stirred at -78°C for 4 hours. Methanol (4 mL) and 1.0 M HCl (10 mL) were added sequentially, and the mixture was warmed to 23°C. The resulting suspension was diluted with Et₂O (150 mL), and was washed with 1.0 M HCl (3 x 100 mL), half-saturated NaHCO₃ (100 mL), and water (100 mL). The organic layer was dried over MgSO₄, filtered, and concentrated to give crude Boc-L-(Tr-Gln)-H (13.8 g, 97% yield) as a white solid: mp = 114-116°C; IR (cm⁻¹) 3313, 1697, 1494; ¹H NMR (CDCl₃) δ 1.44 (s, 9H), 1.65-1.75 (m, 1H), 2.17-2.23 (m, 1H), 2.31-2.54 (m, 2H), 4.11 (s, br, 1H), 5.38-5.40 (m, 1H), 7.11 (s, 1H), 7.16-7.36 (m, 15H), 9.45 (s, 1H).

### Preparation of Intermediate Ethyl-3-(Boc-L-(Tr-Gln))-E-Propenoate

Sodium bis(trimethylsilyl)amide (22.9 mL of a 1.0 M solution in THF, 22.9 mmol, 1.0 equiv.) was added to a solution of triethyl phosphonoacetate (5.59 g, 22.9 mmol, 1.0 equiv.) in THF (200 mL) at -78°C, and the resulting solution was stirred for 20 minutes at that temperature. Crude Boc-L-(Tr-Gln)-H (10.8 g, 22.9 mmol, 1 equiv.) in THF (50 mL) was added via cannula, and the reaction mixture was stirred for 2 hours at -78°C, warmed to 0°C for 10 minutes, and then partitioned between 0.5 M HCl (150 mL) and a 1:1 mixture of EtuAc and hexanes (2 x 150 mL). The combined organic layers were dried over Na₂SO₄ and were concentrated. Purification of the residue by flash column chromatography (40% EtOAc in hexanes) provided ethyl-3-[Boc-L-(Tr-*Gln*)]-E-propenoate (10.9 g, 88% yield) as a white foam: IR (cm⁻¹) 3321, 1710; ¹H NMR (CDCl₃) δ 1.27 (t, 3H, *J* = 7.2), 1.42 (s, 9H), 1.70-1.78 (m, 1H), 1.80-1.96 (m, 1H), 2.35 (t, 2H, *J* = 7.0), 4.18 (q, 2H, *J* = 7.2), 4.29 (s, br, 1 H), 4.82-4.84 (m, 1H), 5.88 (dd, 1H, *J* = 15.7, 1.6), 6.79 (dd, 1H, *J* = 15.7, 5.3), 6.92 (s, 1H), 7.19-7.34 (m, 15H); Anal. (C₃₃H₃₈N₂O₅) C, H, N.

### Preparation of Intermediate Ethyl-3-(Boc-L-Phe-L-(Tr-Gln))-E-Propenoate

A solution of HCl in 1,4-dioxane (4.0 M, 15 mL), was added to a solution of ethyl-3-(Boc-L-(Tr-*Gln*))-E-propenoate (3.26 g, 6.01 mmol, 1 equiv.) in the same solvent (15 mL) at 23°C. After 2 h, the volatiles were removed under reduced pressure to afford ethyl-3-(H₂N-L-(Tr-*Gln*))-E-propenoate•HCl. This material was dissolved in CH₂Cl₂ (60 mL) and Boc-L-Phe-OH (1.59 g, 6.01 mmol, 1.0 equiv.), HOBt (1.22 g, 9.02 mmol, 1.5 equiv.), 4-methylmorpholine (1.98 mL, 18.03 mmol, 3 equiv.), and EDC (1.73 g, 9.02 mmol, 1.5 equiv.) were added sequentially. The reaction mixture was stirred at 23°C overnight, and then was partitioned between water (100 mL) and CH₂Cl₂ (2 x 100 mL). The combined organic layers were dried over Na₂SO₄, concentrated, and the residue was purified by flash column chromatography (40% EtOAc in hexane) to afford ethyl-3-(Boc-L-Phe-L-(Tr-*Gln*))-E-propenoate (3.55 g, 85%) as white foam: IR (cm⁻¹) 3306, 1706, 1661; ¹H NMR (CDCl₃) δ 1.29 (t, 3H, *J* = 7.2), 1.38 (s, 9H), 1.65-1.76 (m, 1H), 1.87-1.99 (m, 1H), 2.25-2.27 (m, 2H), 2.94-3.01 (m, 2H), 4.14-4.26 (m, 3H), 4.48-4.53 (m, 1H), 4.95 (s, br, 1H), 5.64 (d, 1H, *J* = 15.8), 6.29 (d, 1H, *J* = 8.1), 6.64 (dd, 1H, *J* = 15.8, 5.4), 6.80 (s, br, 1H), 7.14-7.32 (m, 20H); Anal. (C₄₂H₄₇N₃O₆) C, H, N.

### Preparation of Intermediate Ethyl-3-(Boc-L-Leu-L-Phe-L-(Tr-Gln))-E-Propenoate

A solution of HCl in 1,4-dioxane (4.0 M, 15 mL) was added to a solution of ethyl-3-(Boc-L-Phe-L-(Tr-*Gln*))-E-propenoate (6.40 g, 9.28 mmol, 1 equiv.) in the same solvent (15 mL) at 23°C. After 2 hours, the volatiles were removed under reduced pressure. The residue was dissolved in CH₂Cl₂ (100 mL), and Boc-L-Leu-OH (2.58 g, 11.1 mmol, 1.2 equiv.), HOBt (1.88 g, 13.9 mmol, 1.5 equiv.), 4-methylmorpholine (3.06 mL, 27.8 mmol, 3 equiv.), and EDC (2.67 g, 13.92 mmol, 1.5 equiv.) were added sequentially. The reaction mixture was stirred at 23°C overnight, and then was partitioned between water (100 mL) and CH₂Cl₂ (2 x 100 mL). The combined organic layers were dried over Na₂SO₄ and concentrated, and the residue was purified by flash column chromatography (2% CH₃OH in CH₂Cl₂) to afford ethyl-3-(Boc-L-Leu-L-Phe-L-(Tr-*Gln*))-E-propenoate (6.46 g, 87% yield) as white foam: IR (cm⁻¹) 3284, 1651, 1515; ¹H NMR (CDCl₃) δ 0.86 (d, 3H, *J* = 6.0), 0.89 (d, 3H, *J* = 6.0), 1.29 (t, 3H, *J* = 7.2), 1.34 (s, 9H), 1.38-1.60 (m, 3H), 1.62-1.89 (m, 1H), 1.95-1.97 (m, 1H), 2.28-2.30 (m, 2H), 3.06-3.08 (m, 2H), 3.92-3.94 (m, 1H), 4.17 (q, 2H, *J* = 7.2), 4.48-4.51 (m, 2H), 4.67 (m, 1H), 5.66 (d, 1H, *J* = 15.9), 6.51-6.57 (m, 2H), 6.69 (dd, 1H, *J* = 15.6, 5.1), 7.10-7.33 (m, 21H); Anal. (C₄₈H₅₈N₄O_{7•}0.33H₂O) C, H, N.

### Preparation of Intermediate Ethyl-3-((5'-Methylisoxazole-3'-carbonyl)-L-Leu-L-Phe-L-(Tr-Gln))-E-Propenoate

A solution of HCl in 1,4-dioxane (4.0 M, 3 mL), was added to a solution of ethyl-3-(Boc-L-Leu-L-Phe-L-(Tr-*Gln*))-E-propenoate (0.216 g, 0.27 mmol, 1 equiv.) in the same solvent (3 mL) at 23°C. After 2 hours, the volatiles were removed under reduced pressure. The residue was dissolved in CH₂Cl₂ (15 mL), cooled to 0°C, and triethylamine (0.112 mL, 0.81 mmol, 3.0 equiv.) and 5-methylisoxazole-3-carbonyl chloride (0.058 g, 0.40 mmol, 1.5 equiv.) were added sequentially. The reaction mixture was stirred at 0°C for 30 minutes, and then was partitioned between water (50 mL) and CH₂Cl₂ (2 x 50 mL). The combined organic layers were dried over Na₂SO₄ and concentrated, and the residue was purified by flash column chromatography (2% CH₃OH in CH₂Cl₂) to afford ethyl-3-((5'-methylisoxazole-3'-carbonyl)-L-Leu-L-Phe-L-(Tr-*Gln*))-E-propenoate (0.199 g, 91% yield) as a white foam: IR (cm⁻¹) 3286, 1650, 1541; ¹H NMR (CDCl₃) δ 0.86 (d, 3H, *J* = 5.4), 0.89 (d, 3H, *J* = 5.7), 1.28 (t, 3H, *J* = 7.2), 1.43-1.59 (m, 2H), 1.67-1.75 (m, 1H), 1.95-1.99 (m, 2H), 2.28 (t, 2H, *J* = 7.2), 2.41 (s, 3H), 2.97-3.04 (m, 1H), 3.06-3.13 (m, 1H), 4.17 (q, 2H, *J* = 7.2), 4.31-4.33 (m, 1H), 4.48-4.52 (m, 2H), 5.72 (d, 1H, *J* = 15.9), 6.19 (s, 1H), 6.41 (d, 1H, *J* = 7.5), 6.59 (d, 1H, *J* = 8.1), 6.71 (dd, 1H, *J* = 15.3, 6.0), 6.95 (d, 1H, *J* = 6.6), 7.09-7.21 (m, 21 H); Anal. (C₄₈H₅₃N₅O_{7•}H₂O) C, H, N.

### Preparation of Product Ethyl-3-((5'-Methylisoxazole-3'-carbonyl)-L-Leu-L-Phe-L-Gln)-E-Propenoate

Triisopropylsilane (0.077 mL, 0.376 mmol, 1.8 equiv.) and trifluoroacetic acid (3 mL) were added sequentially to a solution of ethyl-3-((5'-methylisoxazole-3'-carbonyl)-L-Leu-L-Phe-L-(Tr-*Gln*))-E-propenoate (0.185 g, 0.21 mmol, 1 equiv.) in CH₂Cl₂ (3 mL) at 23°C, producing a bright yellow solution. The reaction mixture was stirred for 30 minutes at 23°C, during which time it became colorless. The volatiles were removed under reduced pressure, and the resulting white solid was triturated with Et₂O (10 mL), filtered, and air-dried to give ethyl-3-((5'-methylisoxazole-3'-carbonyl)-L-Leu-L-Phe-L-*Gln*)-E-propenoate (0.87 g, 81% yield) as white solid: mp = 223-225°C; IR (cm⁻¹) 3298, 1662, 1544, 1457, 1278; ¹H NMR (DMSO-*d*₆) δ 0.81 (d, 3H, *J* = 6.0 ), 0.85 (d, 3H, *J* = 6.3), 1.23 (t, 3H, *J* = 6.9), 1.38-1.42 (m, 1H), 1.48-1.77 (m, 4H), 2.04 (t, 2H, *J* = 7.2), 2.46 (s, 3H), 2.78-2.86 (m, 1H), 2.93-3.00 (m, 1H), 4.11 (q, 2H, *J* = 7.2), 4.36-4.54 (m, 3H), 5.63 (d, 1 H, *J* = 15.6), 6.56 (s, 1H), 6.68 (dd, 1H, *J* = 15.9, 5.4), 6.76 (s, br, 1H), 7.19 (m, 6H), 8.09 (d, 1H, *J* = 8.1), 8.14 (d, 1H, *J* = 7.8), 8.58 (d, 1H, *J* = 7.5); Anal. (C₂₉H₃₉N₅O₇) C, H, N.

### Example 3 - Preparation of Compound A-2: Ethyl-3-((Isoxazole-5'-carbonyl)-L-Leu-L-Phe-L-Gln)-E-Propenoate

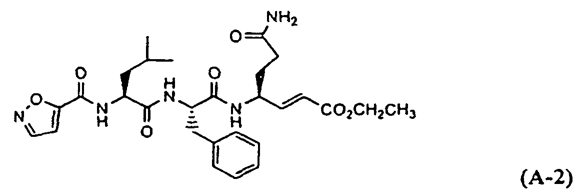

### Preparation of Intermediate Ethyl-3-((Isoxazole-5'-carbonyl)-L-Leu-L-Phe-L-(Tr-Gln))-E-Propenoate

This compound was prepared from ethyl-3-(Boc-L-Leu-L-Phe-L-(Tr-*Gln*))-E-propenoate and isoxazole-5-carbonyl chloride using the procedure described above (Example 2) for the preparation of ethyl-3-((5'-methylisoxazole-3'-carbonyl)-L-Leu-L-Phe-L-(Tr-*Gln*))-E-propenoate: IR (cm⁻¹) 3282, 1643, 1530; ¹H NMR (CDCl₃) δ 0.87 (t, 6H, *J* = 6.6), 1.29 (t, 3H, *J* = 7.2), 1.49-1.64 (m, 3H), 1.69-1.80 (m, 1H), 1.90-1.96 (m, 1H), 2.30 (t, 2H, *J* = 7.2), 2.92-2.96 (m, 1H), 3.02-3.09 (m, 1H), 4.17 (q, 2H, *J* = 7.2), 4.42-4.48 (m, 3H), 5.69 (d, 1H, *J* = 15.3), 6.65 (s, br, 1H), 6.66 (dd, 1H, *J* = 15.9, 5.4), 6.76-6.79 (m, 2H), 7.00-7.31 (m, 22H), 8.24 (s, 1H); Anal. (C₄₇H₅₁N₅O₇•0.75 H₂O) C, H, N.

### Preparation of Ethyl-3-((Isoxazole-5'-carbonyl)-L-Leu-L-Phe-L-Gln)-E-Propenoate

The title compound was prepared from ethyl-3-((isoxazole-5'-carbonyl)-L-Leu-L-Phe-L-(Tr-*Gln*))-E-propenoate using a procedure analogous to that described above (Example 2) for the preparation of ethyl-3-((5'-methylisoxazole-3'-carbonyl)-L-Leu-L-Phe-L-*Gln*)-E-propenoate: mp = 217-220°C; IR (cm⁻¹) 3302, 1655, 1541; ¹H NMR (DMSO-*d*₆) δ 0.81 (d, 3H, *J* = 6.0), 0.86 (d, 3H, *J* = 6.0), 1.21 (t, 3H, *J* = 7.2), 1.42-1.75 (m, 5H), 2.04 (t, 2H, *J* = 7.2), 2.78-2.87 (m, 1H), 2.94-3.01 (m, 1H), 4.11 (q, 2H, *J* = 7.2), 4.37 (m, 1H), 4.41-4.52 (m, 2H), 5.64 (d, 1H, *J* = 15.6), 6.68 (dd, 1H, *J* = 15.9, 5.4), 6.76 (s, br, 1H), 7.12-7.19 (m, 7H), 8.02 (d, 1H, *J* = 8.1), 8.20 (d, 1H, *J* = 8.1), 8.74 (d, 1H, *J* = 1.8), 8.94 (d, 1H, *J* = 7.8); Anal. (C₂₈H₃₇N₅O₇) C, H, N.

### Example 4 - Preparation of Compound A-3: Ethyl-3-((5'-Methylisoxazole-3'-carbonyl)-L-α-(t-Butyl-Gly)-L-(4-Me-Phe)-L-Gln)-E-Propenoate

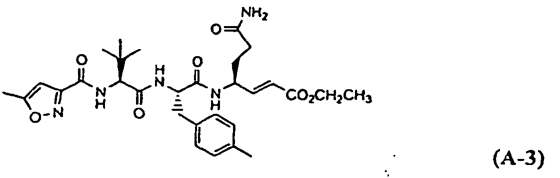

### Preparation of Intermediate Ethyl-3-(Boc-L-(4-Me-Phe)-L-(Tr-Gln))-E-Propenoate

Ethyl-3-(H₂N-L-(Tr-*Gln*))-E-propenoate•HCl (prepared as described in Example 2 above, 1.37 g, 3.10 mmol) was dissolved in DMF (10 mL) at 23°C. Diisopropylethylamine (1.08 mL, 6.20 mmol) was added, followed by Boc-L-(4-Me-Phe)-OH (0.87 g 3.10 mmol). The reaction was cooled to 0°C. HATU (1.18 g, 3.10 mmol) was added, and the reaction allowed to warm to room temperature. The DMF was removed *in vacuo*. The residue was dissolved in EtOAc (30 mL), and the organic phase was washed sequentially with 10% HCl solution (25 mL), saturated NaHCO₃ solution (25 mL), H₂O (25 mL), and brine (25 mL). The solvent was dried (MgSO₄) and filtered, and the residue purified by flash column chromatography (gradient elution, 0→0.75% CH₃OH in CHCl₃) to give ethyl-3-(Boc-L-(4-Me-Phe)-L-(Tr-*Gln*))-E-propenoate (1.48 g, 68% yield) as a white amorphous solid: IR (cm⁻¹) 1713, 1655, 1491, 1175; ¹H NMR (DMSO-d₆) δ 1.20 (t, 3H, *J* = 7.0), 1.30 (s, 9H), 1.62-1.66 (m, 2H), 2.23 (s, 3H), 2.32 (m, 2H), 2.72 (m, 1H), 2.84 (m, 1H), 4.07-4.09 (m, 1H), 4.10 (q, 2H, *J* = 7.0), 4.38 (m, 1H), 5.64 (d, 1H, *J* = 15.5), 6.72 (dd, 1H, *J* = 15.5, 5.5), 6.88 (d, 1H, *J* = 8.0), 7.04 (d, 2H, *J* = 7.7), 7.10 (d, 2H, *J* = 7.7), 7.14-7.28 (m, 15H), 8.02 (d, 1H, *J* = 8.0), 8.53 (s, 1H); Anal. (C₄₃H₄₉N₃O₆) C, H, N.

### Preparation of Intermediate Ethyl-3-(Boc-L-α-(t-Butyl-Gly)-L-(4-Me-Phe)-L-(Tr-Gln))-E-Propenoate

Ethyl-3-(Boc-L-(4-Me-Phe)-L-(Tr-*Gln*))-E-propenoate (1.45 g, 2.06 mmol) was dissolved in 1,4-dioxane (27 mL), and a solution of HCl in 1,4-dioxane (4.0 M, 14 mL) was added. The reaction was stirred at room temperature for 4 hours. The solvent was removed by evaporation, and the residue taken up in EtOAc (50 mL). The organic phase was washed with saturated NaHCO₃ solution (50 mL) and then brine (50 mL), dried (MgSO₄), and the solvent removed under reduced pressure to give 1.23 g of an off-white amorphous solid. This material was coupled with Boc-L-α-(t-Butyl-Gly)-OH using the procedure described for the synthesis of ethyl-3-(Boc-L-(4-Me-Phe)-L-(Tr-*Gln*))-E-propenoate above to afford ethyl-3-(Boc-L-α-(t-Butyl-Gly)-L-(4-Me-Phe)-L-(Tr-*Gln*))-E-propenoate (49% yield) as a white amorphous solid: IR (cm⁻¹) 1655, 1507, 1248, 1171; ¹H NMR (DMSO-*d*₆) δ 0.81 (s, 9H), 1.21 (t, 3H, *J* = 7.0), 1.37 (s, 9H), 1.52-1.70 (m, 2H), 2.22 (s, 3H), 2.26-2.28 (m, 2H), 2.73-2.91 (m, 2H), 3.86 (d, 1H, *J* = 9.6), 4.05-4.14 (m, 2H), 4.31-4.36 (m, 1H), 4.47-4.55 (m, 1H), 5.54 (d, 1H, *J* = 15.4), 6.37 (d, 1H, *J* = 9.6), 6.65 (dd, 1H, *J* = 15.8, 5.5), 7.01 (d, 2H, *J* = 8.1), 7.07 (d, 2H, *J* = 7.7), 7.11-7.32 (m, 15H), 8.03 (d, 1H, *J* = 8.1), 8.10 (d, 1H, *J* = 7.7), 8.49 (s, 1H); Anal. (C₄₉H₆₀N₄O_{7•}0.4H₂O) C, H, N.

### Preparation of Intermediate Ethyl-3-((5'-Methylisoxazole-3'-carbonyl)-L-α-(t-Butyl-Gly)-L-(4-Me-Phe)-L-(Tr-Gln))-E-Propenoate

Ethyl-3-(Boc-L-α-(t-Butyl-Gly)-L-(4-Me-Phe)-L-(Tr-*Gln*))-E-propenoate was deprotected using the procedure described for the deprotection of ethyl-3-(Boc-L-(4-Me-Phe)-L-(Tr-*Gln*))-E-propenoate above, and the resulting amine (0.22 g, 0.30 mmol) was dissolved in CH₂Cl₂ (3 mL). Pyridine (0.025 mL, 0.32 mmol) was added, and the reaction was cooled to 0°C. 5-Methylisoxazole-3-carbonyl chloride (0.046 g, 0.32 mmol) was added. The reaction was allowed to warm to room temperature, and was stirred for one hour. The solvent was removed *in vacuo*, and the residue subjected to flash column chromatography (gradient elution, 0→1% CH₃OH in CH₂Cl₂) to afford ethyl-3-((5'-methylisoxazole-3'-carbonyl)-L-α-(t-Butyl-Gly)-L-(4-Me-Phe)-L-(Tr-*Gln*))-E-propenoate (0.19 g, 77% yield) as a white amorphous solid: IR (cm⁻¹) 1651, 1518; ¹H NMR (DMSO-d₆) δ 0.88 (s, 9H), 1.20 (t, 3H, *J* = 7.0), 1.55-1.67 (m, 2H), 2.14 (s, 3H), 2.18-2.28 (m, 2H), 2.45 (s, 3H), 2.70-2.77 (m, 1H), 2.86-2.93 (m, 1H), 4.07-4.14 (m, 2H), 4.46 (d, 1H, *J* = 9.6), 4.50-4.55 (m, 1H), 5.54 (d, 1H, *J* = 15.8), 6.59 (s, 1H), 6.65 (dd, 1H, *J* = 15.8, 5.5, 15.8), 6.95 (d, 2H, *J* = 8.1), 7.05 (d, 2H, *J* = 8.1), 7.13-7.28 (m, 15H), 7.60 (d, 1H, *J* = 9.6), 8.13 (d, 1H, *J* = 8.1), 8.41 (d, 1H, *J* = 8.1), 8.51 (s, 1H); Anal. (C₄₉H₅₅N₅O₇) C, H, N.

### Preparation of Product Ethyl-3-((5'-Methylisoxazole-3'-carbonyl)-L-α-(t-Butyl-Gly)-L-(4-Me-Phe)-L-Gln)-E-Propenoate

Ethyl-3-((5'-methylisozazole-3'-carbonyl)-L-α-(t-Butyl-Gly)-L-(4-Me-Phe)-L-(Tr-*Gln*))-E-propenoate (0.17 g, 0.20 mmol) was dissolved in CH₂Cl₂ (4 mL) at 23°C. Trifluoroacetic acid (0.4 mL) was added, and the reaction was stirred at room temperature for six hours. The solvents were removed *in vacuo*, and the residue was subjected to flash column chromatography (gradient elution, 0→2% CH₃OH in CH₂Cl₂) to afford ethyl-3-((5'-methylisoxazole-3'-carbonyl)-L-α-(t-Butyl-Gly)-L-(4-Me-Phe)-L-*Gln*)-E-propenoate (0.085 g, 73% yield) as a white amorphous solid: IR (cm⁻¹) 1661, 1541, 1206; ¹H NMR (DMSO-*d*₆) δ 0.88 (s, 9H), 1.21 (t, 3H, *J* = 7.0), 1.60-1.73 (m, 2H), 2.01-2.06 (m, 2H), 2.14 (s, 3H), 2.50 (s, 3H), 2.70-2.77 (m, 1H), 2.86-2.93 (m, 1H), 4.07-4.14 (m, 2H), 4.34-4.37 (m, 1H), 4.45 (d, 1H, *J* = 9.6), 4.50-4.55 (m, 1H), 5.57 (d, 1H, *J* = 15.8), 6.60 (s, 1H), 6.66 (dd, 1H, *J* = 15.8, 5.5), 6.75 (s, br, 1H), 6.96 (d, 2H, *J* = 8.1), 7.06 (d, 2H, *J* = 7.7), 7.17 (s, br, 1H), 7.6.5 (d, 1H, *J* = 9.6), 8.14 (d, 1H, *J* = 8.1), 8.40 (d, 1H, *J* = 7.7); Anal. (C₃₀H₄₁N₅O₇·0.5TFA•0.5H₂O) C, H, N.

### Example 5 - Preparation of Compound A-4: Ethyl-3-((5'-Methylisoxazole-3'-carbonyl)-L-α-(t-Butyl-Gly)-L-(4-F-Phe)-L-Gln)-E-Propenoate

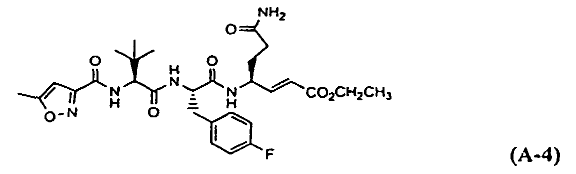

### Preparation of Intermediate Ethyl-3-(Boc-L-(4-F-Phe)-L-(Tr-Gln))-E-Propenoate

Boc-L-(4-F-Phe)-OH (1.41 g, 5.0 mmol) was dissolved in THF (50 mL). Ethyl-3-(H₂N-L-(Tr-*Gln*))-E-propenoate•HCl (prepared as described in Example 2 above, 1.0 g, 5.0 mmol) was added, followed by Et3N (0.70 mL, 5.0 mmol). Carbonyldiimidazole (0.81 g, 5.0 mmol) was added, and the reaction was stirred at room temperature for 20 hours. The solvent was removed *in vacuo*, and the residue subjected to flash column chromatography (gradient elution, 0→1% CH₃OH in CH₂Cl₂) to afford ethyl-3-(Boc-L-(4-F-Phe)-L-(Tr-*Gln*))-E-propenoate (1.13 g, 32% yield) as a white amorphous solid: IR (cm⁻¹) 1712, 1666, 1510, 1169; ¹H NMR (DMSO-d₆) δ 1.20 (t, 3H, *J* = 7.0), 1.29 (s, 9H), 1.61-1.70 (m, 2H), 2.27-2.34 (m, 2H), 2.74-2.78 (m, 1H), 2.86-2.90 (m, 1H), 4.06-4.13 (m, 3H), 4.36-4.40 (m, 1H), 5.58 (d, 1H, *J* = 15.6), 6.71 (dd, 1H, *J* = 15.6, 5.5), 6.98 (d, 1H, *J* = 8.1), 7.03-7.09 (m, 2H), 7.14-7.28 (m, 17H), 8.06 (d, 1H, *J* = 8.1), 8.53 (s, 1H); LRMS (M+Na) 730.

### Preparation of Intermediate Ethyl-3-(Boc-L-α-(t-Butyl-Gly)-L-(4-F-Phe)-L-(Tr-Gln))-E-Propenoate

Ethyl-3-(Boc-L-(4-F-Phe)-L-(Tr-*Gln*))-E-propenoate was deprotected and coupled with Boc-L-α-(t-Butyl-Gly)-OH using the procedures described above to prepare ethyl-3-(Boc-L-α-(t-Butyl-Gly)-L-(4-Me-Phe)-L-(Tr-*Gln*))-E-propenoate, to provide ethyl-3-(Boc-L-α-(t-Butyl-Gly)-L-(4-F-Phe)-L-(Tr-*Gln*))-E-propenoate (54% yield) as a white amorphous solid: IR (cm⁻¹) 1720, 1651, 1506, 1168; ¹H NMR (DMSO-*d*₆) δ 0.80 (s, 9H), 1.20 (t, 3H, *J* = 7.0), 1.36 (s, 9H), 1.53-1.67 (m, 2H), 2.23-2.28 (m, 2H), 2.79-2.94 (m, 2H), 3.85 (d, 1H, *J* = 9.9), 4.09 (q, 2H, *J* = 7.0), 4.31-4.35 (m, 1H), 4.53-4.55 (m, 1H), 5.46 (d, 1H, *J* = 15.8), 6.36 (d, 1H, *J* = 9.2), 6.64 (dd, 1H, *J* = 15.8, 5.5), 6.97-7.03 (m, 2H), 7.13-7.28 (m, 17H), 8.08 (d, 1H, *J* = 8.1), 8.14 (d, 1H, *J* = 8.1), 8.49 (s, 1H); Anal. (C₄₈H₅₇N₄O₇F) C, H, N.

### Preparation of Intermediate Ethyl-3-((5'-Methylisoxazole-3'-carbonyl)-L-α-(t-Butyl-Gly)-L-(4-F-Phe)-L-(Tr-Gln))-E-Propenoate

Ethyl-3-(Boc-L-α-(t-Butyl-Gly)-L-(4-F-Phe)-L-(Tr-*Gln*))-E-propenoate was deprotected and coupled with 5-methylisoxazole-3-carbonyl chloride using the procedures described above to prepare ethyl-3-((5'-methylisoxazole-3'-carbonyl)-L-α-(t-Butyl-Gly)-L-(4-Me-Phe)-L-(Tr-*Gln*))-E-propenoate to afford ethyl-3-((5'-methylisoxazole-3'-carbonyl)-L-α-(t-Butyl-Gly)-L-(4-F-Phe)-L-(Tr-*Gln*))-E-propenoate (74% yield) as a white amorphous solid: IR (cm⁻¹) 1659, 1535, 15 10; ¹H NMR (DMSO-d₆) δ 0.88 (s, 9H), 1.20 (t, 3H, *J* = 7.4), 1.52-1.67 (m, 2H), 2.23-2.28 (m, 2H), 2.45 (s, 3H), 2.75-2.82 (m, 1H), 2.89-2.96 (m, 1H), 4.09 (q, 2H, *J* = 7.0), 4.32-4.36 (m, 1H), 4.45 (d, 1H, *J* = 9.6), 4.50-4.55 (m, 1H), 5.44 (d, 1H, *J* = 15.6), 6.58 (s, 1H), 6.63 (dd, 1H, *J* = 15.6, 5.5, 15.6), 6.93-6.99 (m, 2H), 7.13-7.28 (m, 17H), 7.64 (d, 1H, *J* = 9.6), 8.16 (d, 1H, *J* = 8.5), 8.46 (d, 1H, *J* = 8.1), 8.51 (s, 1H); Anal. (C₄₈H₅₂N₅O₇F) C, H, N.

### Preparation of Ethyl-3-((5'-Methylisoxazole-3'-carbonyl)-L-α-(t-Butyl-Gly)-L-(4-F-Phe)-L-Gln)-E-Propenoate

Ethyl-3-[(5'-methylisoxazole-3'-carbonyl)-L-α-(t-Butyl-Gly)-L-(4-F-Phe)-L-(Tr-*Gln*)]-E-propenoate was deprotected using a procedure analogous to that described above for the preparation of ethyl-3-((5'-methylisoxazole-3'-carbonyl)-L-α-(t-Butyl-Gly)-L-(4-Me-Phe)-L-*Gln*)-E-propenoate to provide ethyl-3-((5'-methylisoxazole-3'-carbonyl)-L-α-(t-Butyl-Gly)-L-(4-F-Phe)-L-*Gln*)-E-propenoate (80% yield) as a white amorphous solid: IR (cm⁻¹) 1653, 1543, 1223; ¹H NMR (DMSO-d₆) δ 0.88 (s, 9H), 1.21 (t, 3H, *J* = 7.0), 1.59-1.75 (m, 2H), 2.01-2.06 (m, 2H), 2.46 (s, 3H), 2.75-2.82 (m, 1H), 2.89-2.96 (m, 1H), 4.09 (q, 2H, *J* = 7.0), 4.33-4.36 (m, 1H), 4.44 (d, 1H, *J* = 9.6), 4.50-4.58 (m, 1H), 5.47 (d, 1H, *J* = 15.8), 6.59 (s, 1H), 6.64 (dd, 1H, *J* = 15.8, 5.5), 6.75 (s, br, 1H), 6.94-7.00 (m, 2H), 7.16 (s, br, 1H), 7.18-7.23 (m, 2H), 7.69 (d, 1H, *J* = 9.6), 8.16 (d, 1H, *J* = 8.1), 8.44 (d, 1H, *J* = 8.1); Anal. (C₂₉H₃₈N₅O₇F) C, H, N.

### Example 7 - Preparation of Compound A-6: Ethyl-3-[(5'-Methylisoxazole-3'-carbonyl)-L-Val-L-(4-F-Phe)-L-Gln]-E-Propenoate

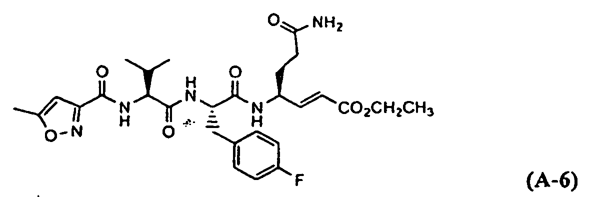

### Preparation of Intermediate Ethyl-3-{Boc-L-(4-F)-Phe-L-(Tr-Gln)}-E-Propenoate

This compound was prepared from ethyl-3-(Boc-L-(Tr-*Gln*))-E-propenoate and Boc-L-(4-F)-Phe-OH using a procedure like that described above (Example 2) for the preparation of ethyl-1-(Boc-L-Phe-L-(Tr-*Gln*))-E-propenoate: IR (cm⁻¹) 3328, 1707, 1506, 1168; ¹H NMR (CDCl₃) δ 1.29 (t, 3H, *J* = 7.2), 1.37 (s, 9H), 1.66-1.78 (m, 1H), 1.88-1.98 (m, 1H), 2.32 (t, 2H, *J* = 6.6), 2.85-2.92 (m, 1H), 2.97-3.04 (m, 1H), 4.18 (q, 2H, *J* = 7.2), 4.52 (m, 1H), 4.96 (m, 1H), 5.60 (d, 1H, *J* = 15.6), 6.56 (d, 1H, *J* = 8.1), 6.66 (dd, 1H, *J* = 15.6, 5.1), 6.80 (s, br, 1H), 6.92-6.98 (m, 2H), 7.08-7.12 (m, 2H), 7.17-7.23 (m, 6H), 7.24-7.33 (m, 10H); Anal. (C₄₂H₄₆FN₃O₆) C, H, N.

### Preparation of Intermediate Ethyl-3-{Boc-L-Val-L-(4-F)-Phe-L-(Tr-Gln)}-E-Propenoate

This compound was prepared from ethyl-3-{Boc-L-(4-F)-Phe-L-(Tr-*Gln*)}-E-propenoate and Boc-L-Val-OH in the manner described above (Example 2) for the preparation of ethyl-3-{Boc-L-Leu-L-Phe-L-(Tr-*Gln*)}-E-propenoate: IR (cm⁻¹) 3319, 1657, 1511, 1172; ¹H NMR (CDCl₃) δ 0.78 (d, 3H, *J* = 6.9), 0.87 (d, 3H, *J* = 6.9), 1.29 (t, 3H, *J* = 7.2), 1.37 (s, 9H), 1.69-1.79 (m, 1H), 1.93-2.06 (m, 2H), 2.33 (t, 2H, *J* = 7.2), 2.97-3.04 (m, 1H), 3.73-3.77 (m, 1H), 4.18 (q, 2H, *J* = 7.2), 4.42-4.54 (m, 2H), 4.80 (d, 1H, *J* = 6.9), 5.61 (dd, 1H, *J* = 15.6, 1.5), 6.44 (d, 1H, *J* = 7.8), 6.69 (dd, 1H, *J* = 15.9, 5.4), 6.71 (s, br, 1H), 6.92-6.98 (m, 2H), 7.07-7.13 (m, 2H), 7.18-7.31 (m, 16H), 8.02 (s, 1H); Anal. (C₄₇H₅₅FN₄O₇) C, H, N.

### Preparation of Intermediate Ethyl-3-{(5'-Methylisoxazole-3'-carbonyl)-L-Val-L-(4-F)-Phe-L-(Tr-Gln)}-E-Propenoate

This compound was prepared from ethyl-3-{Boc-L-Val-L-(4-F)-Phe-L-(Tr-*Gln*)}-E-propenoate and 5-methylisoxazole-3-carbonyl chloride in a manner like that described above (Example 2) for the preparation of ethyl-3-{(5'-methylisoxazole-3'-carbonyl)-L-Leu-L-Phe-L-(Tr-*Gln*)}-E-propenoate: IR (cm⁻¹) 3319, 1657, 1511, 1172; ¹H NMR (CDCl₃) δ 0.84 (d, 3H, *J* = 6.9), 0.89 (d, 3H, *J* = 6.9), 1.30 (t, 3H, *J* = 7.2), 1.68-1.80 (m, 1H), 1.95-2.06 (m, 1H), 2.08-2.17 (m, 1H), 2.34 (t, 2H, *J* = 7.2), 2.44 (s, 3H), 2.87-2.94 (m, 1H), 3.01-3.08 (m, 1H), 4.18 (q, 2H, *J* = 7.2), 4.48-4.56 (m, 2H), 5.68 (dd, 1H, *J* = 15.6, 1.8), 6.23 (s, 1H), 6.39 (d, 1H, *J* = 7.8), 6.70 (dd, 1H, *J* = 15.9, 5.4), 6.84-6.90 (m, 3H), 7.04-7.08 (m, 4H), 7.17-7.30 (m, 16H); Anal. (C₄₇H₅₀N₅O₇) C, H, N.

### Preparation of Product Ethyl-3-{(5'-Methylisoxazole-3'-carbonyl)-L-Val-L-(4-F)-Phe-L-Gln}-E-Propenoate

Ethyl-3-{(5'-Methylisoxazole-3'-carbonyl)-L-Val-L-(4-F)-Phe-L-(Tr-*Gln*)}-E-propenoate was deprotected using the procedure described above (Example 2) for the preparation of ethyl-3-{(5'-methylisoxazole-3'-carbonyl)-L-Leu-L-Phe-L-*Gln*}-E-propenoate: IR (cm⁻¹) 3284, 1652, 1542; ¹H NMR (DMSO-*d*₆) δ 0.76 (d, 3H, *J* = 6.9), 0.79 (d, 3H, *J* = 6.9), 1.20 (t, 3H, *J* = 7.2), 1.57-1.76 (m, 2H), 1.96-2.06 (3H), 2.46 (s, 3H), 2.75-2.83 (m, 1H), 2.89-2.96 (m, 1H), 4.09 (q, 2H, *J* = 7.2), 4.13-4.25 (m, 1H), 4.35 (m, 1H), 4.49-4.56 (m, 1H), 5.53 (d, 1H, *J* = 15.6), 6.57 (s, 1H), 6.66 (dd, 1H, *J* = 15.6, 5.4), 6.75 (s, br, 1H), 6.97-7.03 (m, 2H), 7.17-7.24 (m, 3H), 8.1 (d, 1H, *J* = 7.8), 8.24 (d, 1H, *J* = 8.7), 8.32 (d, 1H, *J* = 8.1); Anal. (C₂₈H₃₆N₅O₇) C, H, N.

### Example 10 - Preparation of Compound B-1: Ethyl-3-{(5'-Methylisoxazole-3'-carbonyl)-L-ValΨ(COCH₂)-L-(4-F-Phe)-L-Gln}-E-Propenoste

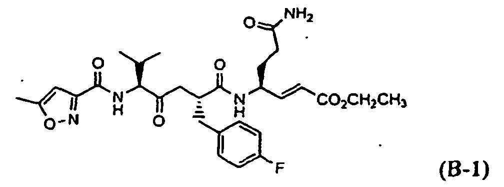

### Preparation of Intermediate trans-6-Methyl-hept-4-enoic Acid.

A solution of isobutyraldehyde (9.59 g, 133 mmol, 1 equiv.) in THF (50 mL) was added dropwise via addition funnel to a solution of vinylmagnesium bromide (133 mL of a 1.0 M solution in THF, 133 mmol, 1.0 equiv.) in THF (300 mL) at 0°C. Upon completion of the addition, the reaction mixture was stirred for 30 min. at 0°C, and then ethyl malonyl chloride (17.0 mL, 133 mmol, 1.0 equiv.) was added. After stirring for 1 h at 0°C, the reaction mixture was partitioned between saturated NH₄Cl (150 mL) and a 1:1 mixture of EtOAc and hexanes (2 x 200 mL). The combined organic layers were dried over Na₂SO₄ and were concentrated. Purification of the residue by filtration through silica gel (eluting with 5% EtOAc in hexanes) afforded the intermediate malonate ester (11.5 g, 40% yield). This material was not characterized, but was combined (neat) with Ti(OEt)₄ (1.13 mL, 5.39 mmol, 0.10 equiv.) and was heated to 190°C for 4 h, and then was cooled to 60°C. EtOH (50 mL) and 6.0 M KOH (50 mL) were added sequentially, and the brown reaction mixture was refluxed for 4 h. After cooling to 23°C, the reaction mixture was filtered through a medium frit, and the filtrate was partitioned between water (150 mL) and Et₂O (2 x 150 mL). The aqueous layer was then acidified to pH = 2 (as indicated by pH paper) with concentrated HCl and was extracted with a 1:1 mixture of EtOAc and hexanes (2 x 150 mL). The combined organic layers were dried over Na₂SO₄, concentrated, and the residue was distilled at reduced pressure to afford trans-6-methyl-hept-4-enoic acid (3.58 g, 47%) as a colorless liquid: bp 107-112°C (1 Torr); IR (cm⁻¹) 2960, 1711; ¹H NMR (CDCl₃) δ 0.96 (d, 6H, *J* = 6.5), 2.18-2.45 (m, 5H), 5.31-5.50 (m, 2H); Anal. (C₈H₁₄O₂) C, H.

### Preparation of Intermediate trans-6-Methyl-hept-4-enoic Acid (2R-hydrox-y-1R-methyl-2-phenyl-ethyl)-methyl Amide.

Oxalyl chloride (2.25 mL, 25.8 mmol, 1.05 equiv.) was added to a solution of trans-6-methyl-hept-4-enoic acid (3.50 g, 24.6 mmol, 1 equiv.) and *N,N*-dimethylformamide (0.03 mL, 0.39 mmol, 0.016 equiv.) in benzene (60 mL) at 23°C. The reaction mixture was stirred at 23°C for 2 h, and then was concentrated under reduced pressure. The resulting oil was dissolved in THF (20 mL) and was added via cannula to a solution of (1*R*,2*R*)-(-)-pseudoephedrine (3.87 g, 23.4 mmol, 1 equiv.) and triethylamine (3.92 mL, 28.1 mmol, 1.2 equiv.) in THF (150 mL) at 0°C. The reaction mixture was stirred at 0°C for 30 min., then was partitioned between half-saturated NH₄Cl (150 mL) and EtOAc (2 x 150 mL). The combined organic layers were dried over Na₂SO₄, concentrated, and the residue purified by flash column chromatography (gradient elution 40→50% EtOAc in hexanes) to afford trans-6-methyl-hept-4-enoic acid (2*R*-hydroxy-1*R*-methyl-2-phenyl-ethyl)-methyl amide (6.31 g, 93%) as a viscous oil: R_{*f*} = 0.35 (50% EtOAc in hexanes); IR (cm⁻¹) 3382, 1622; ¹H NMR (CDCl₃, mixture of rotamers) δ 0.96 (d, *J* = 6.8), 0.97 (d, *J* = 6.5), 1.11 (d, *J* = 6.9), 2.18-2.59 (m), 2.82 (s), 2.92 (s), 3.99-4.04 (m), 4.32-4.42 (m), 4.44-4.49 (m), 4.55-4.62 (m), 5.32-5.49 (m), 7.24-7.42 (m); Anal. (C₁₈H₂₇NO₂) C, H, N.

### Preparation of Intermediate trans-6-Methyl-2S-(4-fluorobenzyl)-hept-4-enoic Acid (2R-Hydroxy-1R-methyl-2-phenylethyl)methyl Amide.

*n*-Butyllithium (32.5 mL of a 1.6 M solution in hexanes, 52.0 mmol, 3.1 equiv.) was added to a suspension of anhydrous lithium chloride (7.18 g, 169 mmol, 10 equiv.) and diisopropylamine (7.80 mL, 55.7 mmol, 3.3 equiv.) in THF (250 mL) at -78°C. The reaction mixture was stirred for 30 min. at -78°C, was maintained at 0°C for 5 min., and subsequently cooled again to -78°C. trans-6-Methyl-hept-4-enoic acid (2*R*-hydroxy-1*R*-methyl-2-phenylethyl)-methyl amide (4.91 g, 17.0 mmol, 1 equiv) in THF (50 mL) was added via cannula, and the resulting solution was stirred at -78°C for 1.75 h, maintained at 0°C for 20 min., stirred at 23°C for 5 min., and then was cooled again to 0°C. A solution of 4-fluorobenzyl bromide (6.34 mL, 50.9 mmol, 3 equiv.) in THF (15 mL) was added and the reaction mixture was stirred at 0°C for 30 min., which was then partitioned between half-saturated NH₄Cl (230 mL) and a 1:1 mixture of EtOAc and hexanes (200 mL, 2 x 150 mL). The combined organic layers were dried over Na₂SO₄ and were concentrated. Purification of the residue by flash column chromatography (gradient elution 20→40% EtOAc in hexanes) provided trans-6-methyl-2*S*-(4-fluorobenzyl)-hept-4-enoic acid (2*R*-hydroxy-1*R*-methyl-2-phenylethyl)methyl amide (6.33 g, 94%) as a viscous oil: R_{*f*}= 0.38 (40% EtOAc in hexanes); IR (cm⁻¹) 3378, 1614; ¹H NMR (CDCl₃, mixture of rotamers) δ 0.85-0.95 (m), 0.96 (d, *J* = 6.8), 2.10-2.32 (m), 2.34-2.46 (m), 2.58 (s), 2.67-2.79 (m), 2.82-2.94 (m), 3.00-3.18 (m), 3.94 (br), 4.37-4.52 (m), 5.24-5.42 (m), 5.44-5.56 (m), 6.89-7.01 (m), 7.08-7.14 (m), 7.19-7.38 (m); Anal. (C₂₅H₃₂FNO₂) C, H, N.

### Preparation of Intermediate 5S-(1R-Bromo-2-methylpropyl)-3R-(4-fluorobenzyl)dihydrofuran-2-one.

*N*-Bromosuccinimide (2.93 g, 16.5 mmol, 1.05 equiv.) was added in small portions over 10 minutes to a solution of trans-6-methyl-2*S*-(4-fluorobenzyl)-hept-4-enoic acid (2*R*-hydroxy-1*R*-methyl-2-phenylethyl)methyl amide (6.24 g, 15.7 mmol, equiv.) and glacial acetic acid (4.49 mL, 78.4 mmol, 5 equiv.) in a 4:1 mixture of THF and H₂O (165 mL) at 0°C. The resulting yellow solution was stirred for 15 min. at 0°C, and then was warmed to 23°C and subsequently refluxed for 45 min. After cooling to 23°C, the reaction mixture was partitioned between half-saturated NaHCO₃ (200 mL) and a 1:1 mixture of EtOAc and hexanes (2 x 200 mL, 100 mL). The combined organic layers were dried over Na₂SO₄ and were concentrated. Flash chromatographic purification of the residue (gradient elution 4→10% EtOAc in hexanes) gave 5*S*-(1*R*-bromo-2-methylpropyl)-3*R*-(4-fluorobenzyl)dihydrofuran-2-one (4.14 g, 80%) as a pale yellow oil (containing approximately 5-10% unidentified impurities by ¹H NMR): R_{*f*} = 0.56 (25% EtOAc in hexanes); IR (cm⁻¹) 1772; ¹H NMR (CDCl₃, major isomer) δ 0.94 (d, 3H, *J* = 6.5), 1.00 (d, 3H, *J* = 6.8), 2.05-2.35 (m, 3H), 2.83 (dd, 1H, *J* = 13.6, 8.4), 2.92-3.03 (m, 1H), 3.11 (dd, 1H, *J* = 13.6, 4.7), 3.90 (dd, 1H, *J* = 9.0, 3.7), 4.33-4.40 (m, 1H), 6.98-7.06 (m, 2H), 7.14-7.20 (m, 2H); Anal. (C₁₅H₁₈BrFO₂) C, H.

### Preparation of Intermediate 5S-(1S-Azido-2-methylpropyl)-3R-(4-fluorobenzyl)dihydrofuran-2-one.

A suspension of sodium azide (1.90 g, 29.2 mmol, 2.5 equiv.) and 5*S*-(1*R*-bromo-2-methylpropyl)-3R-(4-fluorobenzyl)dihydrofuran-2-one (3.85 g, 11.7 mmol, 1 equiv.) in *N,N*-dimethylformamide (40 mL) was heated at 50°C for 67 hours. The reaction mixture was cooled to 23°C and was partitioned between half-saturated NaCl (200 mL) and a 1:1:1 mixture of EtOAc, hexanes and acetone (2 x 200 mL, 100 mL). The combined organic layers were dried over Na₂SO₄, concentrated, and the residue purified by flash column chromatography (gradient elution 9→17% EtOAc in hexanes) to give 5*S*-(1*S*-azido-2-methylpropyl)-3*R*-(4-fluorobenzyl)dihydrofuran-2-one (2.10 g, 62%) as a white solid (containing approximately 5-10% unidentified impurities by ¹H NMR): mp 91-96°C; R_{*f*} = 0.44 (25% EtOAc in hexanes); IR (cm⁻¹) 2097, 1772; ¹H NMR (CDCl₃, major isomer) δ 0.99 (d, 3H, *J* = 6.5), 1.02 (d, 3H, *J* = 6.8), 1.95-2.20 (m, 3H), 2.78-2.88 (m, 1H), 2.94 (dd, 1H, *J* = 7.0, 4.2), 3.03-3.17 (m, 2H), 4.37-4.43 (m, 1H), 6.97-7.09 (m, 2H), 7.14-7.21 (m, 2H).

### Preparation of Intermediate {2-Methyl-1S-(4R-(4-fluorobenzyl)-5-oxotetrahydrofuran-2S-yl)propyl}-carbamic Acid tert-Butyl Ester.

A suspension of 5*S*-(1*S*-azido-2-methylpropyl)-3*R*-(4-fluorobenzyl)dihydrofuran-2-one (2.02 g, 6.93 mmol, 1 equiv.), di-*tert*-butyl dicarbonate (2.12 g, 9.71 mmol, 1.4 equiv.) and Pd/C (10%, 0.20 g) in CH₃OH (100 mL) was stirred under a hydrogen atmosphere (balloon) for 16 hours. The reaction mixture was vacuum filtered through Whatman #3 paper and concentrated. Purification of the residue by flash column chromatography (15% EtOAc in hexanes) provided {2-methyl-1*S*-(4*R*-(4-fluorobenzyl)-5-oxotetrahydrofuran-2*S*-yl)propyl}-carbamic acid *tert*-butyl ester (1.58 g, 62%) as a white foam: R_{*f*} = 0.80 (5% MeOH in CH₂Cl₂); IR (cm⁻¹) 3331, 1766, 1702; ¹H NMR (CDCl₃) δ 0.93 (d, 3H, *J* = 6.8), 0.95 (d, 3H, *J* = 6.5), 1.41 (s, 9H), 1.71-1.83 (m, 1H), 1.95-2.06 (m, 1H), 2.16-2.27 (m, 1H), 2.80 (dd, 1H, *J* = 13.5, 8.6), 2.88-2.99 (m, 1H), 3.09 (dd, 1H, *J* = 13.5, 4.4), 3.32-3.40 (m, 1H), 4.42-4.48 (m, 2H), 6.95-7.03 (m, 2H), 7.11-7.18 (m, 2H); Anal. (C₂₀H₂₈FNO₄) C, H, N.

### Preparation of Intermediate Ethyl-3-{Boc-L-ValΨ(COCH₂)-L-(4-F-Phe)-L-(Tr-Gln)}-E-Propenoate.

Lithium hydroxide (9.62 mL of a 1 M aqueous solution, 9.62 mmol, 5 equiv.) was added to a solution of {2-methyl-1*S*-(4*R*-(4-fluorobenzyl)-5-oxotetrahydrofuran-2*S*-yl)propyl}-carbamic acid *tert*-butyl ester (0.703 g, 1.92 mmol, 1 equiv.) in DME (25 mL) at 23°C. The resulting suspension was stirred at 23°C for 30 min., and then was partitioned between 10% KHSO₄ (50 mL) and CH₂Cl₂ (3 x 100 mL). The combined organic layers were dried over Na₂SO₄, concentrated, and the residue dissolved in CH₂Cl₂ (30 mL). Powdered 4A molecular sieves (0.70 g), 4-methylmorpholine *N*-oxide (0.451 g, 3.85 mmol, 2 equiv.), and tetrapropylammonium perruthenate (0.068 g, 0.19 mmol, 0.10 equiv.) were added sequentially. The resulting dark reaction mixture was stirred for 1.33 hours at 23°C, then was vacuum filtered through Whatman #3 paper and then through Whatman #5 paper. The filtrate was concentrated under reduced pressure to provide a dark residue, which was dissolved in CH₂Cl₂ (30 mL). Crude ethyl-3-(H₂N-L-(Tr-*Gln*))-E-propenoate•HCl (2.30 mmol, 1.2 equiv., prepared as described in Example 2 for the preparation of ethyl-3-{(5'-methylisoxazole-3'-carbonyl)-L-Leu-L-Phe-L-*Gln*}-E-propenoate), 4-methylmorpholine (0.846 mL, 7.69 mmol, 4 equiv.), HOBt (0.390 g, 2.89 mmol, 1.5 equiv.), and EDC (0.553 g, 2.88 mmol, 1.5 equiv.) were added sequentially, and the reaction mixture was stirred for 19 hours at 23°C and then was partitioned between brine (100 mL) and CH₂Cl₂ (3 x 100 mL). The combined organic layers were dried over Na₂SO₄ and were concentrated. Purification of the residue by flash column chromatography (gradient elution 35→40% EtOAc in hexanes) provided ethyl-3-{Boc-L-ValΨ(COCH₂)-L-(4-F-Phe)-L-(Tr-*Gln*)}-E-propenoate (0.820 g, 53%) as a tan foam: R_{*f*} = 0.50 (50% EtOAc in hexanes); IR (cm⁻¹) 3307, 1708, 1666; ¹H NMR (CDCl₃) δ 0.67 (d, 3H, *J* = 6.8), 0.92 (d, 3H, *J* = 6.8), 1.28 (t, 3H, *J* = 7.2), 1.40 (s, 9H), 1.53-1.67 (m, 1H), 1.91-2.04 (m, 2H), 2.32-2.41 (m, 2H), 2.46-2.55 (m, 1H), 2.63 (dd, 1H, *J* = 12.1, 5.9), 2.69-2.80 (m, 1H), 2.83 (dd, 1H, *J* = 12.1, 8.2), 3.03 (dd, 1H, *J* = 17.7, 10.0), 4.05-4.11 (m, 1H), 4.17 (q, 2 H, *J* = 7.2), 4.40-4.50 (m, 1H), 4.84 (d, 1H, *J* = 8.4), 5.38 (d, 1H, *J* = 15.7), 6.01 (d, 1H, *J* = 8.4), 6.60 (dd, 1H, *J* = 15.7, 5.0), 6.92-6.99 (m, 2H), 7.03-7.12 (m, 3H), 7.17-7.30 (m, 15H); Anal. (C₄₈H₅₆FN₃O₇) C, H, N.

### Preparation of Product Ethyl-3-{(5'-Methylisoxazole-3'-carbonyl)-L-ValΨ(COCH₂)-L-(4-F-Phe)-L-Gln}-E-Propenoate

Ethyl-3-{Boc-L-ValΨ(COCH₂)-L-(4-F-Phe)-L-(Tr-*Gln*)}-E-propenoate was converted to product ethyl-3-{(5'-methylisoxazole-3'-carbonyl)-L-ValΨ(COCH₂)-L-(4-F-Phe)-L-*Gln*}-E-propenoate in a manner analogous to that described in Example 2 above for the conversion of ethyl-3-{Boc-L-Leu-L-Phe-L-(Tr-*Gln*)}-E-propenoate to product ethyl-3-{(5'-methylisoxazole-3'-carbonyl)-L-Leu-L-Phe-L-*Gln*}-E-propenoate: mp = 220°C (dec); R_{*f*}= 0.35 (10% CH₃OH in CH₂Cl₂); IR (cm⁻¹) 3277, 1715, 1643; ¹H NMR (DMSO-*d*₆) δ 0.81 (d, 3H, *J* = 6.2), 0.87 (d, 3H, *J* = 6.9), 1.21 (t, 3H, *J* = 6.5), 1.59-1.67 (m, 2H), 2.03 (s, br, 2H), 2.21-2.24 (m, 1H), 2.46 (s, 3H), 2.57-2.68 (m, 3H), 2.80-2.95 (m, 2H), 4.09 (q, 2H, *J* = 6.5), 4.30-4.34 (m, 2H), 5.41 (d, 1H, *J* = 15.5), 6.55 (s, 1H), 6.61 (dd, 1H, *J* = 15.5, 5.5), 6.73 (s, 1H), 6.99-7.16 (m, 5H), 8.01 (d, 1H, *J* = 7.8), 8.69 (d, 1H, *J* = 8.7); Anal. (C₂₉H₃₇FN₄O₇) C, H, N.

### Example 11 - Preparation of Compound A-10: Ethyl-3-((5'-Methylisoxazole-3'-carbonyl)-L-Leu-NCH₃-L-Phe-L-Gln}-E-Propenoate

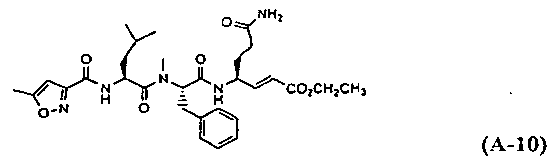

### Preparation of Intermediate Boc-L-Leu-NCH₃-L-Phe-OCH₃

NCH₃-L-Phe-OCH₃•HCl (1.4 g) was dissolved in CH₂Cl₂ (50 mL) and poured into a combination of aqueous (aq) 1 N NaOH (7 mL) and saturated aqueous NaHCO₃ (25 mL). After mixing, the organic phase was separated and the aqueous phase was washed with CH₂Cl₂ (3 x 50 mL). The combined organic phases were dried over Na₂SO₄ and evaporated to give the free amine as a clear colorless oil (1.14 g, 5.90 mmol). A solution of this amine and (iPr)₂NEt (1.13 mL, 6.49 mmol) in DMF (10 mL) was added dropwise to a 0°C solution of Boc-L-Leu-OH (1.50 g, 6.49 mmol) and HOBt (0.877 g, 6.49 mmol) in DMF (10 mL). DCC (1.47 g, 7.12 mmol) was added. The reaction mixture was stirred at 0°C for 1 h, and was then stirred at 23°C for 48 h. The mixture was filtered to remove the precipitate (ppt) and the filtrate was evaporated. The residue was dissolved in CH₂Cl₂ (200 mL), washed with saturated aqueous NaHCO₃ (40 mL), dried over Na₂SO₄ and evaporated. The residue was purified by flash column chromatography (25% EtOAc in hexanes) to give Boc-L-Leu-NCH₃-L-Phe-OCH₃ as a white solid (2.04 g, 85%): mp = 126-127°C; IR (cm⁻¹) 3401, 3319, 1743, 1708, 1649; ¹H NMR (CDCl₃) (major isomer) δ 0.92 (d, 3H, *J* = 6.8), 0.95 (d, 3H, *J* = 6.5), 1.32-1.48 (m, 2H), 1.41 (s, 9H), 1.61-1.77 (m, 1H), 2.90 (s, 3H), 3.04 (dd, 1H, *J* = 14.5, 10.5), 3.37 (dd, 1H, *J* = 14.5, 5.5), 3.72 (s, 3H), 4.48-4.57 (m, 1H), 4.98-5.04 (m, 1H), 5.20 (dd, 1H, *J* = 10.5, 5.5), 7.16-7.32 (m 5H); Anal. (C₂₂H₃₄N₂O₅) C, H, N.

### Preparation of Intermediate Boc-L-Leu-NCH₃-L-Phe-OH

Boc-L-Leu-NCH₃-L-Phe-OCH₃ (0.625 g, 1.54 mmol) was dissolved in CH₃OH (20 mL) and cooled to 0°C. Aqueous NaOH (6.15 mL of a 2N solution, 12.3 mmol) was added dropwise. The reaction mixture was stirred for 3 h at 23°C, and then poured into 10% aqueous KHSO₄ (150 mL). This mixture was extracted with CH₂Cl₂ (3 x 100 mL), and the combined organic phases were dried over Na₂SO₄ and evaporated to give Boc-L-Leu-NCH₃-L-Phe-OH as a white foam (0.617 g, quant.), which was used without purification.

### Preparation of Intermediate Ethyl-3-{Boc-L-Leu-NCH₃-L-Phe-L-(Tr-Gln)}-E-Propenoate

This intermediate was prepared from Boc-L-Leu-NCH₃-L-Phe-OH and ethyl-3-{H₂N-L-(Tr-*Gln*)}-E-propenoate•HCl (prepared as described in Example 2) in a manner analogous to that described for the preparation of ethyl-3-{Boc-L-Leu-L-Phe-L-(Tr-*Gln*)}-E-propenoate in Example 2 above: IR (cm⁻¹) 3295, 1713, 1672, 1649; ¹H NMR (CDCl₃)(mixture of isomers) δ 0.65 (d, *J* = 6.2), 0.66 (d, *J* = 6.5), 0.84 (d, *J* = 6.5), 0.88 (d, *J* = 6.5), 1.02-1.22 (m), 1.23-1.38 (m), 1.33 (s), 1.41 (s), 1.55-1.82 (m), 1.89-2.07 (m), 2.23-2.30 (m), 2.90 (s), 2.94 (s), 3.01 (dd, *J* = 14.6, 10.9), 3.03-3.13 (m), 3.26-3.37 (m), 3.27 (dd, *J* = 14.6, 3.4), 3.42-3.54 (m), 4.00-4.22 (m), 4.37-4.73 (m), 4.82-4.89 (m), 5.63-5.70 (m), 5.95 (dd, *J* = 15.9, 1.2), 6.23-6.28 (m), 6.66-6.75 (m), 6.79-6.89 (m), 7.09-7.34 (m), 8.14 (d, *J* = 8.7); Anal. (C₄₉H₆₀N₄O₇) C, H, N.

### Preparation of Product Ethyl-3-{(5'-Methylisoxazole-3'-carbonyl)-L-Leu-NCH₃-L-Phe-L-Gln}-E-Propenoate

Ethyl-3-{Boc-L-Leu-NCH₃-L-Phe-L-(Tr-*Gln*)}-E-propenoate was converted to product ethyl-3-{(5'-methylisoxazole-3'-carbonyl)-L-Leu-NCH₃-L-Phe-L-*Gln*}-E-propenoate in a manner analogous to that described in Example 2 above for the conversion of ethyl-3-{Boc-L-Leu-L-Phe-L-(Tr-*Gln*)}-E-propenoate to product ethyl-3-{(5'-methylisoxazole-3'-carbonyl)-L-Leu-L-Phe-L-*Gln*}-E-propenoate: R_{*f*}= 0.23 (5% CH₃OH in CH₂Cl₂); IR (cm⁻¹) 3295, 1713, 1666, 1637; ¹H NMR (CDCl₃) (mixture of isomers) δ 0.65 (d, *J* = 6.5), 0.71 (d, *J* = 6.5), 0.93 (d, *J* = 6.5), 0.94 (d, *J* = 6.5), 1.30 (t, *J* = 7.2), 1.24-1.73 (m), 1.81-2.22 (m), 2.45 (s), 2.48 (s), 2.86-2.93 (m), 2.96 (s), 2.97 (s), 3.03-3.14 (m), 3.21-3.31 (m), 3.48 (dd, *J* = 14.0, 5.9), 4.19 (q, *J* = 7.2), 4.20 (q, *J* = 7.2), 4.38-4.45 (m), 4.52-4.70 (m), 4.74-4.81 (m), 5.62-5.67 (m), 5.73-5.79 (m), 5.81 (dd, *J* = 15.6, 1.6), 5.99 (dd, *J* = 15.6, 1.6), 6.03-6.09 (m), 6.35 (s), 6.39 (s), 6.40-6.45 (m), 6.77-6.94 (m), 7.42 (d, *J* = 7.2), 8.13 (d, *J* = 7.8).

### Example 12 - Preparation of Compound C-1: Ethyl-3-{(5'-Niethylisoxazole-3'-carbonyl)-L-Val-O-L-(4-F-Phe)-L-Gln}-E-Propenoate

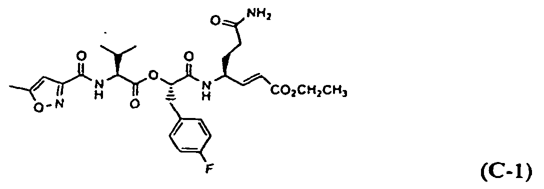

### Preparation of Intermediate Allyl (S)-I-Hydroxy-3-(4-fluorophenyl)propionate

In a flask fitted with a thermometer and a reflux condenser was dissolved methyl (*S*)-1-hydroxy-3-(4-fluorophenyl)propionate (prepared from L-H₂N-(4-F-Phe)-OCH₃ by the method described in Hoffman et al., *Tetrahedron* **1992**, vol. 48, 3007) (0.99 g, 5.0 mmol) in allyl alcohol (50 mL). Titanium tetraisopropoxide (1.53 mL, 5.0 mmol) was added, and the reaction brought to 90°C for 3.5 h. The reaction was cooled to room temperature and poured into 250 mL of 1:1 EtOAc/saturated NH₄Cl solution. The organic phase was separated and washed with water (100 mL), brine (100 mL), dried (MgSO₄), and the solvent removed. The residue was subjected to flash column chromatography eluting with a gradient of 5-10% EtOAc/hexanes to afford 0.77 g (68%) of allyl (*S*)-1-hydroxy-3-(4-fluorophenyl)propionate as a clear liquid: R_{*f*} = 0.21 (15% EtOAc/hexanes); IR (neat) 3470 (broad), 1734, 1510, 1221 cm⁻¹; ¹H NMR (DMSO-d₆) δ 1.24-1.27 (m, 1H), 2.92-2.99 (m, 1H), 3.09-3.15 (m, 1H), 4.43-4.47 (m, 1H), 4.65 (d, 2H, *J* = 5.9), 5.28-5.37 (m, 2H), 5.86-5.95 (m, 1H), 6.95-7.01 (m, 2H), 7.16-7.21 (m, 2H).

### Preparation of Intermediate Boc-L-Val-O-L-(4-F-Phe)-OCH₂CH=CH₂

Allyl (*S*)-1-hydroxy-3-(4-fluorophenyl)propionate (0.070 g, 0.31 mmol) was dissolved in CH₂Cl₂ (20 mL). Boc-L-Val-OH (0.068 g, 0.31 mmol) was added, followed by DMAP (0.004 g, 0.03 mmol) and DCC (0.067 g, 0.33 mmol). The reaction was stirred at room temperature overnight, and the solvent was removed *in vacuo*. The residue was subjected to flash column chromatography eluting with a gradient of 3-5% EtOAc/hexanes. The Boc-L-Val-O-L-(4-F-Phe)-OCH₂CH=CH₂ product was obtained as 0.12 g (90%) of a clear oil: R_{*f*}= 0.18 (10% EtOAc/hexanes); IR (neat) 1752, 1717 1510 cm⁻¹; ¹H NMR (DMSO-*d*₆) δ 0.80-0.85 (m, 6H), 1.36 (s, 9H), 1.97-2.04 (m, 1H), 3.07-3.15 (m, 2H), 3.89-3.94 (m, 1H), 4.51-4.55 (m, 2H), 5.17-5.30 (m, 3H), 5.75-5.84 (m, 1H), 7.06-7.17 (m, 3H), 7.27-7.32 (m, 2H); Anal. (C₂₂H₃₀NO₆F) C, H, N.

### Preparation of Intermediate Ethyl-3-{Boc-L-Val-O-L-(4-F-Phe)-L-(Tr-Cln)}-E-Propenoate

Boc-L-Val-O-L-(4-F-Phe)-OCH₂CH=CH₂ (0.65 g, 1.52 mmol) was dissolved in THF (15 mL). Tetrakis(triphenylphosphine)palladium(0) (0.035 g, 0.03 mmol) was added, and the reaction stirred 5 min. at 23°C. Morpholine (0.16 mL, 1.83 mmol) was added dropwise, and the reaction stirred at room temperature for 2 h. The solvent was removed *in vacuo*, and the residue taken up in 50 mL of 4:1 hexanes/Et₂O. The product was extracted into sat. NaHCO₃ solution (50 mL), and the organic phase discarded. The aqueous phase was acidified to pH = 1-2 with solid KHSO₄, and the product re-extracted into EtOAc (50 mL). The organic phase was washed with brine (50 mL), dried (MgSO₄), and concentrated to give 0.50 g (86%) of the free acid as a clear oil. This material was dissolved in DMF (6 mL). Diisopropylethylamine (0.43 mL, 2.50 mmol) was added, followed by ethyl-3-{H₂N-L-(Tr-*Gln*)}-E-propenoate•HCl (prepared as described in Example 2 above, 0.55 g, 1.25 mmol). The reaction was cooled to 0°C. HATU (0.48 g, 1.25 mmol) was added, and the reaction allowed to warm to room temperature. The DMF was removed *in vacuo*. The residue was dissolved with EtOAc (30 mL), and the organic phase washed consecutively with 10% HCl solution (25 mL), saturated NaHCO₃ solution (25 mL), H₂O (25 mL), and brine (25 mL). The organic layer was dried (MgSO₄), filtered, and concentrated, and the residue was purified by flash column chromatography (0→1.0% MeOH/CH₂Cl₂) to give 0.40 g (39%) of ethyl-3-{Boc-L-Val-O-L-(4-F-Phe)-L-(Tr-*Gln*)}-E-propenoate as a white amorphous solid: R_{*f*} = 0.25 (3% MeOH/CHCl₃); IR(KBr) 1691, 1512, 1159 cm⁻¹; ¹H NMR (DMSO-*d*₆) δ 0.78-0.83 (m, 6H), 1.20 (t, 3H, *J* = 7.0), 1.35 (s, 9H), 1.58-1.66 (m, 2H), 1.96-2.02 (m, 1H), 2.19-2.33 (m, 2H), 2.99-3.02 (m, 2H), 3.82-3.87 (m, 1H), 4.09 (q, 2H, *J* = 7.0), 4.33-4.37 (m, 1H), 5.03-5.08 (m, 1H), 5.60 (d, 1H, *J* = 15.8), 6.67 (dd, 1H, *J* = 15.8, 5.5), 7.02-7.08 (m, 2H), 7.14-7.28 (m, 18H), 8.12 (d, 1H, *J* = 8.1), 8.59 (s, 1H); Anal. (C₄₇H₅₄N₃O₈F) C, H, N.

### Preparation of Product Ethyl-3-{(5'-Methylisoxazole-3'-carbonyl)-L-Val-O-L-(4-F-Phe)-L-Gln}-E-Propenoate

Ethyl-3-{Boc-L-Val-O-L-(4-F-Phe)-L-(Tr-*Gln*)}-E-propenoate was converted to product ethyl-3-{(5'-methylisoxazole-3'-carbonyl)-L-Val-O-L-(4-F-Phe)-L-*Gln*}-E-propenoate in a manner analogous to that described in Example 2 above for the conversion of ethyl-3-{Boc-L-Leu-L-Phe-L-(Tr-*Gln*)}-E-propenoate to product ethyl-3-{(5'-methylisoxazole-3'-carbonyl)-L-Leu-L-Phe-L-*Gln*}-E-propenoate: R_{*f*}= 0.05 (3% MeOH/CHCl₃); IR (KBr) 1746, 1719, 1661, 1549 cm⁻¹; ¹H NMR(DMSO-*d*₆) δ 0.87 (d, 3 H, *J* = 6.6), 0.92 (d, 3H, *J* = 6.6), 1.20 (t, 3H, *J* = 7.0), 1.61-1.74 (m, 2H), 1.96-2.01 (m, 2H), 2.15-2.22 (m, 1H), 2.46 (s, 3H), 3.00-3.03 (m, 2H), 4.10 (q, 2H, *J* = 7.0), 4.27-4.32 (m, 1H), 4.33-4.38 (m, 1H), 5.06-5.11 (m, 1H), 5.63 (d, 1H, *J* = 15.6), 6.54 (s, 1H), 6.68 (dd, 1H, *J* = 15.6, 5.5), 6.78 (s, br, 1H), 6.95-7.00 (m, 2H), 7.20-7.24 (m, 3H), 8.06 (d, 1H, *J* = 8.1), 8.87 (d, 1H, *J* = 7.7); Anal. (C₂₈H₃₅N₄O₈F) C, H, N.

### Example 13 - Preparation of Compound A-11: Ethyl-3-{(5'-Methylisoxazole-3'-carbonyl)-L-(2-Naphth)-NCH₃-L-(4-F-Phe)-L-Gln}-E-Propenoate

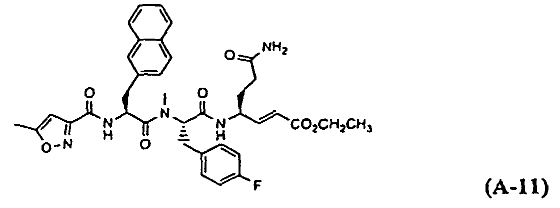

### Preparation of Intermediate Ethyl-3-{Boc-NCH₃-L-(4-F-Phe)-L-(Tr-Gln)}-E-Propenoate

Boc-(4-F-Phe)-OH (4.46 g, 15.75 mmol, 1 equiv.) and CH₃I (7.84 mL, 126 mmol, 8 equiv.) were dissolved in dry THF (100 mL) and cooled to 0°C. NaH (1.89 g, 47.25 mmol, 3 equiv.) was added to this solution with vigorous stirring. After stirring at 23°C for 24 h, EtOAc (3 mL) and H₂O (3 mL) were added carefully to the mixture, and the resulting suspension was evaporated to dryness. After dissolving in H₂O (100 mL), the reaction mixture was washed with Et₂O (2 x 100 mL). The aqueous layer was acidified to pH = 3 with 10% citric acid solution, and then extracted with EtOAc (2 x 100 mL). The combined EtOAc extracts were washed successively with half-saturated NaHCO₃ (150 mL), 5% Na₂S₂O₃ (150 mL), and H₂O (150 mL), dried over Na₂SO₄, and concentrated to give Boc-NCH₃-(4-F-Phe)-OH as a pale yellow foam (4.37 g, 80%), which was used without further purification: ¹H NMR (CDCl₃, mixture of isomers) δ 1.35 (s), 1.40 (s), 2.69 (s), 2.75 (s), 2.96-3.13 (m), 3.23-3.33 (m), 4.54-4.58 (m), 4.76-4.81 (m), 6.96-7.01 (m), 7.15-7.17 (m).

A solution of HCl in 1,4-dioxane (4.0 M, 15 mL) was added to a solution of ethyl-3-(Boc-L-(Tr-*Gln*))-E-propenoate (prepared as described in Example 2 above, 3.57 g, 6.73 mmol, 1 equiv.) in the same solvent (15 mL) at 23°C. After 2 h, the volatiles were removed under reduced pressure. The residue was dissolved in CH₂Cl₂ (50 mL), and Boc-NCH₃-(4-F-Phe)-OH (prepared as in the preceding paragraph, 2.0 g, 6.73 mmol, 1.0 equiv.), HOBt (1.23 g, 9.09 mmol, 1.5 equiv), 4-methylmorpholine (2.0 mL, 18.19 mmol, 3 equiv.), and EDC (1.74 g, 9.09 mmol, 1.5 equiv.) were added sequentially. The reaction mixture was stirred at 23°C overnight, and then was partitioned between water (100 mL) and CH₂Cl₂ (2 x 100 mL). The combined organic layers were dried over Na₂SO₄, concentrated, and the residue was purified by flash column chromatography (30% EtOAc in hexane) to afford ethyl-3-{Boc-NCH₃-L-(4-F-Phe)-L-(Tr-*Gln*)}-E-propenoate (4.07 g, 84%) as white foam: IR(cm⁻¹) 1666, 1510, 1167; ¹H NMR (CDCl₃, mixture of isomers) δ 1.29 (t, *J* = 7.2), 1.37 (s), 1.65-1.75 (m), 1.95-2.06 (m), 2.29-2.33 (m), 2.66 (s), 2.91-2.99 (m), 3.22-3.29 (m), 4.18 (q, *J* = 7.2), 4.52-4.58 (m), 5.68 (d, *J* = 15.9), 6.45 (d, *J* = 8.4), 6.74 (dd, *J* = 15.6, 5.4), 6.91-6.99 (m), 7.11-7.33 (m); Anal. (C₄₃H₄₈FN₃O₆) C, H, N.

### Preparation of Intermediate Ethyl-3-{Boc-L-(2-Naphth)-NCH₃-L-(4-F-Phe)-L-(Tr-Gln)}-E-Propenoate

A solution of HCl in 1,4-dioxane (4.0 M, 3 mL), was added to a solution of ethyl-3-{Boc-NCH₃-L-(4-F-Phe)-L-(Tr-*Gln*)}-E-propenoate (0.388 g, 0.54 mmol, 1 equiv.) in the same solvent (3 mL) at 23°C. After 2 h, the volatiles were removed under reduced pressure. The residue was dissolved in DMF (10 mL), cooled at 0°C, and DIEA (0.188 mL, 1.08 mmol, 2 equiv.), Boc-L-(2-Naphth)-OH (0.170 g, 0.54 mmol, 1.0 equiv.) and HATU (0.205 g, 0.54 mmol, 1 equiv.) were added sequentially. The reaction mixture was stirred at 23°C for 1 h. The volatiles were removed under reduced pressure, and the resulting residue was taken into EtOAc (50 mL), and washed with 0.5 N HCl (50 mL), saturated NaHCO₃ (50 mL) and brine (50 mL). The organic layer was dried over Na₂SO₄, concentrated, and the residue was purified by flash column chromatography (40% EtOAc in hexane) to afford ethyl-3-{Boc-L-(2-Naphth)-NCH₃-L-(4-F-Phe)-L-(Tr-*Gln*)}-E-propenoate (0.437 g, 88%) as white foam: IR (cm⁻¹) 1656, 1509, 1162; ¹H NMR (CDCl₃, mixture of isomers) δ 0.88 (t, *J* = 7.2), 1.27 (s), 1.30 (s), 1.48-1.58 (m), 1.64-1.67 (m), 1.97-2.11 (m), 2.23-2.28 (m), 2.42-2.50 (m), 2.62-2.69 (m), 2.80 (s), 2.90 (s), 3.00-3.07 (m), 3.15-3.20 (m), 3.25-3.32 (m), 4.18 (q, *J* = 7.2), 4.42-4.46 (m), 4.53-4.56 (m), 4.61-4.66 (m), 4.72-4.82 (m), 5.94-5.00 (m), 5.63 (d, *J* = 15.6), 6.12 (d, *J* = 15.6), 6.60 (dd, *J* = 15.6, 5.4), 6.75-6.89 (m), 6.70-7.08 (m), 7.19-7.30 (m), 7.41-7.50 (m), 7.70-7.82 (m), 8.80 (d, *J* = 8.4); Anal. (C₅₆H₅₉FN₄O₇) C, H, N.

### Preparation of Product Ethyl-3-{(5'-Methylisoxazole-3'-carbonyl)-L-(2-Naphth)-NCH₃-L-(4-F-Phe)-L-Gln}-E-Propenoate

Ethyl-3-{Boc-L-(2-Naphth)-NCH₃-L-(4-F-Phe)-L-(Tr-*Gln*)}-E-propenoate was converted to product ethyl-3-{(5'-methylisoxazole-3'-carbonyl)-L-(2-Naphth)-NCH₃-L-(4-F-Phe)-L-*Gln*}-E-propenoate in a manner analogous to that described in Example 2 above for the conversion of ethyl-3-{Boc-L-Leu-L-Phe-L-(Tr-*Gln*)}-E-propenoate to product ethyl-3-{(5'-methylisoxazole-3'-carbonyl)-L-Leu-L-Phe-L-*Gln*}-E-propenoate IR (cm⁻¹) 3296, 1654, 1510; ¹H NMR (DMSO-*d*₆, mixture of isomers) δ 1.17-1.24 (m), 1.62-1.78 (m), 2.04-2.15 (m), 2.38 (s), 2.42 (s), 2.71-2.79 (m), 2.84 (s), 2.87-2.92 (m), 3.03 (s), 3.15 (d, *J* = 7.5), 3.98-4.06 (m), 4.08-4.12 (m), 4.38-4.42 (m), 4.94 (m), 5.03-5.07 (m), 5.09-5.18 (m), 5.66-5.82 (m), 6.42 (s), 6.43 (s), 6.66-6.81 (m), 6.88-6.94 (m), 7.01-7.06 (m), 7.13-7.17 (m), 7.24-7.34 (m), 7.43-7.46 (m), 7.56 (s), 7.76-7.84 (m), 8.08 (d, *J* = 7.8), 8.60 (d, *J* = 8.4), 9.01 (d, *J* = 7.2); Anal. (C₃₇H₄₀FN₅O_{7•}0.75H₂O) C, H, N.

### Example 14 - Preparation of Compound A-9: Ethyl-3-{(5'-Methylisoxazole-3'-carbonyl)-L-His-NCH₃-L-(4-F-Phe)-L-Gln}-E-Propenoate

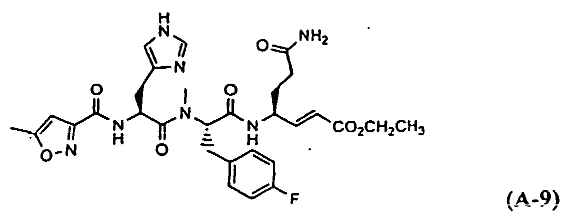

Ethyl-3-{Boc-NCH₃-L-(4-F-Phe)-L-(Tr-*Gln*)}-E-propenoate (described in Example 13 above) was converted to product ethyl-3-{(5'-methylisoxazole-3'-carbonyl)-L-His-NCH₃-L-(4-F-Phe)-L-*Gln*}-E-propenoate in a manner analogous to the preparation of product ethyl-3-{(5'-methylisoxazole-3'-carbonyl)-L-(2-Naphth)-NCH₃-L-(4-F-Phe)-L-*Gln*}-E-propenoate described in Example 13 above (utilizing Boc-L-(Tr-His)-OH in lieu of Boc-L-(2-Naphth)-OH): IR (cm⁻¹) 3302, 1665, 1202; 1 H NMR (DMSO-d₆, mixture of isomers) δ 1.21 (t, *J* = 7.2), 1.70-1.78 (m), 2.05-2.09 (m), 2.41 (s), 2.44 (s), 2.69-3.26 (m), 4.11 (q, *J* = 7.2), 4.38-4.53 (m), 5.07-5.19 (m), 6.51-5.84 (m), 6.44 (s), 6.48 (s), 6.63-6.86 (m), 6.89-7.01 (m), 7.09-7.19 (m), 7.23-7.42 (m), 8.02 (d, *J* = 8.7), 8.15 (d, *J* = 8.1), 8.59 (d, *J* = 8.7), 8.94 (s), 9.04 (s), 9.14 (d, *J* = 6.9); Anal. (C₃₀H₃₆FN₇O₇•TFA•H₂O) C, H, N.

### Example 15 - Preparation of Compound A-12: Ethyl-3-{(5'-Methylisoxazole-3'-carbonyl)-L-Leu-NCH₃-L-(4-F-Phe)-L-Gln}-E-Propenoate

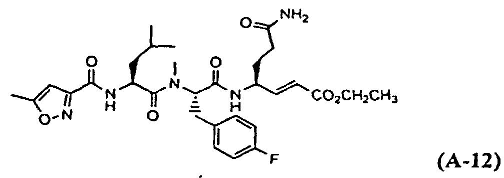

Ethyl-3-{Boc-NCH₃-L-(4-F-Phe)-L-(Tr-*Gln*)}-E-propenoate (described in Example 13 above) was converted to product ethyl-3-{(5'-methylisoxazole-3'-carbonyl)-L-Leu-NCH₃-L-(4-F-Phe)-L-*Gln*}-E-propenoate in a manner analogous to the preparation of product ethyl-3-{(5'-methylisoxazole-3'-carbonyl)-L-(2-Naphth)-NCH₃-L-(4-F-Phe)-L-*Gln*}-E-propenoate described in Example 13 above (utilizing Boc-L-Leu-OH in lieu of Boc-L-(2-Naphth)-OH): IR (cm⁻¹) 3325, 1663, 1171; ¹H NMR (DMSO-*d*₆, mixture of isomers) δ 0.64-0.67 (m), 0.86-0.88 (m), 1.17-1.23 (m), 1.32-1.40 (m), 1.59-1.75 (m), 1.98-2.07 (m), 2.42 (s), 2.45 (s), 2.08 (s), 2.86-2.93 (m), 2.99 (s), 3.12-3.20 (m), 4.05-4.15 (m), 4.41-4.50 (m), 4.82-5.07 (m), 5.62 (d, *J* = 15.9), 5.86 (d, *J* = 15.9), 6.51 (s), 6.53 (s), 6.68-6.73 (m), 6.93-6.99 (m), 7.09-7.21 (m), 7.26-7.31 (m), 8.03 (d, *J* = 7.8), 8.08 (d, *J* = 7.8), 8.41 (d, *J* = 8.1), 8.94 (d, *J* = 7.2); Anal. (C₃₀H₄₀FN₅O₇•1.25CH₂Cl₂) C, H, N.

### Example 16 - Preparation of Compound A-13: Ethyl-3-{(5'-Methylisoxazole-3'-carbonyl)-L-(1-Naphth)-NCH₃-L-(4-F-Phe)-L-Gln}-E-Propenoate

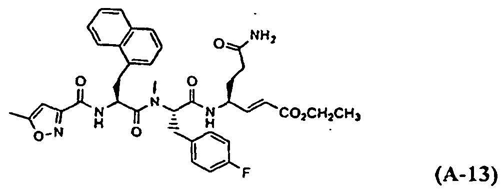

Ethyl-3-{Boc-NCH₃-L-(4-F-Phe)-L-(Tr-*Gln*)}-E-propenoate (described in Example 13 above) was converted to product ethyl-3-{(5'-methylisoxazole-3'-carbonyl)-L-(1-Naphth)-NCH₃-L-(4-F-Phe)-L-*Gln*}-E-propenoate in a manner analogous to the preparation of product ethyl-3-{(5'-methylisoxazole-3'-carbonyl)-L-(2-Naphth)-NCH₃-L-(4-F-Phe)-L-*Gln*}-E-propenoate described in Example 13 above, but utilizing Boc-L-(1-Naphth)-OH in lieu of Boc-L-(2-Naphth)-OH: IR (cm⁻¹) 3308, 1659, 1169; ¹H NMR (DMSO-*d*₆, mixture of isomers) δ 1.16-1.23 (m), 1.61-1.78 (m), 1.98-2.02 (m), 2.07-2.12 (m), 2.41 (s), 2.43 (s), 2.77 (s), 2.78 (s), 2.84-2.87 (m), 2.93-3.03 (m), 3.08-3.14 (m), 3.31-3.38 (m), 4.00-4.15 (m), 4.27-4.32 (m), 4.40-4.46 (m), 4.58-4.64 (m), 5.07-5.17 (m), 5.57-5.73 (m), 6.45 (s), 6.57-6.61 (m), 6.71-6.88 (m), 6.89-6.91 (m), 7.11-7.19 (m), 7.31-7.38 (m), 7.50-7.58 (m), 7.73-7.78 (m), 7.83-7.94 (m), 8.08 (d, *J* = 8.1), 8.13 (d, *J* = 8.7), 8.62 (d, *J* = 8.1), 9.14 (d, *J* = 8.1); Anal. (C₃₇H₄₀FN₅O₇) C, H, N.

### Examples 21 through 30

For Examples 21-30, the following Compounds **(A-14)** through **(A-23)**, respectively, were prepared using synthetic methods analogous to those described above for compounds of the formula I-A:

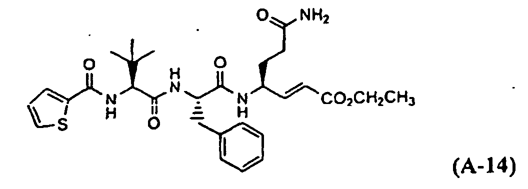

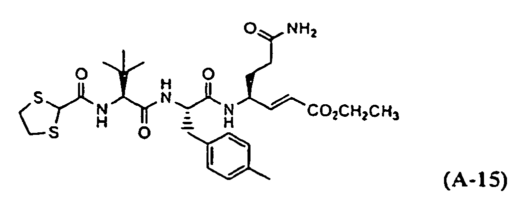

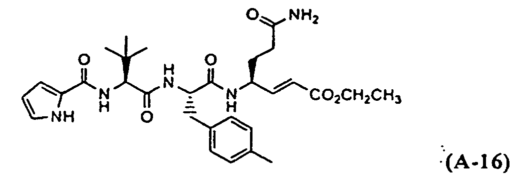

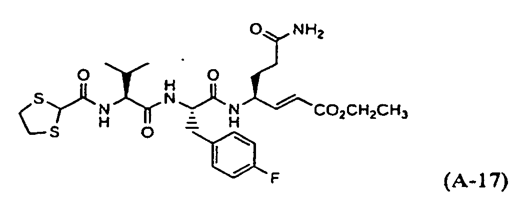

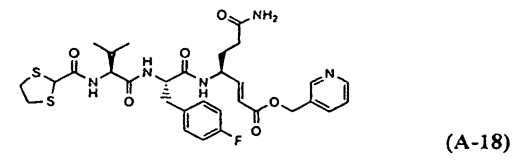

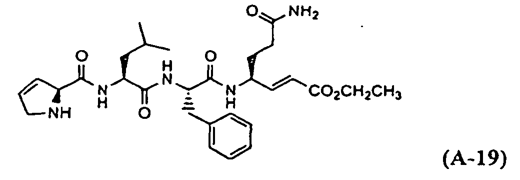

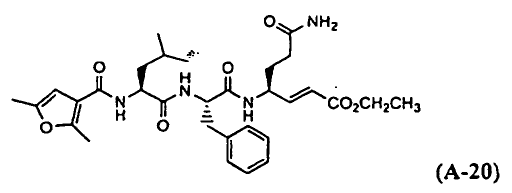

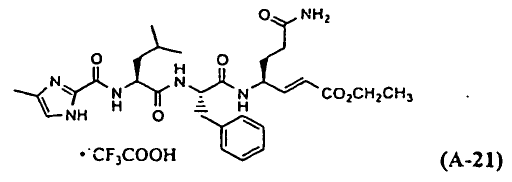

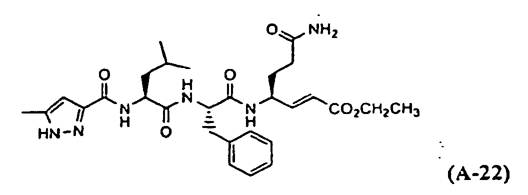

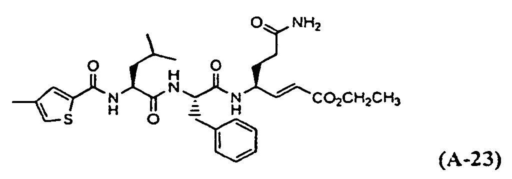

### Example 31 - Preparation of Comparison Compound #2: Ethyl-3-(Cbz-L-Leu-L-Phe-L-Gln)-E-Propenoate

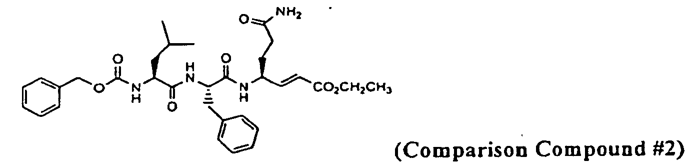

### Preparation of Intermediate [Boc-L-(Tr-Gln)]-N(OMe)Me

Isobutyl chloroformate (4.77 mL, 36.8 mmol, 1.0 equiv.) was added to a solution of *N*-α-Boc-γ-trityl-L-glutamine (18.7 g, 36.7 mmol, 1 equiv.) and 4-methylmorpholine (8.08 mL, 73.5 mmol, 2.0 equiv.) in CH₂Cl₂ (250 mL) at 0°C. The reaction mixture was stirred at 0°C for 20 minutes, and then *N,O*-dimethylhydroxylamine hydrochloride (3.60 g, 36.7 mmol, 1.0 equiv.) was added. The resulting solution was stirred at 0°C for 20 minutes and at 23°C for 2 hours, and then was partitioned between water (150 mL) and CH₂Cl₂ (2 x 150 mL). The combined organic layers were dried over Na₂SO₄ and were concentrated. Purification of the residue by flash column chromatography (gradient elution 40→20% hexanes in EtOAc) provided {Boc-L-(Tr-Gln)}-N(OMe)Me (16.1 g, 82%) as a white foam: IR (cm⁻¹) 3411, 3329, 3062, 1701, 1659; ¹H NMR (CDCl₃) δ 1.42 (s, 9H), 1.63-1.77 (m, 1H), 2.06-2.17 (m, 1H), 2.29-2.43 (m, 2H), 3.17 (s, 3H), 3.64 (s, 3H), 4.73 (s, br, 1H), 5.38-5.41 (m, 1H), 7.20-7.31 (m, 15H); Anal. (C₃₁H₃₇N₃O₅) C, H, N.

### Preparation of Intermediate {Boc-L-(Tr-Gln)}-H

Diisobutylaluminum hydride (50.5 mL of a 1.5 M solution in toluene, 75.8 mmol, 2.5 equiv.) was added to a solution of {Boc-L-(Tr-Gln)}-N(OMe)Me (16.1 g, 30.3 mmol, 1 equiv.) in THF at -78°C, and the reaction mixture was stirred at -78°C for 4 h. Methanol (4 mL) and 1.0 M HCl (10 mL) were added sequentially, and the mixture was warmed to 23°C. The resulting suspension was diluted with Et₂O (150 mL) and was washed with 1.0 M HCl (3 x 100 mL), half-saturated NaHCO₃ (100 mL), and water (100 mL). The organic layer was dried over MgSO₄, filtered, and was concentrated to give crude {Boc-L-(Tr-Gln)}-H (13.8 g, 97%) as a white solid: mp = 114-116°C; IR (cm⁻¹) 3313, 1697, 1494; ¹H NMR (CDCl₃) δ 1.44 (s, 9H), 1.65-1.75 (m, 1H), 2.17-2.23 (m, 1H), 2.31-2.54 (m, 2H), 4.11 (s, br, 1H), 5.38-5.40 (m, 1H), 7.1 (s, 1H), 7.16-7.36 (m, 15H), 9.45 (s, 1H).

### Preparation of Intermediate Ethyl-3-{Boc-L-(Tr-Gln)}-E-Propenoate

Sodium bis(trimethylsilyl)amide (22.9 mL of a 1.0 M solution in THF, 22.9 mmol, 1.0 equiv.) was added to a solution of triethyl phosphonoacetate (5.59 g, 22.9 mmol, 1.0 equiv.) in THF (200 mL) at -78°C, and the resulting solution was stirred for 20 minutes at that temperature. Crude {Boc-L-(Tr-Gln)}-H (10.8 g, 22.9 mmol, 1 equiv.) in THF (50 mL) was added via cannula, and the reaction mixture was stirred for 2 hours at -78°C, warmed to 0°C for 10 minutes, and partitioned between 0.5 M HCl (150 mL) and a 1:1 mixture of EtOAc and hexanes (2 x 150 mL). The combined organic layers were dried over Na₂SO₄ and were concentrated. Purification of the residue by flash column chromatography (40% EtOAc in hexanes) provided ethyl-3-{Boc-L-(Tr-*Gln*)}-E-propenoate (10.9 g, 88%) as a white foam: IR (cm⁻¹) 3321, 1710; ¹H NMR (CDCl₃) δ 1.27 (t, 3H, *J* = 7.2), 1.42 (s, 9H), 1.70-1.78 (m, 1H), 1.80-1.96 (m, 1H), 2.35 (t, 2H, *J* = 7.0), 4.18 (q, 2H, *J* = 7.2), 4.29 (s, br, 1H), 4.82-4.84 (m, 1H), 5.88 (dd, 1H, *J* = 15.7, 1.6), 6.79 (dd, 1H, *J* = 15.7, 5.3), 6.92 (s, 1H), 7.19-7.34 (m, 15H); Anal. (C₃₃H₃₈N₂O₅) C, H, N.

### Preparation of Intermediate Ethyl-3-{Cbz-L-Leu-L-Phe-L-(Tr-Gln)}-E-Propenoate

A solution of HCl in 1,4-dioxane (4.0 M, 20 mL), was added to a solution of ethyl-3-{Boc-L-(Tr-*Gln*)}-E-propenoate (1.00 g, 1.84 mmol, 1 equiv.) in the same solvent (20 mL) at 23°C. After 3 hours, the volatiles were removed under reduced pressure. The residue was dissolved in CH₂Cl₂ (50 mL) and Cbz-L-Leu-L-Phe-OH (0.759 g, 1.84 mmol, 1.0 equiv.), HOBt (0.373 g, 2.76 mmol, 1.5 equiv.), 4-methylmorpholine (0.809 mL, 7.36 mmol, 4.0 equiv.), and EDC (0.529 g, 2.76 mmol, 1.5 equiv.) were added sequentially. The reaction mixture was stirred at 23°C for 18 hours, and then was partitioned between water (150 mL) and EtOAc (2 x 150 mL). The combined organic layers were dried over Na₂SO₄ and were concentrated. Flash chromatographic purification of the residue (5% CH₃OH in CH₂Cl₂) afforded ethyl-3-{Cbz-L-Leu-L-Phe-L-(Tr-*Gln*)}-E-propenoate (1.25 g, 83%) as a white solid: mp = 192-194°C; IR (cm⁻¹) 3295, 1696, 1678, 1655, 1519; ¹H NMR (CDCl₃) δ 0.84 (d, 3H, *J* = 6.5), 0.86 (d, 3H, *J* = 6.5), 1.24-1.32 (m, 1H), 1.28 (t, 3H, *J* = 7.2), 1.43-1.75 (m, 3H), 1.91-2.06 (m, 1H), 2.20-2.38 (m, 2H), 2.93-3.02 (m, 1H), 3.07-3.18 (m, 1H), 3.95-4.02 (m, 1H), 4.17 (q, 2H, *J* = 7.2), 4.43-4.55 (m, 2H), 4.82-4.95 (m, 2H), 5.69 (d, 1H, *J* = 15.7), 6.46 (d, 1H, *J* = 7.5), 6.60 (d, 1H, *J* = 8.1), 6.69 (dd, 1H, *J* = 15.7, 5.1), 7.09-7.38 (m, 27H); Anal. (C₅₁H₅₆N₄O₇) C, H, N.

### Preparation of Product Ethyl-3-(Cbz-L-Leu-L-Phe-L-Gln)-E-Propenoate

Trifluoroacetic acid (20 mL) was added to a solution of ethyl-3-{Cbz-L-Leu-L-Phe-L-(Tr-*Gln*)}-E-propenoate (1.25 g, 1.49 mmol, 1 equiv.) and triisopropylsilane (1.53 mL, 7.47 mmol, 5.0 equiv.) in CH₂Cl₂ (20 mL) at 23°C, producing a bright yellow solution. The reaction mixture was stirred for 20 minutes at 23°C, during which time it became colorless. Carbon tetrachloride (20 mL) was added, and the volatiles were removed under reduced pressure. The residue was triturated with Et₂O (20 mL), and the resulting white solid was collected by vacuum filtration, washed with Et₂O (3 x 50 mL), and air-dried to afford ethyl-3-(Cbz-L-Leu-L-Phe-L-*Gln*)-E-propenoate (0.717 g, 81%): mp = 219-221°C; IR (cm⁻¹) 3300, 1672, 1535; ¹H NMR (DMSO-*d*₆) δ 0.78 (d, 3H, *J* = 6.8), 0.82 (d, 3H, *J* = 6.5), 1.21 (t, 3H, *J* = 7.0), 1.25-1.37 (m, 2H), 1.42-1.54 (m, 1H), 1.58-1.80 (m, 2H), 2.02-2.09 (m, 2H), 2.84 (dd, 1H, *J* = 13.2, 8.9), 2.97 (dd, 1H, *J* = 13.2, 5.8), 3.93-4.01 (m, 1H), 4.11 (q, 2H, *J* = 7.0), 4.33-4.52 (m, 2H), 4.97 (d, 1H, *J* = 12.3), 5.04 (d, 1H, *J* = 12.3), 5.64 (d, 1H, *J* = 15.9), 6.69 (dd, 1H, *J* = 15.9, 5.4), 6.76 (s, 1H), 7.13-7.37 (m, 11H), 7.43 (d, 1H, *J* = 7.8), 7.99 (d, 1H, *J* = 8.1), 8.04 (d, 1H, *J* = 8.1); Anal. (C₃₂H₄₂N₄O₇) C, H, N.

### Example 32 - Preparation of Comparison Compound #3: Ethyl-3-(Cbz-L-Val-L-Phe-L-Gln)-E-Propenoate

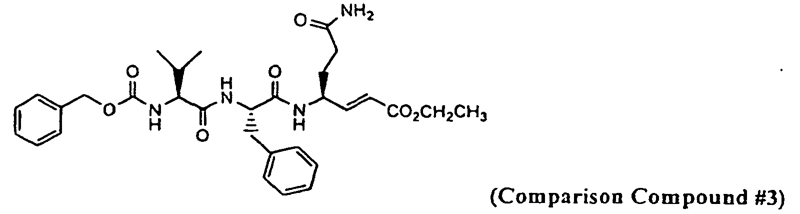

### Preparation of Intermediate Ethyl-3-{Boc-L-Phe-L-(Tr-Gln)}-E-Propenoate

A solution of HCl in 1,4-dioxane (4.0 M, 10 mL) was added to a solution of ethyl-3-{Boc-L-(Tr-*Gln*)}-E-propenoate (prepared as described in Example 31, 3.05 g, 5.62 mmol, I equiv.) in the same solvent (20 mL) at 23°C. After 3 hours, the volatiles were removed under reduced pressure. The residue was dissolved in CH₂Cl₂ (50 mL), and Boc-L-Phe-OH (1.49 g, 5.62 mmol, 1.0 equiv.), HOBt (0.911 g, 6.74 mmol, 1.2 equiv.), 4-methylmorpholine (1.85 mL, 16.8 mmol, 3.0 equiv.) and EDC (1.29 g, 6.73 mmol, 1.2 equiv.) were added sequentially. The reaction mixture was stirred at 23°C for 18 hours, and was then partitioned between water (150 mL) and EtOAc (2 x 150 mL). The combined organic layers were dried over Na₂SO₄ and were concentrated. Flash chromatographic purification of the residue (gradient elution, 40→50% EtOAc in hexanes) afforded ethyl-3-{Boc-L-Phe-L-(Tr-*Gln*)}-E-propenoate (2.77 g, 71%) as a white foam: IR (cm⁻¹) 3306, 1706, 1661; ¹H NMR (CDCl₃) δ 1.29 (t, 3H, *J* = 7.2), 1.38 (s, 9H), 1.65-1.76 (m, 1H); 1.87-1.99 (m, 1H), 2.25-2.27 (m, 2H), 2.94-3.01 (m, 2H), 4.14-4.26 (m, 3H), 4.48-4.53 (m, 1H), 4.95 (s, br, 1H), 5.64 (d, 1H, *J* = 15.8), 6.29 (d, 1H, *J* = 8.1), 6.64 (dd, 1H, *J* = 15.8, 5.4), 6.80 (s, br, 1H), 7.14-7.32 (m, 20H); Anal. (C₄₂H₄₇N₃O₆) C, H, N.

### Preparation of Intermediate Ethyl-3-{Cbz-L-Val-L-Phe-L-(Tr-Gln)}-E-Propenoate

A solution of HCl in 1,4-dioxane (4.0 M, 8 mL) was added to a solution of ethyl-3-{Boc-L-Phe-L-(Tr-*Gln*)}-E-propenoate (0.296 g, 0.429 mmol, 1 equiv.) in the same solvent (10 mL) at 23°C. After 3 hours, the volatiles were removed under reduced pressure. The residue was dissolved in CH₂Cl₂ (10 mL), and then Cbz-L-Val-OH (0.108 g, 0.430 mmol, 1.0 equiv.), HOBt (0.070 g, 0.518 mmol, 1.2 equiv.), 4-methylmorpholine (0.142 mL, 1.29 mmol, 3.0 equiv.) and EDC (0.099 g, 0.516 mmol, 1.2 equiv.) were added sequentially. The reaction mixture was stirred at 23°C for 4 hours, and then was partitioned between water (100 mL) and EtOAc (2 x 100 mL). The combined organic layers were dried over Na₂SO₄ and were concentrated. Flash chromatographic purification of the residue (3% CH₃OH in CH₂Cl₂) afforded ethyl-3-{Cbz-L-Val-L-Phe-L-(Tr-*Gln*)}-E-propenoate (0.220 g, 62%) as a white solid: mp = 195-198°C; IR (cm⁻¹) 3284, 1689, 1646; ¹H NMR (CDCl₃) δ 0.69 (d, 3H, *J* = 6.9), 0.82 (d, 3H, *J* = 6.5), 1.28 (t, 3H, *J* = 7.2), 1.63-1.74 (m, 1H), 1.96-2.02 (m, 2H), 2.22-2.35 (m, 2H), 2.93 (dd, 1H, *J* = 14.0, 7.6), 3.10 (dd, 1H, *J* = 14.0, 6.7), 3.81-3.85 (m, 1H), 4.17 (q, 2H, *J* = 7.2), 4.48-4.58 (m, 2H), 4.87 (d, 1H, *J* = 12.0), 4.94 (d; 1H, *J* = 12.0), 5.06 (d, 1H, *J* = 6.9), 5.67 (d, 1H, *J* = 15.6), 6.43 (d, 1H, *J* = 7.5), 6.63-6.72 (m, 2H), 7.10-7.40 (m, 26H); Anal. (C₅₀H₅₄N₄O₇) C, H, N.

### Preparation of Product Ethyl-3-(Cbz-L-Val-L-Phe-L-Gln)-E-Propenoate

Trifluoroacetic acid (5 mL) was added to a solution of ethyl-3-{Cbz-L-Val-L-Phe-L-(Tr-*Gln*)}-E-propenoate (0.188 g, 0.229 mmol, 1 equiv.) and triisopropylsilane (0.300 mL, 1.46 mmol, 6.4 equiv.) in CH₂Cl₂ (10 mL) at 23°C, producing a bright yellow solution. The reaction mixture was stirred for 20 min. at 23°C, during which time it became colorless. Carbon tetrachloride (10 mL) was added, and the volatiles were removed under reduced pressure. The residue was triturated with Et₂O (20 mL), and the resulting white solid was collected by vacuum filtration, washed with Et₂O (3 x 50 mL), and air-dried to afford ethyl-3-(Cbz-L-Val-L-Phe-L-*Gln*)-E-propenoate (0.094 g, 71%): mp = 240°C (dec); IR (cm⁻¹) 3263, 1686, 1640; ¹H NMR (DMSO-*d*₆) δ 0.73 (d, 6H, *J* = 6.9), 1.21 (t, 3H, *J* = 7.2), 1.60-1.75 (m, 2H), 1.83-1.90 (m, 1H), 2.03-2.08 (m, 2H), 2.83 (dd, 1H, *J* = 13.6, 8.6), 2.96 (dd, 1H, *J* = 13.6, 6.2), 3.79-3.84 (m, 1H), 4.10 (q, 2H, *J* = 7.2), 4.37-4.49 (m, 1H), 4.51-4.56 (m, 1H), 4.99 (d, 1H, *J* = 12.5), 5.06 (d, 1H, *J* = 12.5), 5.61 (d, 1H, *J* = 15.5), 6.67 (dd, 1H, *J* = 15.5, 5.5), 6.76 (s, 1H), 7.13-7.36 (m, 12H), 8.06 (d, 2H, *J* = 8.1); Anal. (C₃₁H₄₀N₄O₇) C, H, N.

Results of biochemical and biological tests conducted using various compounds of the invention are described below.

### BIOCHEMICAL AND BIOLOGICAL EVALUATION

### Inhibition of Rhinovirus 3C Protease

Stock solutions (50 mM, in DMSO) of various compounds were prepared; dilutions were in the same solvent. Recombinant rhinovirus 3C proteases (see Birch et al., "Purification of recombinant human rhinovirus 14 3C protease expressed in Escherichia coli," *Protein Expr. Pur*. **1995,** 6(5), 609-618) from serotypes 14, 16, and 2 were prepared by the following standard chromatographic procedures: (1) ion exchange using Q Sepharose Fast Flow from Pharmacia; (2) affinity chromatography using Affi-Gel Blue from Biorad; and (3) sizing using Sephadex G-100 from Pharmacia. Each assay sample contained 2% DMSO, 50 mM tris pH 7.6, 1 mM EDTA, a test compound at the indicated concentration, approximately 1µM substrate, and 50-100 nM protease. The k_{obs/I} values were obtained from reactions initiated by addition of enzyme rather than substrate. RVP. activity was measured in the fluorescence resonance energy transfer assay. The substrate was (N-terminal) DABCYL-(Gly-Arg-Ala-Val-Phe-Gln-Gly-Pro-Val-Gly)-EDANS. In the uncleaved peptide, the EDANS fluorescence was quenched by the proximal DABCYL moiety. When the peptide was cleaved, the quenching was relieved, and activity was measured as an increase in fluorescence signal. Data were analyzed using standard non-linear fitting programs (Enzfit), and are shown in Tables 1 and 2 below. In Table 1, all data are for rhinovirus 3C protease from HRV serotype-14 (produced from the infectious cDNA clone constructed by Dr. Robert Rueckert, Institute for Molecular Virology, University of Wisconsin, Madison, Wisconsin). Table 2 shows protease-inhibiting activity of compounds against proteases from several rhinovirus of serotype other than RVP serotype-14. The data in the column designated k_{obs}/[I] were measured from progress curves in enzyme start experiments.

### Antirbinoviral H1-HeLa Cell Culture Assay

In this cell protection assay, the ability of compounds to protect cells against HRV infection was measured by the XTT dye reduction method, which is described in Weislow et al., *J. Natl. Cancer Inst*. **1989,** vol. 81, 577-586.

H1-HeLa cells were infected with HRV-14 at a multiplicity of infection (m.o.i.) of 0.13 (virus particles/cell) or mock-infected with medium only. Infected or mock-infected cells were resuspended at 8 x 10⁵ cells per mL, and incubated with appropriate concentrations of the compounds to be tested. Two days later, XTT/PMS was added to test plates and the amount of formazan produced was quantified spectrophotometrically at 450/650 nm. The EC₅₀ value was calculated as the concentration of compound that increased the percentage of formazan production in compound-treated, virus-infected cells to 50% of that produced by compound-free, mock-infected cells. The 50% cytotoxic dose (CC₅₀) was calculated as the concentration of compound that decreased the percentage of formazan produced in compound-treated, mock-infected cells to 50% of that produced by compound-free, mock-infected cells. The therapeutic index (TI) was calculated by dividing the CC₅₀ value by the EC₅₀ value.

All strains of human rhinovirus (HRV) for use in this assay were purchased from American Type Culture Collection (ATCC), except for HRV serotype-14 (produced from the infectious cDNA clone constructed by Dr. Robert Rueckert, Institute for Molecular Virology, University of Wisconsin, Madison, Wisconsin). HRV stocks were propagated and viral assays were performed in H1-HeLa cells (ATCC). Cells were grown in minimal essential medium with 10% fetal bovine serum, available from Life Technologies (Gaithersburg, MD).

The compounds were tested against control compounds WIN 51711, WIN 52084, and WIN 54954 (obtained from Sterling-Winthrop Pharmaceuticals), Pirodavir (obtained from Janssen Pharmaceuticals), and Pleconaril (prepared according to the method described in Diana et al., *J. Med. Chem* **1995**, vol. 38, 1355). Antiviral data obtained for the test compounds are shown in Tables I and 3, where all data are for HRV serotype-14 unless otherwise noted in parentheses. The designation "ND" indicates that a value was not determined for that compound.

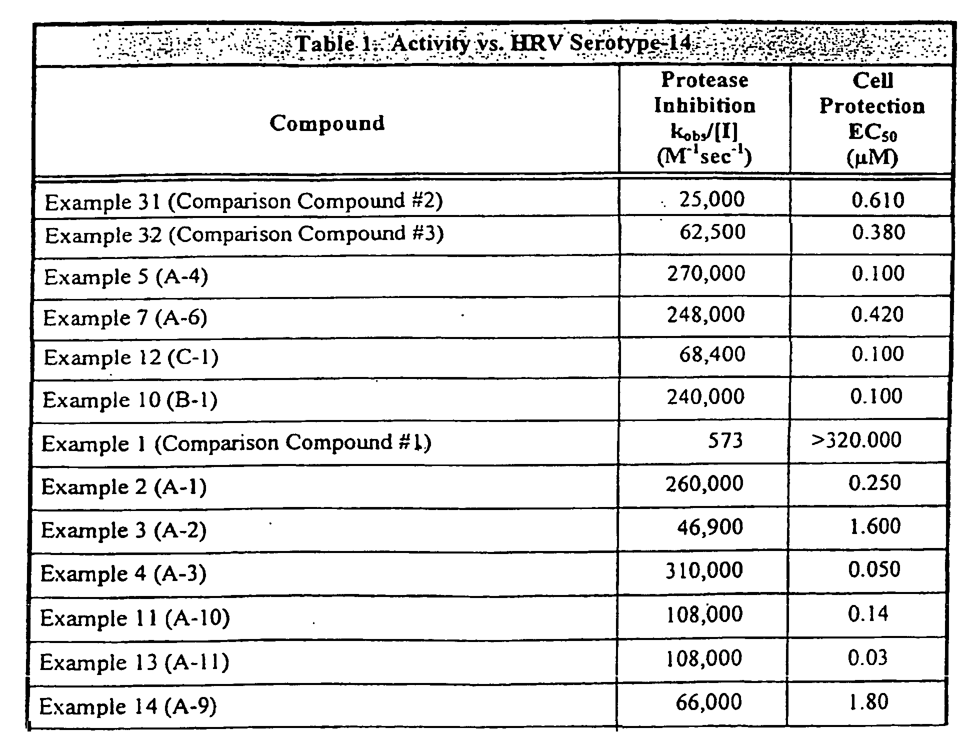

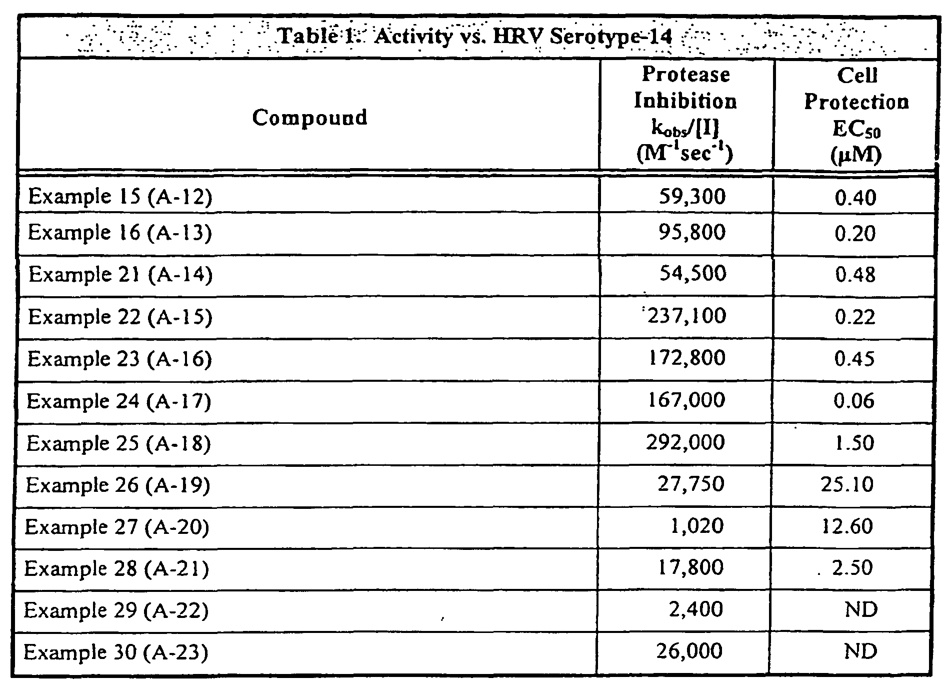

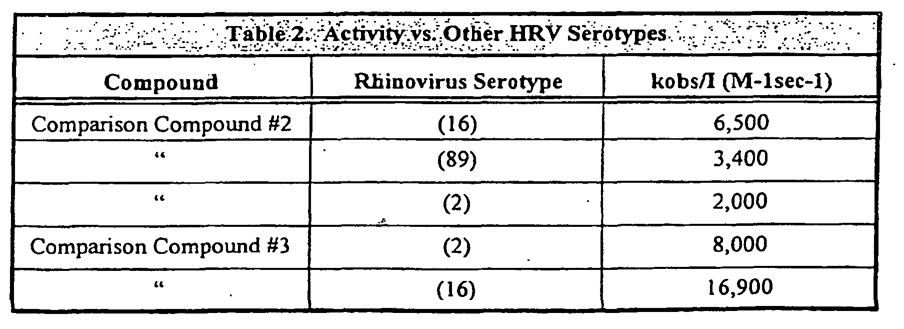

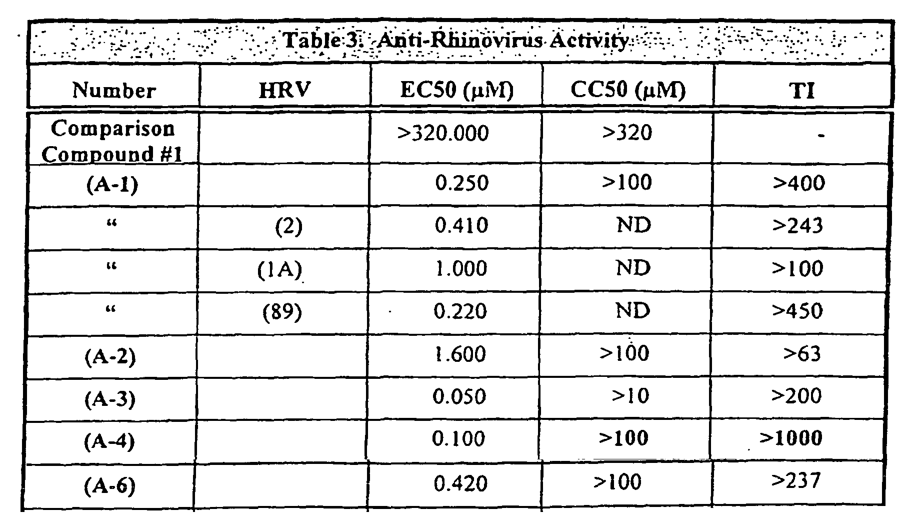

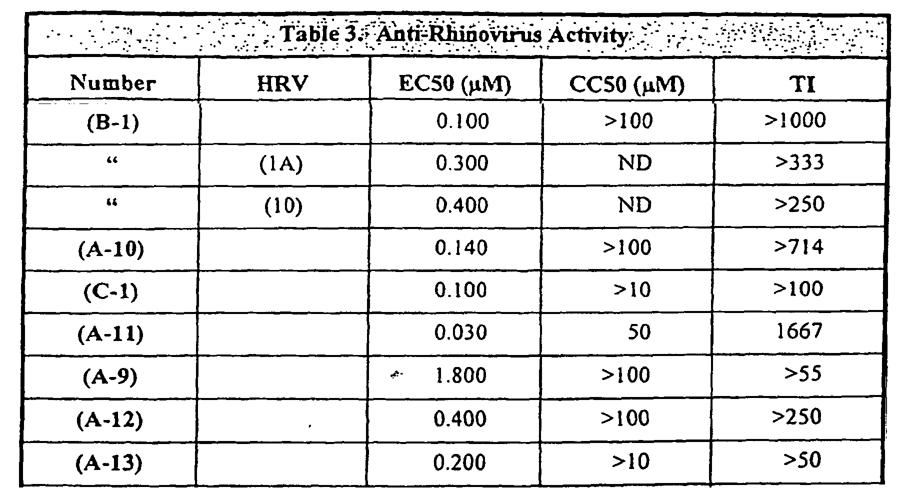

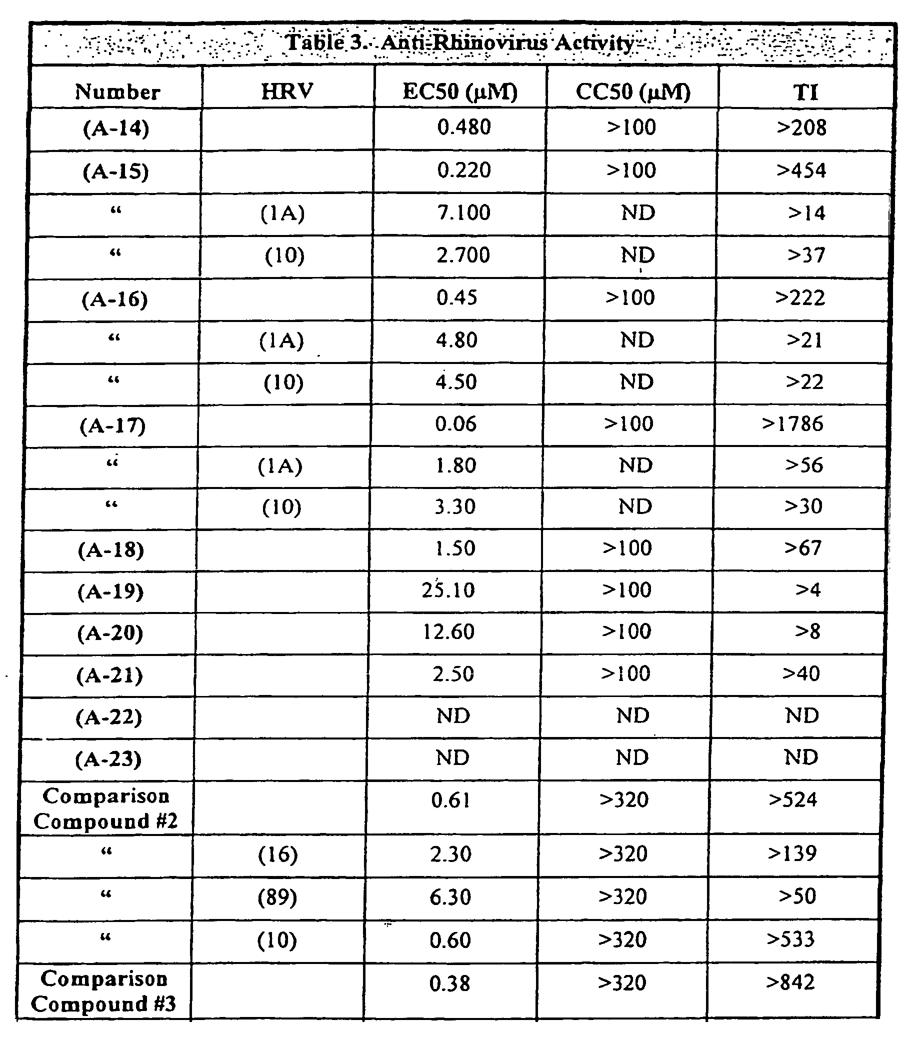

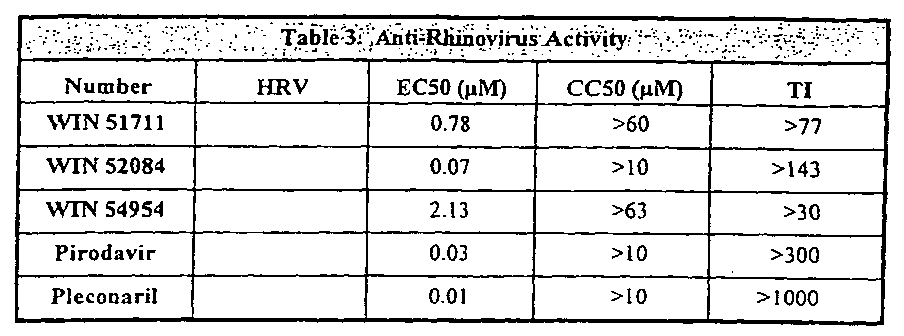

## Claims

1. A compound of the formula I: 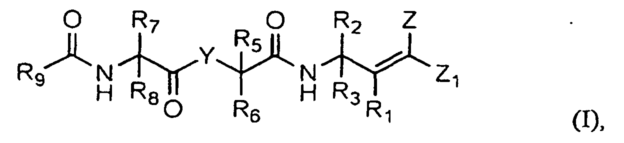 wherein:
Y is -N(R_{y})-, -C(R_{y})(R_{y})-, or -O-, where each R_{y} is independently H or C₁-C₄ alkyl;
R₁ is H, F, an alkyl group, OH, SH, or an O-alkyl group;
R₂ and R₃ are each independently H; 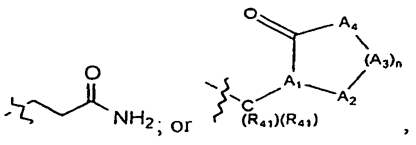 where n is an integer from 0 to 5, A₁ is CH or N, A₂ and each A₃ are independently selected from C(R₄₁)(R₄₁), N(R₄₁), S, S(O), S(O)₂, and O, and A₄ is NH or NR₄₁, where each R₄₁ is independently H or C₁-C₄ alkyl, provided that no more than 2 heteroatoms occur consecutively in the ring formed by A₁, A₂, (A₃)ₙ, A₄ and C=O; and provided that at least one of R₂ and R₃ is 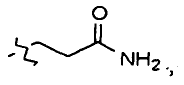
R₅ and R₆ are each independently H, F, an alkyl group, a cycloalkyl group, a heterocycloalkyl group, an aryl group, or a heteroaryl group;
R₇ and R₈ are each independently H, an alkyl group, a cycloalkyl group, a heterocycloalkyl group, an aryl group, a heteroaryl group, -OR₁₇, -SR₁₇, -NR₁₇R₁₈, -NR₁₉NR₁₇R₁₈, or -NR₁₇OR₁₈, where R₁₇, R₁₈, and R₁₉ are each independently H, an alkyl group, a cycloalkyl group, a heterocycloalkyl group, an aryl group, a heteroaryl group, or an acyl group;
R₉ is a five-membered heterocycle having from one to three heteroatoms selected from O, N, and S; and
Z and Z₁ are each independently H, F, an alkyl group, a cycloalkyl group, a heterocycloalkyl group, an aryl group, a heteroaryl group, -C(O)R₂₁, -CO₂R₂₁, -CN, -C(O)NR₂₁,R₂₂, -C(O)NR₂₁OR₂₂, -C(S)R₂₁, -C(S)NR₂₁R₂₂, -NO₂, -SOR₂₁, -SO₂R₂₁, -SO₂NR₂₁R₂₂, -SO(NR₂₁)(OR₂₂), -SONR₂₁, -SO₃R₂₁, -PO(OR₂₁)₂, -PO(R₂₁)(R₂₂), -PO(NR₂₁R₂₂)(OR₂₃), PO(NR₂₁R₂₂)(NR₂₃R₂₄), -C(O)NR₂₁NR₂₂R₂₃, or -C(S)NR₂₁NR₂₂R₂₃, where R₂₁, R₂₂, R₂₃, and R₂₄ are each independently H, an alkyl group, a cycloalkyl group, a heterocycloalkyl group, an aryl group, a heteroaryl group, an acyl group, or a thioacyl group, or any two of R₂₁, R₂₂, R₂₃, and R₂₄, together with the atom(s) to which they are bonded, form a heterocycloalkyl group, provided that Z and Z₁ are not both H;
or Z₁ and R₁, together with the atoms to which they are bonded, form a cycloalkyl or heterocycloalkyl group;
or Z and Z₁, together with the atoms to which they are bonded, form a cycloalkyl or heterocycloalkyl group;
or a pharmaceutically acceptable salt, or solvate thereof, under the proviso that the following compounds are excluded: 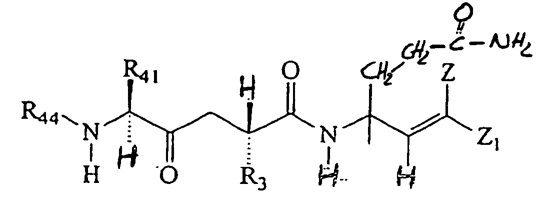 wherein R₃, Z, Z₁, R₄₁ and R₄₄ are as follows:
R₃ is CH₂(p-F)Ph, Z is H, Z₁ is CO₂CH₂CH₃, R₄₁ is CH(CH₃)₂, and R₄₄ is 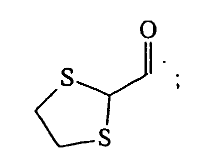
R₃ is CH₂(*p*-F)Ph, Z is H, Z₁ is 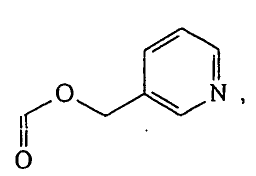 R₄₁ is CH(CH₃)₂, and R₄₄ is 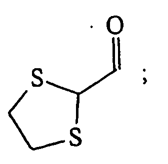
R₃ is CH₂(*p*-F)Ph, Z is H, Z₁ is CO₂CH₂CH₃, R₄₁ is CH(CH₃)₂, and R₄₄ is 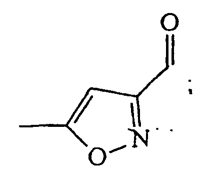 and 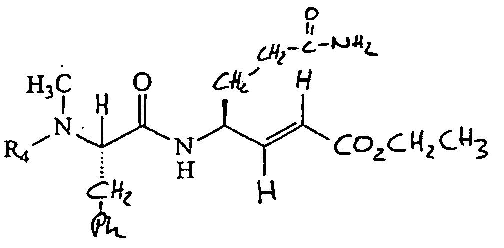
wherein R₄ is:

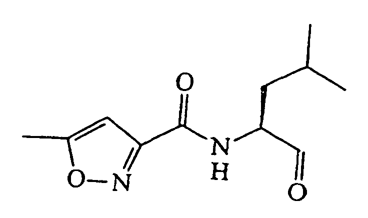

2. A compound, pharmaceutically acceptable salt, or solvate according to claim 1, wherein R₂ and R₃ are each independently H; 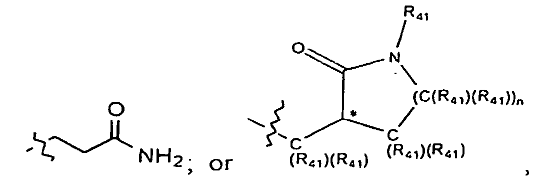 where n is an integer from 0 to 5, each R₄₁ is independently H or lower alkyl, and the stereochemistry at the carbon denoted with an asterisk (*) may be R or S; provided that at least one of R₂ and R₃ is

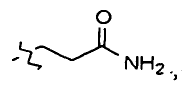

3. A compound, pharmaceutically acceptable salt, or solvate according to claim 1, wherein Y is -N(R_{y})-, where R_{y} is H or C₁-C₄ alkyl.

4. A compound, pharmaceutically acceptable salt, or solvate according to claim 3, wherein: Z and Z₁ are each independently selected from H, F, C₁-C₄ alkyl, -CO₂R₂₁, and -C(O)NR₂₁R₂₂, where R₂₁ and R₂₂ are each independently H, an alkyl group, a cycloalkyl group, a heterocycloalkyl group, an aryl group, a heteroaryl group, an acyl group, or a thioacyl group, or R₂₁ and R₂₂, together with the atom(s) to which they are bonded, form a heterocycloalkyl group, provided that Z and Z₁ are not both H;
or Z₁ and R₁, together with the atoms to which they are bonded, form a cycloalkyl or heterocycloalkyl group;
or Z and Z₁, together with the atoms to which they are bonded, form a cycloalkyl or heterocycloalkyl group.

5. A compound, pharmaceutically acceptable salt, or solvate according to claim 3, wherein R₁ is H, F, or methyl.

6. A compound, pharmaceutically acceptable salt, or solvate according to claim 3, wherein at least one of R₂ or R₃ is

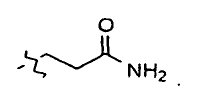

7. A compound, pharmaceutically acceptable salt, or solvate according to claim 6, wherein one of R₅ and R₆ is H and the other is alkyl or aryl.

8. A compound, pharmaceutically acceptable salt, or solvate according to claim 3, wherein one of R₅ and R₆ is H and the other is alkyl or aryl.

9. A compound, pharmaceutically acceptable salt, or solvate according to claim 3, wherein one of R₅ and R₆ is H and the other is unsubstituted or substituted phenylmethyl.

10. A compound, pharmaceutically acceptable salt, or solvate according to claim 3, wherein R₇ and R₈ are each independently H, an alkyl group, a cycloalkyl group, a heterocycloalkyl group, an aryl group, or a heteroaryl group.

11. A compound, pharmaceutically acceptable salt, or solvate according to claim 3, wherein one of R₇ and R₈ is H and the other is alkyl or aryl.

12. A compound, pharmaceutically acceptable salt, or solvate according to claim 3, wherein one of R₇ and R₈ is H and the other is 2-propyl, 2-methyl-2-propyl, 2-methyl-1-propyl, phenylmethyl, or naphthylmethyl.

13. A compound, pharmaceutically acceptable salt, or solvate according to claim 3, wherein R₉ is a five-membered heterocycle having at least one nitrogen heteroatom and one oxygen heteroatom.

14. A compound, pharmaceutically acceptable salt, or solvate according to claim 3, wherein R₉ is selected from substituted and unsubstituted 1,2-oxazolyl, 1,3-oxazolyl, and 1,2,4-oxadiazolyl..

15. A compound, pharmaceutically acceptable salt, or solvate according to claim 3, wherein R₉ is 3-isoxazolyl or 5-isoxazolyl unsubstituted or substituted with one or two substituents selected from methyl and halogens.

16. A compound according to claim 3 of the formula I-A": 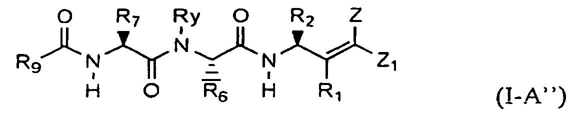 wherein R₁, R₂, R₆, R₇, R₉, R_{y}, Z, and Z₁ are as defined in claim 3,
or a pharmaceutically acceptable salt, or solvate thereof.

17. A compound, pharmaceutically acceptable salt, or solvate according to claim 16, wherein R₂ is

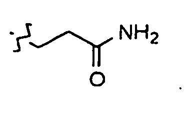

18. A compound, pharmaceutically acceptable salt, or solvate according to claim 16, wherein:
R_{y} is H or methyl;
R₁ is H, F, or methyl;
Z and Z₁ are each independently selected from H, F, -CO₂R₂₁, -CN, and -C(O)NR₂₁,R₂₂, where R₂₁ and R₂₂ are each independently H, an alkyl group, a cycloalkyl group, a heterocycloalkyl group, an aryl group, a heteroaryl group, an acyl group, or a thioacyl group, or R₂₁ and R₂₂, together with the atom(s) to which they are bonded, form a heterocycloalkyl group, provided that Z and Z₁ are not both H;
or Z₁ and R₁, together with the atoms to which they are bonded, form a cycloalkyl or heterocycloalkyl group;
or Z₁ and R₁, together with the atoms to which they are bonded, form a cycloalkyl or heterocycloalkyl group;
or Z and Z₁, together with the atoms to which they are bonded, form a cycloalkyl or heterocycloalkyl group;
R₂ is 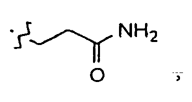
R₆ is unsubstituted or substituted phenylmethyl;
R₇ is alkyl or aryl; and
R₉ is 3-isoxazolyl or 5-isoxazolyl unsubstituted or substituted with one or two substituents selected from methyl and halogens.

19. A compound, pharmaceutically acceptable salt, or solvate according to claim 18, wherein R₇ is selected from 2-propyl, 2-methyl-2-propyl, 2-methyl-1-propyl, phenylmethyl, and naphthylmethyl.

20. A compound, pharmaceutically acceptable salt, or solvate according to claim 16, wherein R_{y}, R₁, and Z are each H, and:
R₂ is CH₂CH₂C(O)NH₂, R₆ is CH₂Ph, R₇ is CH₂CH(CH₃)₂, Z₁ is CO₂CH₂CH₃, and R₉ is 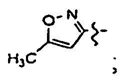
R₂ is CH₂CH₂C(O)NH₂, R₆ is CH₂Ph, R₇ is CH₂CH(CH₃)₂, Z₁ is CO₂CH₂CH₃, and R₉ is 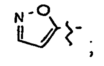
R₂ is CH₂CH₂C(O)NH₂, R₆ is 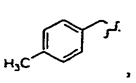 R₇ is C(CH₃)₃, Z₁ is CO₂CH₂CH₃, and R₉ is 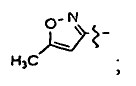
R₂ is CH₂CH₂C(O)NH₂, R₆ is 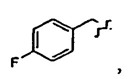 R₇ is C(CH₃)₃, Z₁ is CO₂CH₂CH₃ and R₉ is 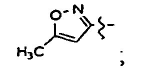
R₂ is CH₂CH₂C(O)NH₂, R₆ is 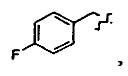 R₇ is CH(CH₃)₂, Z₁ is CO₂CH₂CH₃, and R₉ is

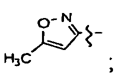

21. A compound, pharmaceutically acceptable salt, or solvate according to claim 16, wherein R_{y} is CH₃, R₁ and Z are each H, and:
R₂ is CH₂CH₂C(O)NH₂, R₆ is 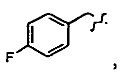 R₇ is 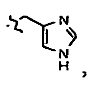 Z₁ is CO₂CH₂CH₃, and R₉ is 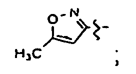
R₂ is CH₂CH₂C(O)NH₂, R₆ is CH₂Ph, R₇ is CH₂CH(CH₃)₂, and R₉ is 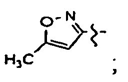
R₂ is CH₂CH₂C(O)NH₂, R₆ is 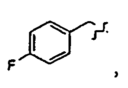 R₇ is 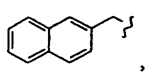 and R₉ is 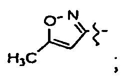
R₂ is CH₂CH₂C(O)NH₂, R₆ is 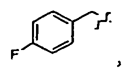 R₇ is CH₂CH(CH₃)₂, and R₉ is 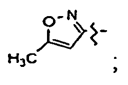 or
R₂ is CH₂CH₂C(O)NH₂, R₆ is 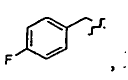 R₇ is 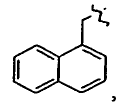 and R₉ is

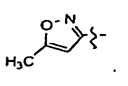

22. A compound, pharmaceutically acceptable salt, or solvate according to claim 16 selected from the group consisting of:

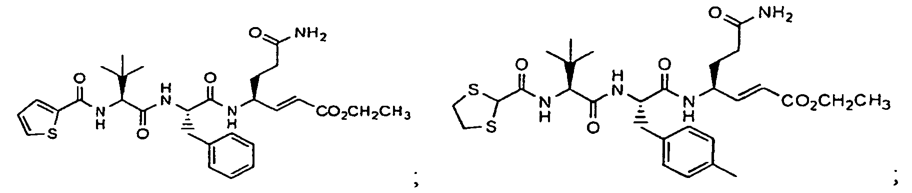

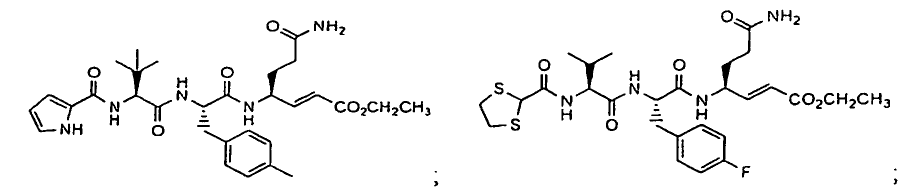

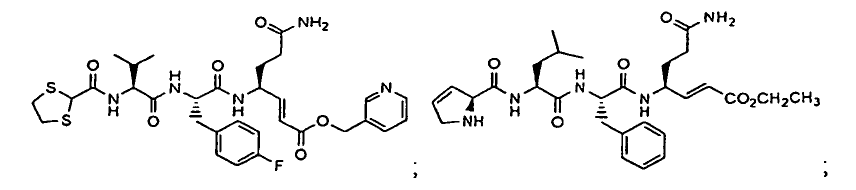

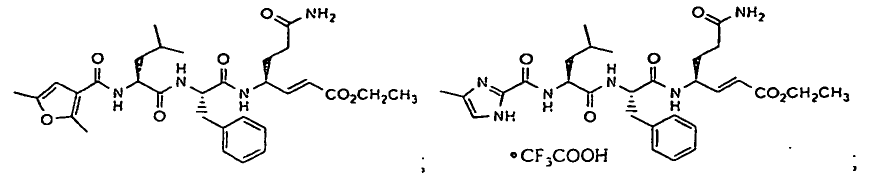

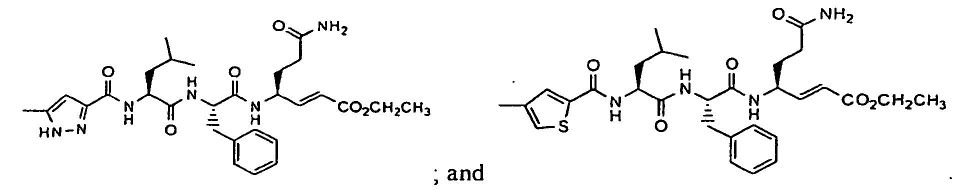

23. A compound, pharmaceutically acceptable salt, or solvate according to claim 1, wherein Y is -CH₂-.

24. A compound, pharmaceutically acceptable salt, or solvate according to claim 23, wherein Z and Z₁ are each independently selected from H, F, C₁-C₄ alkyl, -CO₂R₂₁, and -C(O)NR₂₁,R₂₂, where R₂₁ and R₂₂ are each independently H, an alkyl group, a cycloalkyl group, a heterocycloalkyl group, an aryl group, a heteroaryl group, an acyl group, or a thioacyl group, or R₂₁ and R₂₂, together with the atom(s) to which they are bonded, form a heterocycloalkyl group, provided that Z and Z₁ are not both H;
or Z₁ and R₁, together with the atoms to which they are bonded, form a cycloalkyl or heterocycloalkyl group;
or Z and Z₁, together with the atoms to which they are bonded, form a cycloalkyl or heterocycloalkyl group.

25. A compound, pharmaceutically acceptable salt, or solvate according to claim 23, wherein R₁ is H, F, or methyl.

26. A compound, pharmaceutically acceptable salt, or solvate according to claim 23, wherein at least one of R₂ and R₃ is

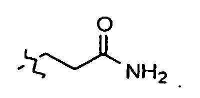

27. A compound, pharmaceutically acceptable salt, or solvate according to claim 26, wherein one of R₃ and R₆ is H and the other is alkyl or aryl.

28. A compound, pharmaceutically acceptable salt, or solvate according to claim 23, wherein one of R₅ and R₆ is H and the other is alkyl or aryl.

29. A compound, pharmaceutically acceptable salt, or solvate according to claim 23, wherein one of R₅ and R₆ is H and the other is unsubstituted or substituted phenylmethyl.

30. A compound, pharmaceutically acceptable salt, or solvate according to claim 23, wherein R₇ and R₈ are each independently H, an alkyl group, a cycloalkyl group, a heterocycloalkyl group, an aryl group, or a heteroaryl group.

31. A compound, pharmaceutically acceptable salt, or solvate according to claim 23, wherein one of R₇ and R₈ is H and the other is alkyl or aryl.

32. A compound, pharmaceutically acceptable salt, or solvate according to claim 23, wherein one of R₇ and R₈ is H and the other is 2-propyl, 2-methyl-2-propyl, 2-methyl-1-propyl, phenylmethyl, or naphthylmethyl.

33. A compound, pharmaceutically acceptable salt, or solvate according to claim 23, wherein R₉ is a five-membered heterocycle having at least one nitrogen heteroatom and one oxygen heteroatom.

34. A compound, pharmaceutically acceptable salt, or solvate according to claim 33, wherein R₉ is unsubstituted or substituted 1,2-oxazolyl, 1,3-oxazolyl, or 1,2,4-oxadiazolyl.

35. A compound, pharmaceutically acceptable salt, or solvate according to claim 34, wherein R₉ is 3-isoxazolyl or 5-isoxazolyl unsubstituted or substituted with one or two substituents selected from methyl and halogens.

36. A compound according to claim 23 of the formula I-B": 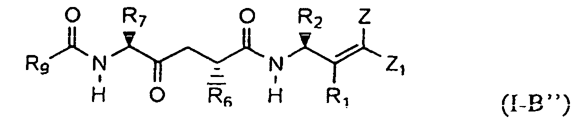 wherein R₁, R₂, R₆, R₇, R₉, Z, and Z₁ are as defined in claim 23,
or a pharmaceutically acceptable salt, or solvate thereof.

37. A compound, pharmaceutically acceptable salt, or solvate according to claim 36, wherein R₂ is

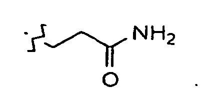

38. A compound, pharmaceutically acceptable salt, or solvate according to claim 36, wherein:
R₁ is H, F, or methyl;
Z and Z₁ are each independently selected from H, F, -CO₂R₂₁, -CN, and -C(O)NR_{21,}R₂₂, where R₂₁ and R₂₂ are each independently H, an alkyl group, a cycloalkyl group, a heterocycloalkyl group, an aryl group, a heteroaryl group, an acyl group, or a thioacyl group, or R₂₁ and R₂₂, together with the atom(s) to which they are bonded, form a heterocycloalkyl group, provided that Z and Z₁ are not both H;
or Z₁ and R₁, together with the atoms to which they are bonded, form a cycloalkyl or heterocycloalkyl group;
or Z and Z₁, together with the atoms to which they are bonded, form a cycloalkyl or heterocycloalkyl group;
R₂ is 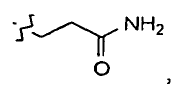
R₆ is unsubstituted or substituted phenylmethyl;
R₇ is alkyl or aryl; and
R₉ is 3-isoxazolyl or 5-isoxazolyl unsubstituted or substituted with one or two substituents selected from methyl and halogens.

39. A compound, pharmaceutically acceptable salt, or solvate according to claim 38, wherein R₇ is selected from 2-propyl, 2-methyl-2-propyl, 2-methyl-1-propyl, phenylmethyl, and naphthylmethyl.

40. A compound, pharmaceutically acceptable salt, or solvate according to claim 36, wherein R₁ is H and:
R₂ is CH₂CH₂C(O)NH₂, R₆ is 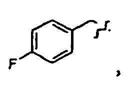 R₇ is CH(CH₃)₂, Z is H, Z₁ is CO₂CH₂CH₃, and R₉ is

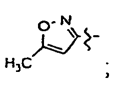

41. A compound, pharmaceutically acceptable salt, or solvate according to claim 1, wherein Y is -O-.

42. A compound, pharmaceutically acceptable salt, or solvate according to claim 41, wherein Z and Z₁ are each independently selected from H, F, C₁-C₄ alkyl, -CO₂R₂₁, and -C(O)NR₂₁,R₂₂, where R₂₁ and R₂₂ are each independently H, an alkyl group, a cycloalkyl group, a heterocycloalkyl group, an aryl group, a heteroaryl group, an acyl group, or a thioacyl group, or R₂₁ and R₂₂, together with the atom(s) to which they are bonded, form a heterocycloalkyl group, provided that Z and Z₁ are not both H;
or Z₁ and R₁, together with the atoms to which they are bonded, form a cycloalkyl or heterocycloalkyl group;
or Z and Z₁, together with the atoms to which they are bonded, form a cycloalkyl or heterocycloalkyl group.

43. A compound, pharmaceutically acceptable salt, or solvate according to claim 41, wherein R₁ is H, F, or methyl.

44. A compound, pharmaceutically acceptable salt, or solvate according to claim 41, wherein at least one of R₂ and R₃ is

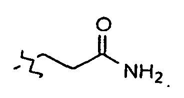

45. A compound, pharmaceutically acceptable salt, or solvate according to claim 44, wherein one of R₅ and R₆ is H and the other is alkyl or aryl.

46. A compound, pharmaceutically acceptable salt, or solvate according to claim 41, wherein one of R₅ and R₆ is H and the other is alkyl or aryl.

47. A compound, pharmaceutically acceptable salt, or solvate according to claim 41, wherein one of R₅ and R₆ is H and the other is unsubstituted or substituted phenylmethyl.

48. A compound, pharmaceutically acceptable salt, or solvate according to claim 41, wherein R₇ and R₈ are each independently H, an alkyl group, a cycloalkyl group, a heterocycloalkyl group, an aryl group, or a heteroaryl group.

49. A compound, pharmaceutically acceptable salt, or solvate according to claim 41, wherein one of R₇ and R₈ is H and the other is alkyl or aryl.

50. A compound, pharmaceutically acceptable salt, or solvate according to claim 42, wherein one of R₇ and R₈ is H and the other is 2-propyl, 2-methyl-2-propyl, 2-methyl-1-propyl, phenylmethyl, or naphthylmethyl.

51. A compound, pharmaceutically acceptable salt, or solvate according to claim 41, wherein R₉ is a five-membered heterocycle having at least one nitrogen heteroatom and one oxygen heteroatom.

52. A compound, pharmaceutically acceptable salt, or solvate according to claim 41, wherein R₉ is selected from substituted and unsubstituted 1,2-oxazolyl, 1,3-oxazolyl, and 1,2,4-oxadiazolyl.

53. A compound, pharmaceutically acceptable salt, or solvate according to claim 41, wherein R₉ is 3-isoxazolyl or 5-isoxazolyl unsubstituted or substituted with one or two substituents selected from methyl and halogens.

54. A compound according to claim 41, of the formula I-C": 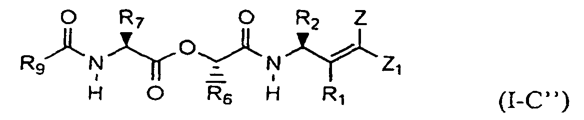 wherein R₁, R₂, R₆, R₇, R₉, Z, and Z₁ are as defined in claim 42,
or a pharmaceutically acceptable salt, or solvate thereof

55. A compound, pharmaceutically acceptable salt, or solvate according to claim 54, wherein R₂ is

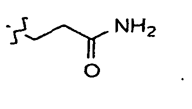

56. A compound, pharmaceutically acceptable salt, or solvate according to claim 54, wherein:
R₁ is H, F, or methyl;
Z and Z₁ are each independently selected from H, F, -CO₂R₂₁, -CN, and -C(O)NR₂₁,R₂₂, where R₂₁ and R₂₂ are each independently H, an alkyl group, a cycloalkyl group, a heterocycloalkyl group, an aryl group, a heteroaryl group, an acyl group, or a thloacyl group, or R₂₁ and R₂₂, together with the atom(s) to which they are bonded, form a heterocycloalkyl group, provided that Z and Z₁ are not both H;
or Z₁ and R₁, together with the atoms to which they are bonded, form a cycloalkyl or heterocycloalkyl group,
or Z and Z₁, together with the atoms to which they are bonded, form a cycloalkyl or heterocycloalkyl group;
R₂ is 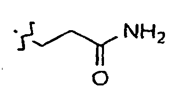
R₆ is unsubstituted or substituted phenylmethyl;
R₇ is alkyl or aryl; and
R₉ is 3-isoxazolyl or 5-isoxazolyl unsubstituted or substituted with one or two substituents selected from methyl and halogens.

57. A compound, pharmaceutically acceptable salt, or solvate according to claim 56, wherein R₇ is selected from 2-propyl, 2-methyl-2-propyl, 2-methyl-1-propyl, phenylmethyl, and naphthylmethyl.

58. A compound, pharmaceutically acceptable salt, or solvate according to claim 54, wherein R₁ is H, Z is H, and
R₂ is CH₂CH₂C(O)NH₂, R₆ is 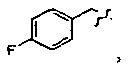 R₇ is CH(CH₃)₂, Z₁ is CO₂CH₂CH₃, and R₉ is

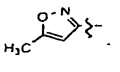

59. A compound, pharmaceutically acceptable salt, or solvate according to claims 1 or 58, having an antipicornaviral activity corresponding to an EC₅₀ less than or equal to 100 µM in an H1-HeLa cell culture assay.

60. A compound, pharmaceutically acceptable salt, or solvate according to claims 1 or 58, having an antirhinoviral activity corresponding to an EC₅₀ less than or equal to 10 µM in an H1-HeLa cell culture assay.

61. A pharmaceutical composition comprising:
(a) a therapeutically effective amount of at least one antipicornaviral agent that is a compound, pharmaceutically acceptable salt, of solvate as defined in any of claims 1 to 60; and
(b) a pharmaceutically acceptable carrier, diluent, vehicle, or excipient.

62. Use of a compound, pharmaceutically acceptable salt, or solvate as defined in any of claims 1 to 60 for the preparation of a medicament for the treatment of a mammalian disease condition mediated by picornaviral protease activity.

63. The use according to claim 62 for inhibiting the activity of a picornaviral 3C protease.

64. The use according to claim 63, wherein the picornaviral 3C protease is a rhinoviral protease.

## Patentansprüche

1. Verbindung der Formel I: 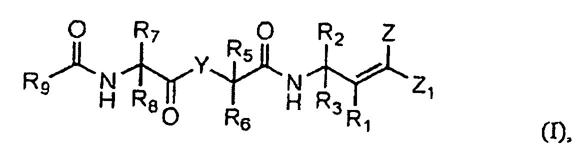 wobei:
Y -N(R_{y})-, -C(Ry)(Ry)- oder -O- ist, wobei R_{y} jeweils unabhängig H oder C₁-C₄-Alkyl darstellt;
R₁ H, F, eine Alkylgruppe, OH, SH oder eine O-Alkylgruppe darstellt;
R₂ und R₃ jeweils unabhängig H, 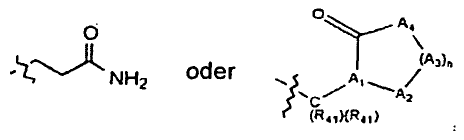 darstellen, wobei n eine ganze Zahl von 0 bis 5 ist, A₁ CH oder N ist, A₂ und jedes A₃ unabhängig ausgewählt sind unter C(R₄₁)(R₄₁), N(R₄₁), S, S(O), S(O)₂ und O, und A₄ NH oder NR₄₁ darstellt, wobei jedes R₄₁ unabhängig voneinander H oder C₁-C₄-Alkyl darstellt, mit der Maßgabe, dass nicht mehr als 2 Heteroatome aufeinanderfolgend im Ring auftreten, geformt durch A₁, A₂, (A₃)ₙ, A₄ und C=O; und mit der Maßgabe, dass mindestens einer von R₂ und R₃ 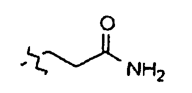 darstellt;
R₅ und R₆ jeweils unabhängig voneinander H, F, eine Alkylgruppe, eine Cycloalkylgruppe, eine Heterocycloalkylgruppe, eine Arylgruppe oder eine Heteroarylgruppe darstellen;
R₇ und R₈ jeweils unabhängig voneinander H, eine Alkylgruppe, eine Cycloalkylgruppe, eine Heterocycloalkylgruppe, eine Arylgruppe, eine Heteroarylgruppe, -OR₁₇, -SR₁₇, -NR₁₇R₁₈, -NR₁₉NR₁₇R₁₈ oder -NR₁₇OR₁₈ darstellen, wobei R₁₇, R₁₈ und R₁₉ jeweils unabhängig voneinander H, eine Alkylgruppe, eine Cycloalkylgruppe, eine Heterocycloalkylgruppe, eine Arylgruppe, eine Heteroarylgruppe oder eine Acylgruppe darstellen;
R₉ ein fünfgliedriger Heterozyklus ist, mit 1 bis 3 Heteroatomen, ausgewählt unter O, N und S; und
Z und Z₁ jeweils unabhängig voneinander H, F, eine Alkylgruppe, eine Cycloalkylgruppe, eine Heterocycloalkylgruppe, eine Arylgruppe, eine Heteroarylgruppe, -C(O)R₂₁, -CO₂R₂₁, -CN, -C(O)NR₂₁R₂₂, -C(O)NR₂₁OR₂₂, -C(S)R₂₁, -C(S)NR₂₁R₂₂, -NO₂, -SOR₂₁, -SO₂R₂₁, -SO₂NR₂₁R₂₂, -SO(NR₂₁)(OR₂₂), -SONR₂₁, -SO₃R₂₁, -PO(OR₂₁)₂, -PO(R₂₁)(R₂₂), -PO(NR₂₁R₂₂)(OR₂₃), PO(NR₂₁R₂₂)(NR₂₃R₂₄), -C(O)NR₂₁NR₂₂R₂₃ oder -C(S)NR₂₁NR₂₂R₂₃ darstellen, wobei R₂₁, R₂₂, R₂₃ und R₂₄ jeweils unabhängig voneinander H, eine Alkylgruppe, eine Cycloalkylgruppe, eine Heterocycloalkylgruppe, eine Arylgruppe, eine Heteroarylgruppe, eine Acylgruppe oder eine Thioacylgruppe darstellen oder irgendwelche zwei unter R₂₁, R₂₂, R₂₃ und R₂₄, zusammen mit dem Atom oder den Atomen, an welche sie gebunden sind, eine Heterocycloalkylgruppe formen, mit der Maßgabe, dass Z und Z₁ nicht beide H sind;
oder Z₁ und R₁, zusammen mit den Atomen, an welche sie gebunden sind, formen eine Cycloalkylgruppe oder eine Heterocycloalkylgruppe;
oder Z und Z₁, zusammen mit den Atomen, an welche sie gebunden sind, formen eine Cycloalkylgruppe oder eine Heterocycloalkylgruppe;
oder ein pharmazeutisch akzeptables Salz oder Solvat davon, mit der Maßgabe, dass die folgenden Verbindungen ausgeschlossen sind: 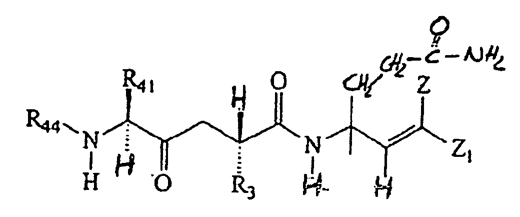 wobei R₃, Z, Z₁, R₄₁ und R₄₄ wie folgt sind:
R₃ ist CH₂(p-F)Ph, Z ist H, Z₁ ist CO₂CH₂CH₃, R₄₁ ist CH(CH₃)₂ und R₄₄ ist 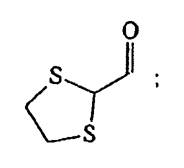
R₃ ist CH₂(p-F)Ph, Z ist H, Z₁ ist 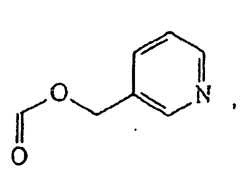 R₄₁ ist CH(CH₃)₂ und R₄₄ ist 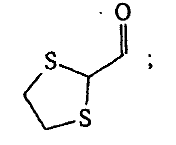
R₃ ist CH₂(p-F)Ph, Z ist H, Z₁ ist CO₂CH₂CH₃, R₄₁ ist CH(CH₃)₂ und R₄₄ ist 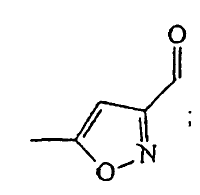 und 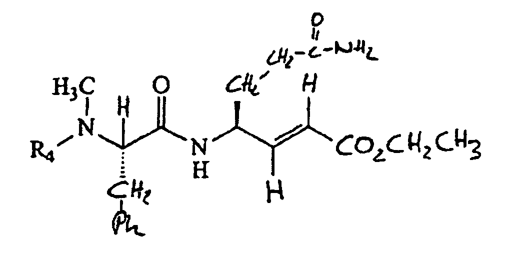
wobei R₄ 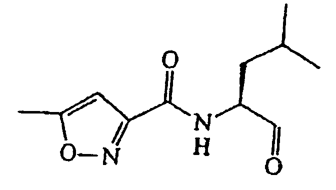 ist.

2. Verbindung, pharmazeutisch akzeptables Salz oder Solvat in Übereinstimmung mit Anspruch 1, wobei R₂ und R₃ jeweils unabhängig voneinander H; 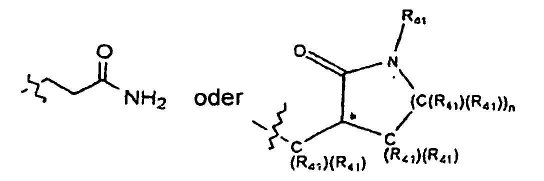 darstellen, wobei n eine ganze Zahl von 0 bis 5 ist, jedes R₄₁ unabhängig H oder Niederalkyl darstellt, und die Stereochemie am Kohlenstoffatom, versehen mit einem Sternchen (*) R oder S sein kann, mit der Maßgabe, dass mindestens einer von R₂ und R₃ 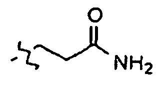 darstellt.

3. Verbindung, pharmazeutisch akzeptables Salz oder Solvat in Übereinstimmung mit Anspruch 1, wobei Y -N(Ry)- darstellt, wobei R_{y} H oder C₁-C₄-Alkyl ist.

4. Verbindung, pharmazeutisch akzeptables Salz oder Solvat nach Anspruch 3, wobei
Z und Z₁ jeweils unabhängig ausgewählt sind unter H, F, C₁-C₄-Alkyl, -CO₂R₂₁ und -C(O)NR₂₁R₂₂, wobei R₂₁ und R₂₂ jeweils unabhängig voneinander H, eine Alkylgruppe, eine Cycloalkylgruppe, eine Heterocycloalkylgruppe, eine Arylgruppe, eine Heteroarylgruppe, eine Acylgruppe oder eine Thioacylgruppe darstellen, oder R₂₁ und R₂₂, zusammen mit dem Atom oder den Atomen, an welche sie gebunden sind, eine Heterocycloalkylgruppe formen, mit der Maßgabe, dass Z und Z₁ nicht beide H sind;
oder Z₁ und R₁, zusammen mit den Atomen, an welche sie gebunden sind, eine Cycloalkylgruppe oder Heterocycloalkylgruppe formen;
oder Z und Z₁, zusammen mit den Atomen, an welche sie gebunden sind, eine Cycloalkylgruppe oder Heterocycloalkylgruppe formen.

5. Verbindung, pharmazeutisch akzeptables Salz oder Solvat nach Anspruch 3, wobei R₁ H, F oder Methyl ist.

6. Verbindung, pharmazeutisch akzeptables Salz oder Solvat nach Anspruch 3, wobei mindestens einer von R₂ oder R₃ 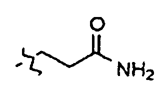 ist.

7. Verbindung, pharmazeutisch akzeptables Salz oder Solvat nach Anspruch 6, wobei einer von R₅ und R₆ H ist und der andere Alkyl oder Aryl ist.

8. Verbindung, pharmazeutisch akzeptables Salz oder Solvat nach Anspruch 3, wobei einer von R₅ und R₆ H ist und der andere Alkyl oder Aryl ist.

9. Verbindung, pharmazeutisch akzeptables Salz oder Solvat nach Anspruch 3, wobei einer von R₅ und R₆ H ist und der andere nichtsubstituiertes oder substituiertes Phenylmethyl ist.

10. Verbindung, pharmazeutisch akzeptables Salz oder Solvat nach Anspruch 3, wobei R₇ und R₈ jeweils unabhängig H, eine Alkylgruppe, eine Cycloalkylgruppe, eine Heterocycloalkylgruppe, eine Arylgruppe oder eine Heteroarylgruppe darstellen.

11. Verbindung, pharmazeutisch akzeptables Salz oder Solvat nach Anspruch 3, wobei einer von R₇ und R₈ H ist und der andere Alkyl oder Aryl ist.

12. Verbindung, pharmazeutisch akzeptables Salz oder Solvat nach Anspruch 3, wobei einer von R₇ und R₈ H ist und der andere 2-Propyl, 2-Methyl-2-propyl, 2-Methyl-1-propyl, Phenylmethyl oder Naphthylmethyl ist.

13. Verbindung, pharmazeutisch akzeptables Salz oder Solvat nach Anspruch 3, wobei R₉ ein fünfgliedriger Heterozyklus ist, mit mindestens einem Stickstoffatom und einem Sauerstoffatom.

14. Verbindung, pharmazeutisch akzeptables Salz oder Solvat nach Anspruch 3, wobei R₉ ausgewählt ist unter substituiertem und nichtsubstituierten 1,2-Oxazolyl, 1,3-Oxazolyl und 1,2,4-Oxadiazolyl.

15. Verbindung, pharmazeutisch akzeptables Salz oder Solvat nach Anspruch 3, wobei R₉ 3-Isoxazolyl oder 5-Isoxazolyl ist, nichtsubstituiert oder substituiert mit einem oder zwei Substituenten, ausgewählt unter Methyl und Halogenen.

16. Verbindung nach Anspruch 3 der Formel I-A": 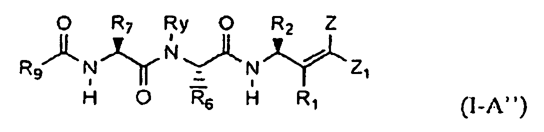 wobei R₁, R₂, R₆, R₇, R₉, R_{y}, Z und Z₁ wie in Anspruch 3 definiert sind, oder ein pharmazeutisch akzeptables Salz oder Solvat davon.

17. Verbindung, pharmazeutisch akzeptables Salz oder Solvat nach Anspruch 16, wobei R₂ 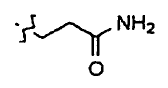 ist.

18. Verbindung, pharmazeutisch akzeptables Salz oder Solvat nach Anspruch 16, wobei:
R_{y} H oder Methyl ist;
R₁ H, F oder Methyl ist;
Z und Z₁ jeweils unabhängig voneinander ausgewählt sind unter H, F, -CO₂R₂₁, -CN und -C(O)NR₂₁R₂₂, wobei R₂₁ und R₂₂ jeweils unabhängig H, eine Alkylgruppe, eine Cycloalkylgruppe, eine Heterocycloalkylgruppe, eine Arylgruppe, eine Heteroarylgruppe, eine Acylgruppe oder eine Thioacylgruppe darstellen, oder R₂₁ und R₂₂, zusammen mit dem Atom oder den Atomen, an welche sie gebunden sind, eine Heterocycloalkylgruppe formen, mit der Maßgabe, dass Z und Z₁ nicht beide H sind;
oder Z₁ und R₁, zusammen mit den Atomen, an welche sie gebunden sind, eine Cycloalkylgruppe oder Heterocycloalkylgruppe formen;
oder Z₁ und R₁, zusammen mit den Atomen, an welche sie gebunden sind, eine Cycloalkylgruppe oder Heterocycloalkylgruppe formen;
oder Z und Z₁, zusammen mit den Atomen, an welche sie gebunden sind, eine Cycloalkylgruppe oder Heterocycloalkylgruppe formen;
R₂ 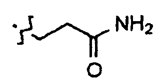 darstellt;
R₆ nichtsubstituiertes oder substituiertes Phenylmethyl darstellt;
R₇ Alkyl oder Aryl darstellt; und
R₉ 3-Isoxazolyl oder 5-Isoxazolyl darstellt, nichtsubstituiert oder substituiert mit einem oder zwei Substituenten, ausgewählt unter Methyl und Halogenen.

19. Verbindung, pharmazeutisch akzeptables Salz oder Solvat nach Anspruch 18, wobei R₇ ausgewählt ist unter 2-Propyl, 2-Methyl-2-propyl, 2-Methyl-1-propyl, Phenylmethyl und Naphthylmethyl.

20. Verbindung, pharmazeutisch akzeptables Salz oder Solvat nach Anspruch 16, wobei R_{y}, R₁ und Z jeweils H sind und:
R₂ CH₂CH₂C(O)NH₂ ist, R₆ CH₂Ph ist, R₇ CH₂CH(CH₃)₂ ist, Z₁ CO₂CH₂CH₃ ist und R₉ 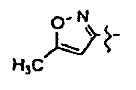 ist;
R₂ CH₂CH₂C(O)NH₂ ist, R₆ CH₂Ph ist, R₇ CH₂CH(CH₃)₂ ist, Z₁ CO₂CH₂CH₃ ist und R₉ 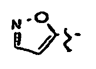 ist;
R₂ CH₂CH₂C(O)NH₂ ist, R₆ 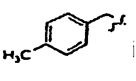 ist, R₇ C(CH₃)₃ ist, Z₁ CO₂CH₂CH₃ ist und R₉ 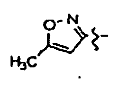 ist;
R₂ CH₂CH₂C(O)NH₂ ist, R₆ 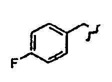 ist, R₇ C(CH₃)₃ ist, Z₁ CO₂CH₂CH₃ ist und R₉ 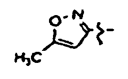 ist;
R₂ CH₂CH₂C(O)NH₂ ist, R₆ 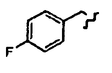 ist, R₇ CH(CH₃)₂ ist, Z₁ CO₂CH₂CH₃ ist und R₉ 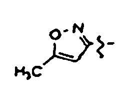 ist.

21. Verbindung, pharmazeutisch akzeptables Salz oder Solvat nach Anspruch 16, wobei R_{y} CH₃ ist, R₁ und Z jeweils H sind, und
R₂ ist CH₂CH₂C(O)NH₂, R₆ ist 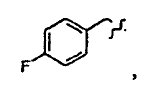 R₇ ist 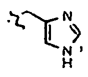 Z₁ ist CO₂CH₂CH₃ und R₉ ist 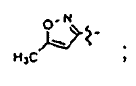 R₂ ist CH₂CH₂C(O)NH₂, R₆ ist CH₂Ph, R₇ ist CH₂CH(CH₃)₂ und R₉ ist 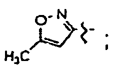 R₂ ist CH₂CH₂C(O)NH₂, R₆ ist 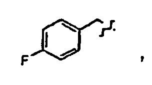 R₇ ist 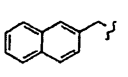 und R₉ ist 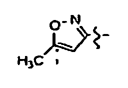 R₂ ist CH₂CH₂C(O)NH₂, R₆ ist 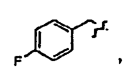 R₇ ist CH₂CH(CH₃)₂ und R₉ ist 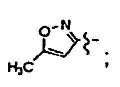 oder
R₂ ist CH₂CH₂C(O)NH₂, R₆ ist 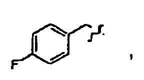 R₇ ist 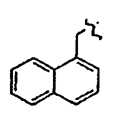 und R₉ ist

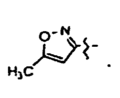

22. Verbindung, pharmazeutisch akzeptables Salz oder Solvat nach Anspruch 16, ausgewählt aus der Gruppe, bestehend aus:

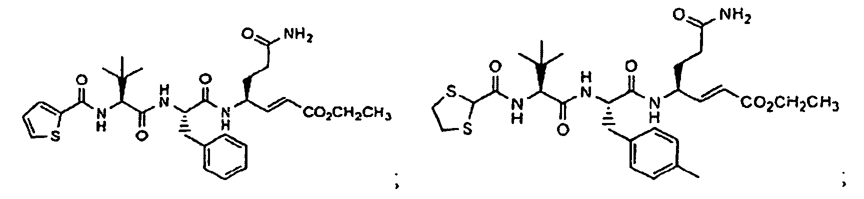

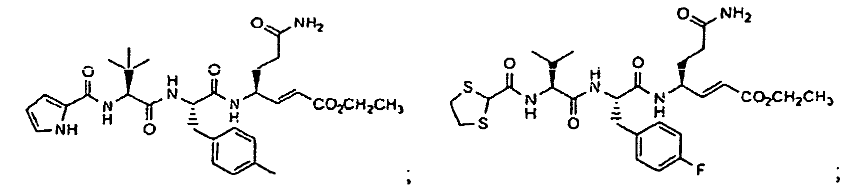

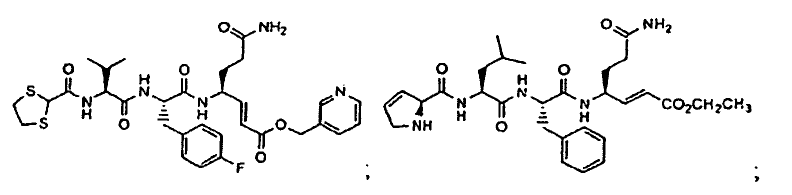

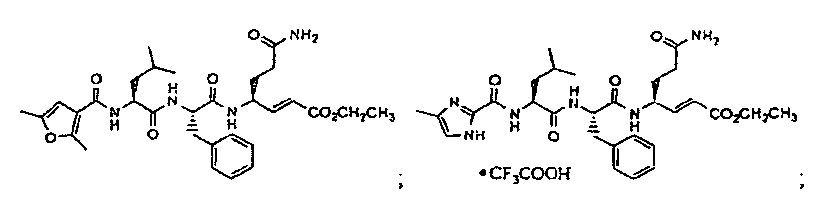

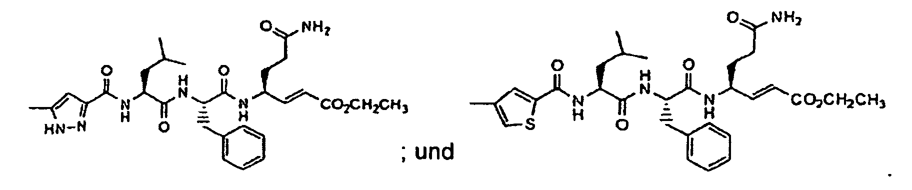

23. Verbindung, pharmazeutisch akzeptables Salz oder Solvat nach Anspruch 1, wobei Y -CH₂- ist.

24. Verbindung, pharmazeutisch akzeptables Salz oder Solvat nach Anspruch 23, wobei Z und Z₁ jeweils unabhängig voneinander ausgewählt sind unter H, F, C₁-C₄-Alkyl, -CO₂R₂₁ und -C(O)NR₂₁R₂₂, wobei R₂₁ und R₂₂ jeweils unabhängig H, eine Alkylgruppe, eine Cycloalkylgruppe, eine Heterocycloalkylgruppe, eine Arylgruppe, eine Heteroarylgruppe, eine Acylgruppe oder eine Thioacylgruppe darstellen, oder R₂₁ und R₂₂, zusammen mit dem Atom oder den Atomen, an welche sie gebunden sind, eine Heterocycloalkylgruppe formen, mit der Maßgabe, dass Z und Z₁ nicht beide H sind;
oder Z₁ und R₁, zusammen mit den Atomen, an welche sie gebunden sind, eine Cycloalkylgruppe oder Heterocycloalkylgruppe formen;
oder Z und Z₁, zusammen mit den Atomen, an welche sie gebunden sind, eine Cycloalkylgruppe oder Heterocycloalkylgruppe formen.

25. Verbindung, pharmazeutisch akzeptables Salz oder Solvat nach Anspruch 23, wobei R₁ H, F oder Methyl ist.

26. Verbindung, pharmazeutisch akzeptables Salz oder Solvat nach Anspruch 23, wobei mindestens einer von R₂ und R₃ 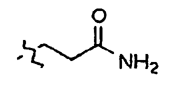 ist.

27. Verbindung, pharmazeutisch akzeptables Salz oder Solvat nach Anspruch 26, wobei einer von R₅ und R₆ H ist und der andere Alkyl oder Aryl ist.

28. Verbindung, pharmazeutisch akzeptables Salz oder Solvat nach Anspruch 23, wobei einer von R₅ und R₆ H ist und der andere Alkyl oder Aryl ist.

29. Verbindung, pharmazeutisch akzeptables Salz oder Solvat nach Anspruch 23, wobei einer von R₅ und R₆ H ist und der andere nichtsubstituiertes oder substituiertes Phenylmethyl ist.

30. Verbindung, pharmazeutisch akzeptables Salz oder Solvat nach Anspruch 23, wobei R₇ und R₈ jeweils unabhängig H, eine Alkylgruppe, eine Cycloalkylgruppe, eine Heterocycloalkylgruppe, eine Arylgruppe oder eine Heteroarylgruppe darstellen.

31. Verbindung, pharmazeutisch akzeptables Salz oder Solvat nach Anspruch 23, wobei einer von R₇ und R₈ H ist und der andere Alkyl oder Aryl ist.

32. Verbindung, pharmazeutisch akzeptables Salz oder Solvat nach Anspruch 23, wobei einer von R₇ und R₈ H ist und der andere 2-Propyl, 2-Methyl-2-propyl, 2-Methyl-1-propyl, Phenylmethyl oder Naphthylmethyl ist.

33. Verbindung, pharmazeutisch akzeptables Salz oder Solvat nach Anspruch 23, wobei R₉ ein fünfgliedriger Heterozyklus ist, mit mindestens einem Stickstoffatom und einem Sauerstoffatom.

34. Verbindung, pharmazeutisch akzeptables Salz oder Solvat nach Anspruch 33, wobei R₉ nichtsubstituiertes oder substituiertes 1,2-Oxazolyl, 1,3-Oxazolyl oder 1,2,4-Oxadiazolyl ist.

35. Verbindung, pharmazeutisch akzeptables Salz oder Solvat nach Anspruch 34, wobei Rg 3-Isoxazolyl oder 5-Isoxazolyl darstellt, nichtsubstituiert oder substituiert mit einem oder zwei Substituenten, ausgewählt unter Methyl und Halogenen.

36. Verbindung nach Anspruch 23 der Formel I-B": 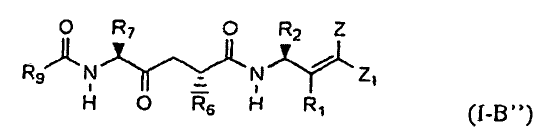 wobei R₁, R₂, R₆, R₇, R₉, Z und Z₁ wie in Anspruch 23 definiert sind, oder ein pharmazeutisch akzeptables Salz oder Solvat davon.

37. Verbindung, pharmazeutisch akzeptables Salz oder Solvat nach Anspruch 36, wobei R₂ 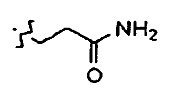 ist.

38. Verbindung, pharmazeutisch akzeptables Salz oder Solvat nach Anspruch 36, wobei:
R₁ H, F oder Methyl ist;
Z und Z₁ jeweils unabhängig voneinander ausgewählt sind unter H, F, -CO₂R₂₁, -CN und -C(O)NR₂₁R₂₂, wobei R₂₁ und R₂₂ jeweils unabhängig voneinander H, eine Alkylgruppe, eine Cycloalkylgruppe, eine Heterocycloalkylgruppe, eine Arylgruppe, eine Heteroarylgruppe, eine Acylgruppe oder eine Thioacylgruppe darstellen, oder R₂₁ und R₂₂, zusammen mit dem Atom oder den Atomen, an welche sie gebunden ist, eine Heterocycloalkylgruppe formen, mit der Maßgabe, dass Z und Z₁ nicht beide H sind;
oder Z₁ und R₁, zusammen mit den Atomen, an welche sie gebunden sind, eine Cycloalkylgruppe oder eine Heterocycloalkylgruppe formen;
oder Z und Z₁, zusammen mit den Atomen, an welche sie gebunden sind, eine Cycloalkylgruppe oder eine Heterocycloalkylgruppe formen;
R₂ 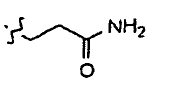 ist;
R₆ ein nichtsubstituiertes oder substituiertes Phenylmethyl darstellt;
R₇ Alkyl oder Aryl darstellt; und
R₉ 3-Isoxazolyl oder 5-Isoxazolyl darstellt, nichtsubstituiert oder substituiert mit einem oder zwei Substituenten, ausgewählt unter Methyl und Halogenen.

39. Verbindung, pharmazeutisch akzeptables Salz oder Solvat nach Anspruch 38, wobei R₇ ausgewählt ist unter 2-Propyl, 2-Methyl-2-propyl, 2-Methyl-1-propyl, Phenylmethyl und Naphthylmethyl.

40. Verbindung, pharmazeutisch akzeptables Salz oder Solvat nach Anspruch 36, wobei R₁ H ist und:
R₂ ist CH₂CH₂C(O)NH₂, R₆ ist 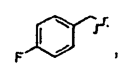 R₇ ist CH(CH₃)₂, Z ist H, Z₁ ist CO₂CH₂CH₃ und R₉ ist

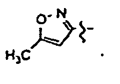

41. Verbindung, pharmazeutisch akzeptables Salz oder Solvat nach Anspruch 1, wobei Y -O- ist.

42. Verbindung, pharmazeutisch akzeptables Salz oder Solvat nach Anspruch 41, wobei Z und Z₁ jeweils unabhängig voneinander ausgewählt sind unter H, F, C₁-C₄-Alkyl, -CO₂R₂₁ und -C(O)NR₂₁R₂₂, wobei R₂₁ und R₂₂ jeweils unabhängig voneinander H, eine Alkylgruppe, eine Cycloalkylgruppe, eine Heterocycloalkylgruppe, eine Arylgruppe, eine Heteroarylgruppe, eine Acylgruppe oder eine Thioacylgruppe darstellen; oder R₂₁ und R₂₂, zusammen mit dem Atom oder den Atomen, an welche sie gebunden sind, eine Heterocycloalkylgruppe formen, mit der Maßgabe, dass Z und Z₁ nicht beide H sind;
oder Z₁ und R₁, zusammen mit den Atomen, an welche sie gebunden sind, eine Cycloalkylgruppe oder Heterocycloalkylgruppe formen;
oder Z und Z₁, zusammen mit den Atomen, an welche sie gebunden sind, eine Cycloalkylgruppe oder Heterocycloalkylgruppe formen.

43. Verbindung, pharmazeutisch akzeptables Salz oder Solvat nach Anspruch 41, wobei R₁ H, F oder Methyl ist.

44. Verbindung, pharmazeutisch akzeptables Salz oder Solvat nach Anspruch 41, wobei mindestens einer von R₂ und R₃ 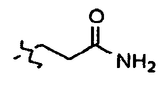 ist.

45. Verbindung, pharmazeutisch akzeptables Salz oder Solvat nach Anspruch 44, wobei einer von R₅ und R₆ H ist und der andere Alkyl oder Aryl ist,

46. Verbindung, pharmazeutisch akzeptables Salz oder Solvat nach Anspruch 41, wobei einer von R₅ und R₆ H ist und der andere Alkyl oder Aryl ist.

47. Verbindung, pharmazeutisch akzeptables Salz oder Solvat nach Anspruch 41, wobei einer von R₅ und R₆ H ist und der andere nichtsubstituiertes oder substituiertes Phenylmethyl ist.

48. Verbindung, pharmazeutisch akzeptables Salz oder Solvat nach Anspruch 41, wobei R₇ und R₈ jeweils unabhängig H, eine Alkylgruppe, eine Cycloalkylgruppe, eine Heterocycloalkylgruppe, eine Arylgruppe oder eine Heteroarylgruppe darstellen.

49. Verbindung, pharmazeutisch akzeptables Salz oder Solvat nach Anspruch 41, wobei einer von R₇ und R₈ H ist und der andere Alkyl oder Aryl ist.

50. Verbindung, pharmazeutisch akzeptables Salz oder Solvat nach Anspruch 42, wobei einer von R₇ und R₈ H ist und der andere 2-Propyl, 2-Methyl-2-propyl, 2-Methyl-1-propyl, Phenylmethyl oder Naphthylmethyl ist.

51. Verbindung, pharmazeutisch akzeptables Salz oder Solvat nach Anspruch 41, wobei R₉ ein fünfgliedriger Heterozyklus ist, mit mindestens einem Stickstoffatom und einem Sauerstoffatom.

52. Verbindung, pharmazeutisch akzeptables Salz oder Solvat nach Anspruch 41, wobei R₉ ausgewählt ist unter substituiertem und nichtsubstituiertem 1,2-Oxazolyl, 1,3-Oxazolyl und 1,2,4-Oxadiazolyl.

53. Verbindung, pharmazeutisch akzeptables Salz oder Solvat nach Anspruch 41, wobei R₉ 3-lsoxazolyl oder 5-Isoxazolyl darstellt, nichtsubstituiert oder substituiert mit einem oder zwei Substituenten, ausgewählt unter Methyl und Halogenen.

54. Verbindung nach Anspruch 41 der Formel I-C": 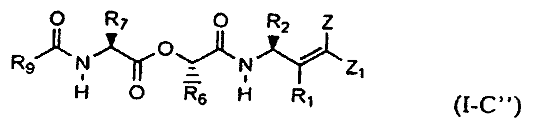 wobei R₁, R₂, R₆, R₇, R₉, Z und Z₁ wie in Anspruch 42 definiert sind, oder ein pharmazeutisch akzeptables Salz oder Solvat davon.

55. Verbindung, pharmazeutisch akzeptables Salz oder Solvat nach Anspruch 54, wobei R₂ 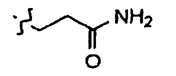 ist.

56. Verbindung, pharmazeutisch akzeptables Salz oder Solvat nach Anspruch 54, wobei:
R₁ H, F oder Methyl ist;
Z und Z₁ jeweils unabhängig voneinander ausgewählt sind unter H, F, -CO₂R₂₁, -CN und -C(O)NR₂₁R₂₂, wobei R₂₁ und R₂₂ jeweils unabhängig voneinander H, eine Alkylgruppe, eine Cycloalkylgruppe, eine Heterocycloalkylgruppe, eine Arylgruppe, eine Heteroarylgruppe, eine Acylgruppe oder eine Thioacylgruppe darstellen, oder R₂₁ und R₂₂, zusammen mit dem Atom oder den Atomen, an welche sie gebunden sind, eine Heterocycloalkylgruppe formen, mit der Maßgabe, dass Z und Z₁ nicht beide H sind;
oder Z und R₁, zusammen mit den Atomen, an welche sie gebunden sind, eine Cycloalkylgruppe oder Heterocycloalkylgruppe formen,
oder Z und Z₁, zusammen mit den Atomen, an welche sie gebunden sind, eine Cycloalkylgruppe oder Heterocycloalkylgruppe formen;
R₂ 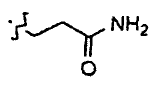 ist;
R₆ nichtsubstituiertes oder substituiertes Phenylmethyl ist;
R₇ Alkyl oder Aryl ist; und
R₉ 3-Isoxazolyl oder 5-Isoxazolyl darstellt, nichtsubstituiert oder substituiert mit einem oder zwei Substituenten, ausgewählt unter Methyl und Halogenen.

57. Verbindung, pharmazeutisch akzeptables Salz oder Solvat nach Anspruch 56, wobei R₇ ausgewählt ist unter 2-Propyl, 2-Methyl-2-propyl, 2-Methyl-1-propyl, Phenylmethyl und Naphthylmethyl.

58. Verbindung, pharmazeutisch akzeptables Salz oder Solvat nach Anspruch 54, wobei R₁ H ist, Z H ist und:
R₂ ist CH₂CH₂C(O)NH₂, R₆ ist 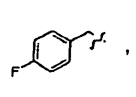 R₇ ist CH(CH₃)₂, Z₁ ist CO₂CH₂CH₃ und R₉ ist

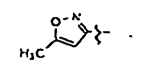

59. Verbindung, pharmazeutisch akzeptables Salz oder Solvat nach Ansprüchen 1 oder 58 mit einer antipicornaviralen Aktivität, korrespondierend zu einem EC₅₀ Wert von weniger als oder gleich 100 µM in einem H1-HeLa-Zellkulturassay.

60. Verbindung, pharmazeutisch akzeptables Salz oder Solvat nach Ansprüchen 1 oder 58, mit einer antirhinoviralen Aktivität korrespondierend einem EC₅₀ Wert von weniger als oder gleich 10µM in einem H1-HeLa-Zellkulturassay.

61. Pharmazeutische Zusammensetzung, umfassend:
(a) eine therapeutisch effektive Menge mindestens eines antipicornaviralen Mittels, was eine Verbindung, ein pharmazeutisch akzeptables Salz oder ein Solvat ist, wie in irgendeinem der Ansprüche 1 bis 60 definiert; und
(b) einen pharmazeutisch akzeptablen Träger, ein pharmazeutisch akzeptables Verdünnungsmittel, Vehikel oder Hilfsmittel.

62. Verwendung einer Verbindung, eines pharmazeutisch akzeptablen Salzes oder Solvats wie in irgendeinem der Ansprüche 1 bis 60 definiert für die Herstellung eines Medikaments für die Behandlung eines Krankheitszustands bei Säugetieren, vermittelt durch picornavirale Proteaseaktivität.

63. Verwendung nach Anspruch 62 zur Inhibierung der Aktivität einer picornaviralen 3C Protease.

64. Verwendung nach Anspruch 63, wobei die picornavirale 3C Protease eine rhinovirale Protease ist.

## Revendications

1. Composé de la formule I : 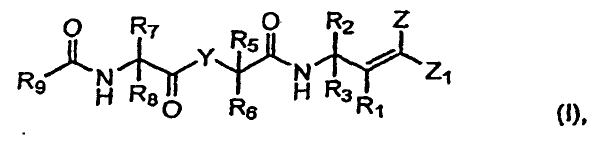 dans lequel :
Y est -N(R_{y})-, -C(R_{y}) (R_{y})- ou -O-, où chaque R_{y} est indépendamment H ou un radical alkyle en C₁ à C₄ ;
R₁ est H, F, un radical alkyle, OH, SH ou un radical O-alkyle ;
R₂ et R₃ sont chacun indépendamment, H, 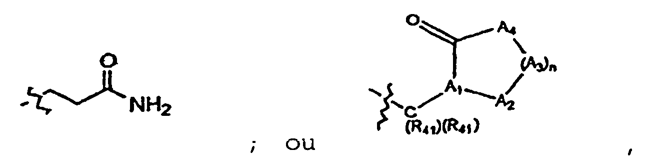 où n est un entier allant de 0 à 5, A₁ est CH ou N, A₂ et chaque A₃ sont indépendamment choisis parmi C(R₄₁)(R₄₁), N(R₄₁), S, S(O), S (O)₂ et O, et A₄ est NH ou NR₄₁, où chaque R₄₁ est indépendamment H ou un radical alkyle en C₁ à C₄, pourvu que pas plus de 2 hétéroatomes ne soient consécutifs dans le cycle formé par A₁, A₂, (A₃)ₙ, A₄ et C=O, et pourvu qu'au moins un parmi R₂ et R₃ soit 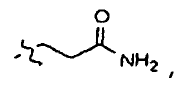
R₅ et R₆ sont chacun indépendamment H, F, un radical alkyle, un radical cycloalkyle, un radical hétérocycloalkyle, un radical aryle ou un radical hétéroaryle ;
R₇ et R₈ sont chacun indépendamment H, un radical alkyle, un radical cycloalkyle, un radical hétérocycloalkyle, un radical aryle, un radical hétéroaryle, -OR₁₇, -SR₁₇, -NR₁₇R₁₈, -NR₁₉NR₁₇R₁₈ ou -NR₁₇OR₁₈, où R₁₇, R₁₈ et R₁₉ sont chacun indépendamment H, un radical alkyle, un radical cycloalkyle, un radical hétérocycloalkyle, un radical aryle, un radical hétéroaryle, ou un radical acyle ;
R₉ est un hétérocycle à cinq membres, ayant un à trois hétéroatomes choisis parmi O, N et S ; et
Z et Z₁ sont chacun indépendamment, H, F, un radical alkyle, un radical cycloalkyle, un radical hétérocycloalkyle, un radical aryle, un radical hétéroaryle, -C(O)R₂₁, -CO₂R₂₁, -CN, -C(O)NR₂₁R₂₂, -C(O)NR₂₁OR₂₂, -C(S)R₂₁, -C(S)NR₂₁R₂₂, -NO₂, -SOR₂₁, -SO₂R₂₁, -SO₂NR₂₁R₂₂, -SO(NR₂₁)(OR₂₂), -SONR₂₁, -SO₃R₂₁, -PO(OR₂₁)₂, -PO(R₂₁)(R₂₂), -PO(NR₂₁R₂₂)(OR₂₃), -PO(NR₂₁R₂₂)(NR₂₃R₂₄), -C(O)NR₂₁NR₂₂R₂₃ ou -C(S)NR₂₁NR₂₂R₂₃, où R₂₁, R₂₂, R₂₃ et R₂₄ sont chacun indépendamment H, un radical alkyle, un radical cycloalkyle, un radical hétérocycloalkyle, un radical aryle, un radical hétéroaryle, un radical acyle ou un radical thioacyle, ou deux quelconques parmi R₂₁, R₂₂, R₂₃ et R₂₄ forment conjointement avec le ou les atomes auxquels ils sont liés, un radical hétérocycloalkyle, pourvu que Z et Z₁ ne soient pas tous deux H ;
ou Z₁ et R₁, conjointement avec les atomes auxquels ils sont liés, forment un radical cycloalkyle ou hétérocycloalkyle ;
ou Z et Z₁, conjointement avec les atomes auxquels ils sont liés, forment un radical cycloalkyle ou hétérocycloalkyle ;
ou un sel pharmaceutiquement acceptable ou un solvate de celui-ci, à condition que les composés suivants soient exclus : 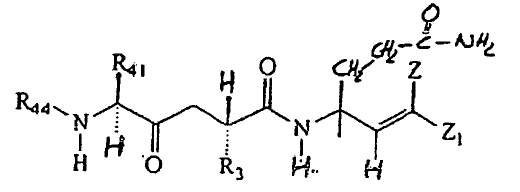 où R₃, Z, Z₁, R₄₁ et R₄₄ sont les suivants :
R₃ est CH₂(*p*-F)Ph, Z est H, Z₁ est CO₂CH₂CH₃, R₄₁ est CH(CH₃)₂ et R₄₄ est 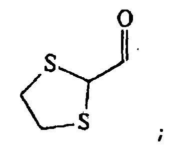
R₃ est CH₂(*p*-F)Ph, Z est H, Z₁ est 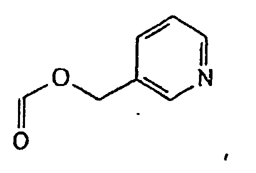 R₄₁ est CH (CH₃)₂ et R₄₄ est 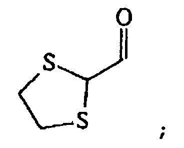
R₃ est CH₂(*p*-F)Ph, Z est H, Z₁ est CO₂CH₂CH₃, R₄₁ est CH(CH₃)₂ et R₄₄ est 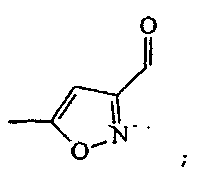 et 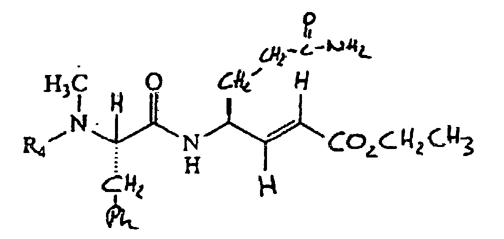
où R₄ est :

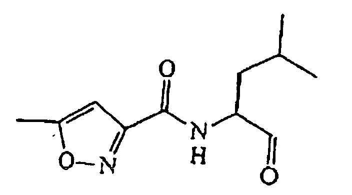

2. Composé, sel pharmaceutiquement acceptable ou solvate selon la revendication 1, dans lequel R₂ et R₃ sont chacun indépendamment H ; 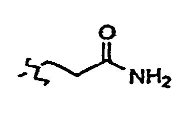 ou 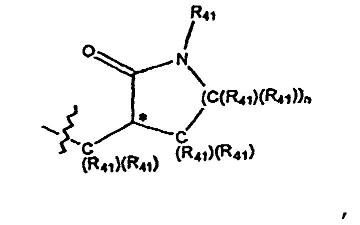 où n est un entier allant de 0 à 5, chaque R₄₁ est indépendamment H ou un radical alkyle inférieur, et la stéréochimie au carbone portant un astérisque (*) peut être R ou S, pourvu qu'au moins un parmi R₂ et R₃ soit

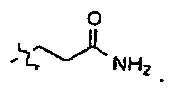

3. Composé, sel pharmaceutiquement acceptable ou solvate selon la revendication 1, dans lequel Y est -N(R_{y})-, où R_{y} est H ou un radical alkyle en C₁ à C₄.

4. Composé, sel pharmaceutiquement acceptable ou solvate selon la revendication 3, dans lequel : Z et Z₁ sont chacun indépendamment choisis parmi H, F, un radical alkyle en C₁ à C₄, -CO₂R₂₁, et -C(O)NR₂₁R₂₂, où R₂₁ et R₂₂ sont chacun indépendamment H, un radical alkyle, un radical cycloalkyle, un radical hétérocycloalkyle, un radical aryle, un radical hétéroaryle, un radical acyle ou un radical thioacyle, ou R₂₁ et R₂₂, conjointement avec le ou les atomes auxquels ils sont liés, forment un radical hétérocycloalkyle, pourvu que Z et Z₁ ne soient pas tous deux H ;
ou Z₁ et R₁, conjointement avec les atomes auxquels ils sont liés, forment un radical cycloalkyle ou hétérocycloalkyle ;
ou Z et Z₁, conjointement avec les atomes auxquels ils sont liés, forment un radical cycloalkyle ou hétérocycloalkyle.

5. Composé, sel pharmaceutiquement acceptable ou solvate selon la revendication 3, dans lequel R₁ est H, F ou méthyle.

6. Composé, sel pharmaceutiquement acceptable ou solvate selon la revendication 3, dans lequel au moins un parmi R₂ ou R₃ est

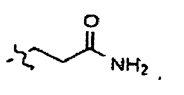

7. Composé, sel pharmaceutiquement acceptable ou solvate selon la revendication 6, dans lequel l'un parmi R₅ et R₆ est H et l'autre est alkyle ou aryle.

8. Composé, sel pharmaceutiquement acceptable ou solvate selon la revendication 3, dans lequel l'un parmi R₅ et R₆ est H et l'autre est alkyle ou aryle.

9. Composé, sel pharmaceutiquement acceptable ou solvate selon la revendication 3, dans lequel l'un parmi R₅ et R₆ est H et l'autre est phénylméthyle substitué ou non substitué.

10. Composé, sel pharmaceutiquement acceptable ou solvate selon la revendication 3, dans lequel R₇ et R₈ sont chacun indépendamment H, un radical alkyle, un radical cycloalkyle, un radical hétérocycloalkyle, un radical aryle ou un radical hétéroaryle.

11. Composé, sel pharmaceutiquement acceptable ou solvate selon la revendication 3, dans lequel l'un parmi R₇ et R₈ est H et l'autre est alkyle ou aryle.

12. Composé, sel pharmaceutiquement acceptable ou solvate selon la revendication 3, dans lequel l'un parmi R₇ et R₈ est H et l'autre est 2-propyle, 2-méthyl-2-propyle, 2-méthyl-1-propyle, phénylméthyle
ou naphtylméthyle.

13. Composé, sel pharmaceutiquement acceptable ou solvate selon la revendication 3, dans lequel R₉ est un hétérocycle à cinq membres ayant au moins un hétéroatome azote et un hétéroatome oxygène.

14. Composé, sel pharmaceutiquement acceptable ou solvate selon la revendication 3, dans lequel R₉ est choisi parmi les radicaux 1,2-oxazolyle, 1,3-oxazolyle et 1,2,4-oxadiazolyle substitués et non substitués.

15. Composé, sel pharmaceutiquement acceptable ou solvate selon la revendication 3, dans lequel R₉ est 3-isoxazolyle ou 5-isoxazolyle non substitué ou substitué avec un ou deux substituants choisis parmi méthyle et les halogènes.

16. Composé selon la revendication 3 de la formule I-A'' : 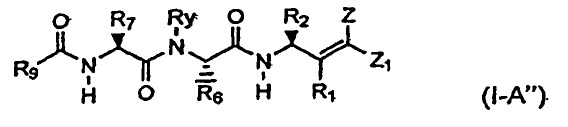 dans laquelle R₁, R₂, R₆, R₇, R₉, R_{y}, Z et Z₁ sont tels que définis dans la revendication 3,
ou un sel pharmaceutiquement acceptable ou un solvate de celui-ci.

17. Composé, sel pharmaceutiquement acceptable ou solvate selon la revendication 16, dans lequel R₂ est

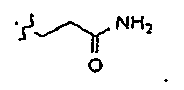

18. Composé, sel pharmaceutiquement acceptable ou solvate selon la revendication 16, dans lequel :
R_{y} est H ou méthyle ;
R₁ est H, F ou méthyle ;
Z et Z₁ sont chacun indépendamment choisis parmi H, F, -CO₂R₂₁, -CN et -C(O)NR₂₁R₂₂, où R₂₁ et R₂₂ sont chacun indépendamment H, un radical alkyle, un radical cycloalkyle, un radical hétérocycloalkyle, un radical aryle, un radical hétéroaryle, un radical acyle ou un radical thioacyle, ou R₂₁ et R₂₂, conjointement avec le
ou les atomes auxquels ils sont liés, forment un radical hétérocycloalkyle, pourvu que Z et Z₁ ne soient pas tous deux H ;
ou Z₁ et R₁, conjointement avec les atomes auxquels ils sont liés, forment un radical cycloalkyle ou hétérocycloalkyle ;
ou Z et Z₁, conjointement avec les atomes auxquels ils sont liés, forment un radical cycloalkyle ou hétérocycloalkyle ;
R₂ est 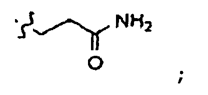 R₆ est phénylméthyle non substitué ou substitué ;
R₇ est alkyle ou aryle ; et
R₉ est 3-isoxazolyle ou 5-isoxazolyle non substitué ou substitué par un ou deux substituants choisis parmi méthyle et les halogènes.

19. Composé, sel pharmaceutiquement acceptable ou solvate selon la revendication 18, dans lequel R₇ est choisi parmi 2-propyle, 2-méthyl-2-propyle, 2-méthyl-1-propyle, phénylméthyle et naphtylméthyle.

20. Composé, sel pharmaceutiquement acceptable ou solvate selon la revendication 16, dans lequel R_{y}, R₁ et Z sont chacun H et :
R₂ est CH₂CH₂C(O)NH₂, R₆ est CH₂Ph, R₇ est CH₂CH(CH₃)₂, Z₁ est CO₂CH₂CH₃ et R₉ est 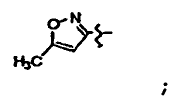
R₂ est CH₂CH₂C(O)NH₂, R₆ est CH₂Ph, R₇ est CH₂CH (CH₃)₂, Z₁ est CO₂CH₂CH₃ et R₉ est 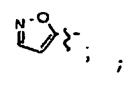
R₂ est CH₂CH₂C(O)NH₂, R₆ est 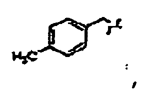 R₇ est C(CH₃)₃, Z₁ est CO₂CH₂CH₃ et R₉ est 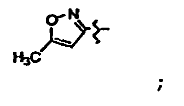
R₂ est CH₂CH₂C(O)NH₂, R₆ est 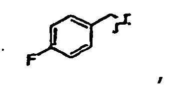 R₇ est C(CH₃)₃, Z₁ est CO₂CH₂CH₃ et R₉ est 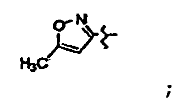
R₂ est CH₂CH₂C(O)NH₂, R₆ est 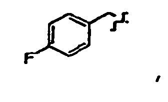 R₇ est CH(CH₃)₂, Z₁ est CO₂CH₂CH₃ et R₉ est

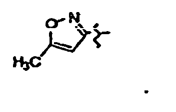

21. Composé, sel pharmaceutiquement acceptable ou solvate selon la revendication 16, dans lequel R_{y} est CH₃, R₁ et Z sont chacun H et :
R₂ est CH₂CH₂C(O)NH₂, R₆ est 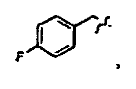 R₇ est 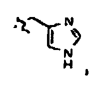 Z₁ est CO₂CH₂CH₃ et R₉ est 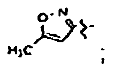
R₂ est CH₂CH₂C(O)NH₂, R₆ est CH₂Ph, R₇ est CH₂CH (CH₃)₂ et R₉ est 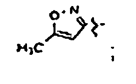
R₂ est CH₂CH₂C(O)NH₂, R₆ est 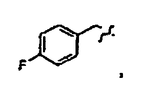 R₇ est 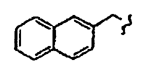 et R₉ est 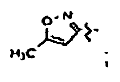
R₂ est CH₂CH₂C(O)NH₂, R₆ est 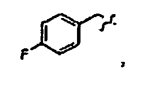 R₇ est CH₂CH(CH₃)₂ et R₉ est 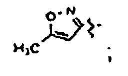 ou
R₂ est CH₂CH₂C(O)NH₂, R₆ est 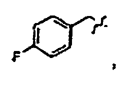 R₇ est 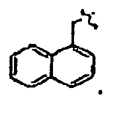 et R₉ est

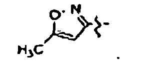

22. Composé, sel pharmaceutiquement acceptable ou solvate selon la revendication 16, choisi dans le groupe constitué de :

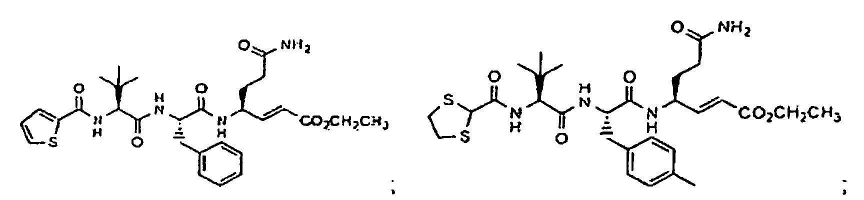

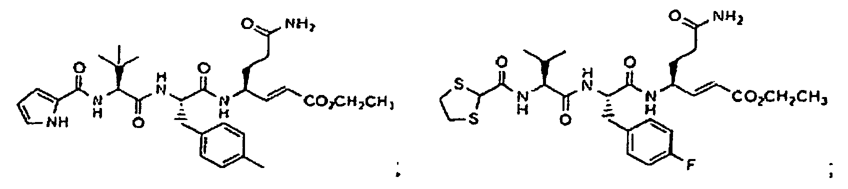

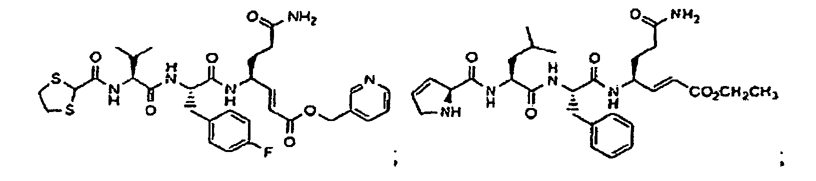

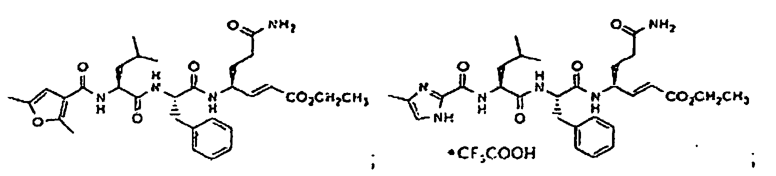

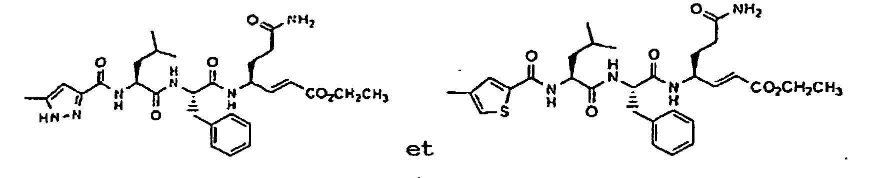

23. Composé, sel pharmaceutiquement acceptable ou solvate selon la revendication 1, dans lequel Y est -CH₂-.

24. Composé, sel pharmaceutiquement acceptable ou solvate selon la revendication 23, dans lequel Z et Z₁ sont chacun indépendamment choisis parmi H, F, un radical alkyle en C₁ à C₄, -CO₂R₂₁ et -C(O)NR₂₁R₂₂, où R₂₁ et R₂₂ sont chacun indépendamment H, un radical alkyle, un radical cycloalkyle, un radical hétérocycloalkyle, un radical aryle, un radical hétéroaryle, un radical acyle ou un radical thioacyle, ou R₂₁ et R₂₂, conjointement avec le ou les atomes auxquels ils sont liés, forment un radical hétérocycloalkyle, pourvu que Z et Z₁ ne soient pas tous deux H ;
ou Z₁ et R₁, conjointement avec les atomes auxquels ils sont liés, forment un radical cycloalkyle ou hétérocycloalkyle ;
ou Z et Z₁, conjointement avec les atomes auxquels ils sont liés, forment un radical cycloalkyle ou hétérocycloalkyle.

25. Composé, sel pharmaceutiquement acceptable ou solvate selon la revendication 23, dans lequel R₁ est H, F ou méthyle.

26. Composé, sel pharmaceutiquement acceptable ou solvate selon la revendication 23, dans lequel au moins l'un parmi R₂ et R₃ est

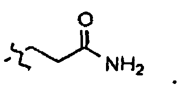

27. Composé, sel pharmaceutiquement acceptable ou solvate selon la revendication 26, dans lequel l'un parmi R₅ ou R₆ est H et l'autre est alkyle ou aryle.

28. Composé, sel pharmaceutiquement acceptable ou solvate selon la revendication 23, dans lequel l'un parmi R₅ et R₆ est H et l'autre est alkyle ou aryle.

29. Composé, sel pharmaceutiquement acceptable ou solvate selon la revendication 23, dans lequel l'un parmi R₅ et R₆ est H et l'autre est phénylméthyle non substitué ou substitué.

30. Composé, sel pharmaceutiquement acceptable ou solvate selon la revendication 23, dans lequel R₇ et R₈ sont chacun indépendamment H, un radical alkyle, un radical cycloalkyle, un radical hétérocycloalkyle, un radical aryle ou un radical hétéroaryle.

31. Composé, sel pharmaceutiquement acceptable ou solvate selon la revendication 23, dans lequel l'un parmi R₇ et R₈ est H et l'autre est alkyle ou aryle.

32. Composé, sel pharmaceutiquement acceptable ou solvate selon la revendication 23, dans lequel l'un parmi R₇ et R₈ est H et l'autre est 2-propyle, 2-méthyl-2-propyle, 2-méthyl-1-propyle, phénylméthyle ou naphtylméthyle.

33. Composé, sel pharmaceutiquement acceptable ou solvate selon la revendication 23, dans lequel R₉ est un hétérocycle à cinq membres ayant au moins un hétéroatome azote et un hétéroatome oxygène.

34. Composé, sel pharmaceutiquement acceptable ou solvate selon la revendication 33, dans lequel R₉ est choisi parmi 1,2-oxazolyle, 1,3-oxazolyle ou 1,2,4-oxadiazolyle non substitué ou substitué.

35. Composé, sel pharmaceutiquement acceptable ou solvate selon la revendication 34, dans lequel R₉ est 3-isoxazolyle ou 5-isoxazolyle non substitué ou substitué par un ou deux substituants choisis parmi méthyle et les halogènes.

36. Composé selon la revendication 23 de la formule I-B" : 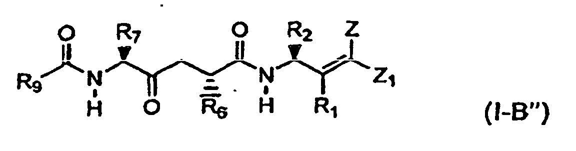 dans laquelle R₁, R₂, R₆, R₇, R₉, Z et Z₁ sont tels que définis dans la revendication 23, ou un sel pharmaceutiquement acceptable ou un solvate de celui-ci.

37. Composé, sel pharmaceutiquement acceptable ou solvate selon la revendication 36, dans lequel R₂ est

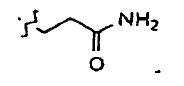

38. Composé, sel pharmaceutiquement acceptable ou solvate selon la revendication 36, dans lequel :
R₁ est H, F ou méthyle ;
Z et Z₁ sont chacun indépendamment choisis parmi H, F, -CO₂R₂₁, -CN et -C(O)NR₂₁R₂₂, où R₂₁ et R₂₂ sont chacun indépendamment H, un radical alkyle, un radical cycloalkyle, un radical hétérocycloalkyle, un radical aryle, un radical hétéroaryle, un radical acyle ou un radical thioacyle, ou R₂₁ et R₂₂, conjointement avec le ou les atomes auxquels ils sont liés, forment un radical hétérocycloalkyle, pourvu que Z et Z₁ ne soient pas tous deux H ;
ou Z₁ et R₁, conjointement avec les atomes auxquels ils sont liés, forment un radical cycloalkyle ou hétérocycloalkyle ;
ou Z et Z₁, conjointement avec les atomes auxquels ils sont liés, forment un radical cycloalkyle ou hétérocycloalkyle ;
R₂ est 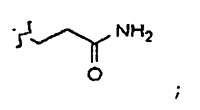
R₆ est phénylméthyle non substitué ou substitué ;
R₇ est alkyle ou aryle ; et
R₉ est 3-isoxazolyle ou 5-isoxazolyle non substitué ou substitué par un ou deux substituants choisis parmi méthyle et les halogènes.

39. Composé, sel pharmaceutiquement acceptable ou solvate selon la revendication 38, dans lequel R₇ est choisi parmi 2-propyle, 2-méthyl-2-propyle, 2-méthyl-1-propyle, phénylméthyle ou naphtylméthyle.

40. Composé, sel pharmaceutiquement acceptable ou solvate selon la revendication 36, dans lequel R₁ est H et :
R₂ est CH₂CH₂C(O)NH₂, R₆ est 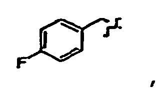 R₇ est CH(CH₃)₂, Z est H, Z₁ est CO₂CH₂CH₃ et R₉ est

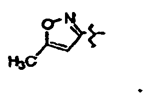

41. Composé, sel pharmaceutiquement acceptable ou solvate selon la revendication 1, dans lequel Y est -O-.

42. Composé, sel pharmaceutiquement acceptable ou solvate selon la revendication 41, dans lequel Z et Z₁ sont chacun indépendamment choisis parmi H, F, un radical alkyle en C₁ à C₄, -CO₂R₂₁ et -C(O)NR₂₁R₂₂, où R₂₁ et R₂₂ sont chacun indépendamment H, un radical alkyle, un radical cycloalkyle, un radical hétérocycloalkyle, un radical aryle, un radical hétéroaryle, un radical acyle ou un radical thioacyle, ou R₂₁ et R₂₂ conjointement avec le ou les atomes auxquels ils sont liés, forment un radical hétérocycloalkyle, pourvu que Z et Z₁ ne soient pas tous deux H ;
ou Z₁ et R₁, conjointement avec les atomes auxquels ils sont liés, forment un radical cycloalkyle ou hétérocycloalkyle ;
ou Z et Z₁, conjointement avec les atomes auxquels ils sont liés, forment un radical cycloalkyle ou hétérocycloalkyle.

43. Composé, sel pharmaceutiquement acceptable ou solvate selon la revendication 41, dans lequel R₁ est H, F ou méthyle.

44. Composé, sel pharmaceutiquement acceptable ou solvate selon la revendication 41, dans lequel au moins l'un parmi R₂ et R₃ est

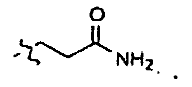

45. Composé, sel pharmaceutiquement acceptable ou solvate selon la revendication 44, dans lequel l'un parmi R₅ et R₆ est H et l'autre est alkyle ou aryle.

46. Composé, sel pharmaceutiquement acceptable ou solvate selon la revendication 41, dans lequel l'un parmi R₅ et R₆ est H et l'autre est alkyle ou aryle.

47. Composé, sel pharmaceutiquement acceptable ou solvate selon la revendication 41, dans lequel l'un parmi R₅ et R₆ est H et l'autre est phénylméthyle non substitué ou substitué.

48. Composé, sel pharmaceutiquement acceptable ou solvate selon la revendication 41, dans lequel R₇ et R₈ sont chacun indépendamment H, un radical alkyle, un radical cycloalkyle, un radical hétérocycloalkyle, un radical aryle ou un radical hétéroaryle.

49. Composé, sel pharmaceutiquement acceptable ou solvate selon la revendication 41, dans lequel l'un parmi R₇ et R₈ est H et l'autre est alkyle ou aryle.

50. Composé, sel pharmaceutiquement acceptable ou solvate selon la revendication 42, dans lequel l'un parmi R₇ et R₈ est H et l'autre est 2-propyle, 2-méthyl-2-propyle, 2-méthyl-1-propyle, phénylméthyle ou naphtylméthyle.

51. Composé, sel pharmaceutiquement acceptable ou solvate selon la revendication 41, dans lequel R₉ est un hétérocycle à cinq membres ayant au moins un hétéroatome azote et un hétéroatome oxygène.

52. Composé, sel pharmaceutiquement acceptable ou solvate selon la revendication 41, dans lequel R₉ est choisi parmi les radicaux 1,2-oxazolyle, 1,3-oxazolyle et 1,2,4-oxadiazolyle substitués et non substitués.

53. Composé, sel pharmaceutiquement acceptable ou solvate selon la revendication 41, dans lequel R₉ est 3-isoxazolyle ou 5-isoxazolyle non substitué ou substitué par un ou deux substituants choisis parmi méthyle et les halogènes.

54. Composé selon la revendication 41 de la formule I-C'' : 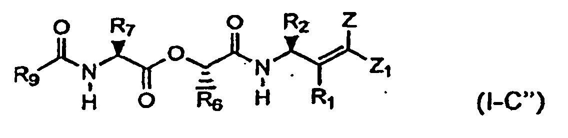 dans laquelle R₁, R₂, R₆, R₇, R₉, Z et Z₁ sont tels que définis dans la revendication 42,
ou un sel pharmaceutiquement acceptable ou un solvate de celui-ci.

55. Composé, sel pharmaceutiquement acceptable ou solvate selon la revendication 54, dans lequel R₂ est

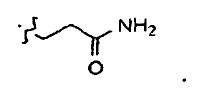

56. Composé, sel pharmaceutiquement acceptable ou solvate selon la revendication 54, dans lequel :
R₁ est H, F ou méthyle ;
Z et Z₁ sont chacun indépendamment choisis parmi H, F, -CO₂R₂₁, -CN et -C(O)NR₂₁R₂₂, où R₂₁ et R₂₂ sont chacun indépendamment H, un radical alkyle, un radical cycloalkyle, un radical hétérocycloalkyle, un radical aryle, un radical hétéroaryle, un radical acyle ou un radical thioacyle, ou R₂₁ et R₂₂, conjointement avec le ou les atomes auxquels ils sont liés, forment un radical hétérocycloalkyle, pourvu que Z et Z₁ ne soient pas tous deux H ;
ou Z₁ et R₁, conjointement avec les atomes auxquels ils sont liés, forment un radical cycloalkyle ou hétérocycloalkyle ;
ou Z et Z₁, conjointement avec les atomes auxquels ils sont liés, forment un radical cycloalkyle ou hétérocycloalkyle ;
R₂ est 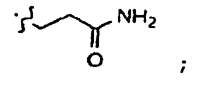
R₆ est phénylméthyle non substitué ou substitué ;
R₇ est alkyle ou aryle ; et
R₉ est 3-isoxazolyle ou 5-isoxazolyle non substitué ou substitué par un ou deux substituants choisis parmi méthyle et les halogènes.

57. Composé, sel pharmaceutiquement acceptable ou solvate selon la revendication 56, dans lequel R₇ est choisi parmi 2-propyle, 2-méthyl-2-propyle, 2-méthyl-1-propyle, phénylméthyle ou naphtylméthyle.

58. Composé, sel pharmaceutiquement acceptable ou solvate selon la revendication 54, dans lequel R₁ est H, Z est H et :
R₂ est CH₂CH₂C(O)NH₂, R₆ est 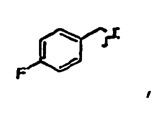 R₇ est CH(CH₃)₂, Z₁ est CO₂CH₂CH₃ et R₉ est

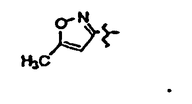

59. Composé, sel pharmaceutiquement acceptable ou solvate selon les revendications 1 ou 58, ayant une activité antipicornavirale correspondant à une CE₅₀ inférieure ou égale à 100 µM dans un dosage de culture de cellules H1-HeLa.

60. Composé, sel pharmaceutiquement acceptable ou solvate selon les revendications 1 ou 58, ayant une activité antirhinovirale correspondant à une CE₅₀ inférieure ou égale à 10 µM dans un dosage de culture de cellules H1-HeLa.

61. Composition pharmaceutique comprenant :
(a) une quantité thérapeutiquement efficace d'au moins un agent antipicornaviral qui est un composé, un sel pharmaceutiquement acceptable ou un solvate tel que défini dans l'une quelconque des revendications 1 à 60 ; et
(b) un support, diluant, véhicule ou excipient pharmaceutiquement acceptable.

62. Utilisation d'un composé, d'un sel pharmaceutiquement acceptable ou d'un solvate tel que défini dans l'une quelconque des revendications 1 à 60, pour la préparation d'un médicament destiné au traitement d'une maladie mammalienne médiée par l'activité protéase des picornavirus.

63. Utilisation selon la revendication 62, pour inhiber l'activité d'une protéase 3C picornavirale.

64. Utilisation selon la revendication 63, dans laquelle la protéase 3C picornavirale est une protéase rhinovirale.
